# EUROPEAN PATENT SPECIFICATION

(11) **EP 2 785 397 B1**
(45) Date of publication and mention of the grant of the patent: **30.12.2020**
(21) Application number: 12853787.5
(22) Date of filing: 30.11.2012
(51) Int. Cl.: A61B 17/42, A61F 6/18, A61M 31/00

(54) **APPARATUS FOR INSERTING A DEVICE OR PHARMACEUTICAL INTO A UTERUS**
VORRICHTUNG ZUM EINSETZEN EINER VORRICHTUNG ODER EINES PHARMAZEUTIKUMS IN EINEN UTERUS
APPAREIL POUR INTRODUIRE UN DISPOSITIF OU UN PRODUIT PHARMACEUTIQUE DANS UN UTÉRUS

(30) Priority: 02.12.2011 US 201161566211 P
(43) Date of publication of application: 08.10.2014
(73) Proprietor: Bioceptive, Inc., New Orleans, Louisiana 70112 (US)
(72) Inventor: CAPPIELLO, Benjamin, New Orleans, Louisiana 70115 (US); KHURANA, Shuchi Priye, Metairie, Louisiana 70005 (US); BACHMAN, Alan, Milford, Connecticut 06460 (US)
(74) Representative: Cooley (UK) LLP
(86) International application number: PCT/US2012/067335
(87) International publication number: WO 2013/082452

(56) References cited:
- EP-B1- 0 513 228
- WO-A2-97/22379
- US-A1- 2005 288 664
- US-A1- 2007 049 951
- US-A1- 2008 033 458
- US-A1- 2010 160 816

## Description

The examples described herein relate to apparatus and methods for inserting a device and/or pharmaceutical into a body cavity. More particularly, the examples described herein relate to apparatus and methods for inserting an intrauterine device (IUD) into the uterus and manipulating a portion of the IUD (e.g., the removal string) during the implantation.

Difficulty of insertion is a significant hurdle to the more widespread use of known intrauterine devices (IUDs) by physicians and health care workers worldwide. A key disadvantage of known methods for IUD insertion relate to the multi-step nature of such known methods and the number of separate medical instruments.

For example, in some known methods of inserting an IUD, a speculum is positioned to visualize the cervix. The cervix is then clamped with downward traction using a cervical tenaculum to substantially straighten and/or align the cervix with the uterine cavity. In certain circumstances, an os finder is used to locate and dilate the cervical os. With the cervical os located, in a vast majority of procedures, a uterine sound is used to determine the depth of the uterine cavity, which is the depth to which the IUD will be inserted. Then the arms of the IUD are folded (either back or forward, depending on the design of the IUD) and positioned within a tube of an inserter. The inserter is then pushed into the vagina allowing the health care provider to find the opening of the cervical canal, and insert, via the cervix, the IUD high into the uterus to the depth measured by the sounding process. The tube of the inserter is pulled back to release the arms of the IUD from the tube at the fundus of the uterus. In some known procedures, the inserter tube is again pushed up against the base of the arms of the IUD to ensure highest achievable placement within the endometrial cavity. The inserter is then extracted from the uterus, cervix, and vagina such that the placement of the IUD is not disrupted. Lastly, the IUD strings are cut to ensure that a sufficient length (e.g., at least 2.5 cm) of the withdrawal string is exposed in the vagina.

The insertion of an IUD according to such known methods can often result in misplacement of the IUD and/or other complications. Said another way, known methods of IUD insertion involve a series of precise operations to ensure proper placement of the IUD. Even slight procedural deviations when using known methods and tools for IUD insertion can lead to uterine wall perforations, increased chance of embedding of the IUD in the endometrium, and/or expulsion of the IUD. In addition, it is possible to push microbes from the vagina into the uterus during the insertion process, which can lead to complications such as pelvic inflammatory disease (PID).

Thus, a need exists for improved apparatus and methods for inserting an intrauterine device (IUD) into the uterus that will reduce these risks and allow IUD insertions to be performed by health care providers across all spectra of medicine.

WO 097/2379 2 and US 2008/033458 both disclose implant apparatus. WO97/22379 describes a method and apparatus for implanting seeds in or about a target area, such as a tumor, within a patient. The implantation device includes an implantation needle having a bore extending longitudinally therethrough from a proximal end to a distal end of the needle, the needle bore being adapted to permit at least one seed to pass therethrough. An elongated plunger extends longitudinally through the implantation device in aligned relation to the needle bore and is selectively movable in the longitudinal direction relative the needle from a retracted position spaced apart from the needle to an extended position wherein the plunger is advanced through the needle bore to eject at least one of the seeds through the bore, out of the distal end of needle and into the target area.; An optical device is carried by and operatively connected to the plunger to provide visual assistance to an operator of the implantation device to guide and verify implantation of the ejected seed into the target area. A seed cartridge is releasably connected to the implantation device to store unused seeds and to load the seeds into the implantation device. US2008/033458 describes a single trigger system and associated method for manipulating tissues and anatomical or other structures in medical applications for the purpose of treating diseases or disorders or other purposes. In one aspect, the system includes a delivery device configured to deploy and implant anchor devices for such purposes.

According to the present invention there is provided an an apparatus as defined in claim 1. In addition, further advantageous embodiments follow from the dependent claims. It is noted that references to "embodiments" throughout the description which are not under the scope of the appended claims represent possible examples and are therefore not part of the present invention unless the context clearly dictates otherwise. The invention is defined by the appended claims.
FIGS. 1 and 2 are schematic illustrations of a delivery device according to an embodiment, in a first and a second configuration, respectively.
FIG. 3 is a schematic illustration of a delivery device according to an embodiment.
FIGS. 4-6 are schematic illustrations of a delivery device according to an embodiment, in a first, a second, and a third configuration, respectively.
FIGS. 7 and 8 are schematic illustrations of a delivery device according to an embodiment, in a first and a second configuration, respectively.
FIGS. 9 and 10 are perspective views of a delivery device according to an embodiment.
FIGS. 11-13 are perspective views of a housing included in the delivery device of FIG. 9.
FIG. 14 is a side view of a first portion of the housing illustrated in FIG. 11.
FIG. 15 is a side view of a second portion of the housing illustrated in FIG. 11.
FIG. 16 is a left side view of the delivery device of FIG. 9 shown without the second portion of the housing of FIG. 15.
FIG. 17 is a right side view of the delivery device of FIG. 9 shown without the first portion of the housing of FIG. 14.
FIG. 18 is a perspective view of a portion of a vacuum assembly included in the delivery device of FIG. 9.
FIG. 19 is a front view of the portion of the vacuum assembly of FIG. 16.
FIG. 20 is a cross-sectional view of the portion of the vacuum assembly of FIG. 18, taken along line X₁-X₁ in FIG. 19.
FIGS. 21 and 22 are a front perspective view and a rear perspective view, respectively, of a portion of the vacuum assembly included in the delivery device of FIG. 9.
FIG. 23 is a perspective view of a guide mechanism included in the delivery device of FIG. 9.
FIG. 24 is an exploded view of the guide mechanism of FIG. 23.
FIG. 25 is an exploded view of an actuator assembly included in the delivery device of FIG. 9.
FIG. 26 is a top perspective view and FIG. 27 is a bottom perspective view of a drive member included in the actuator assembly of FIG. 25.
FIG. 28 is a top perspective view of a transfer mechanism included in the delivery device of FIG. 9.
FIGS. 29 and 30 are a top perspective view and a bottom perspective view, respectively, of a transfer member included in the transfer mechanism of FIG. 28.
FIG. 31 is a top perspective view of a lockout member included in the transfer mechanism of FIG. 28.
FIG. 32 is an exploded view of an insertion assembly included in the delivery device of FIG. 9.
FIGS. 33 and 34 are a top perspective view and a bottom perspective view, respectively, of a carrier included in the insertion assembly of FIG. 32.
FIG. 35 is a perspective view of a slip member included in the insertion assembly of FIG. 32.
FIG. 36 is a perspective view illustrating the carrier of FIG. 33 and the slip member of FIG. 35 being coupled to the transfer mechanism of FIG. 28.
FIG. 37 is a perspective view of a status indicator member included in the insertion assembly of FIG. 32.
FIGS. 38 and 39 are a top perspective view and a bottom perspective view, respectively, of an engagement member included in the insertion assembly of FIG. 32.
FIG. 40 is a bottom perspective views of a push rod tube included in the insertion assembly of FIG. 32.
FIGS. 41 and 42 are perspective views of an outer sheath included in the insertion assembly of FIG. 32.
FIG. 43 is an exploded view of a cutter assembly included in the delivery device of FIG. 9.
FIG. 44 is a side view of the delivery device of FIG. 9 shown without the second portion of the housing of FIG. 15, in a first configuration.
FIGS. 45-47 are enlarged views of a portion of the delivery device indicated by region Z₁ in FIG. 44, illustrating a method of loading an implant into the delivery device.
FIG. 48 is a side view of the delivery device of FIG. 9 shown without the second portion of the housing of FIG. 15, in a second configuration.
FIG. 49 is an enlarged view of a portion of the delivery device in the second configuration, indicated by the region Z₂ in FIG. 48.
FIG. 50 is a side view of the delivery device of FIG. 9 shown without the second portion of the housing of FIG. 15, in a third configuration.
FIG. 51 is an enlarged view of a portion of the delivery device in the third configuration, indicated by the region Z₃ in FIG. 50.
FIG. 52 is an enlarged view of a portion of the delivery device in the third configuration, indicated by the region Z₄ in FIG. 50.
FIG. 53 is a side view of the delivery device of FIG. 9 shown without the second portion of the housing of FIG. 15, in a fourth configuration.
FIG. 54 is an enlarged view of a portion of the delivery device in the fourth configuration, indicated by the region Z₅ in FIG. 53.
FIG. 55 is an enlarged view of a portion of the delivery device in the fourth configuration, indicated by the region Z₆ in FIG. 53.
FIG. 56 is a side view of the delivery device of FIG. 9 shown without the second portion of the housing of FIG. 15, in a fifth configuration.
FIG. 57 is an enlarged view of a portion of the delivery device in the fifth configuration, indicated by the region Z₇ in FIG. 56.
FIG. 58 is a perspective side view of the delivery device of FIG. 9 illustrating a force limiting condition.
FIGS. 59 and 60 are top views of a portion of the delivery device of FIG. 9, illustrating a lockout condition.
FIG. 61 is a side view of the delivery device of FIG. 9 shown without the second portion of the housing of FIG. 15, in a sixth configuration.
FIG. 62 is an enlarged side view of a portion of the delivery device in the sixth configuration, indicated by the region Zg in FIG. 61.
FIG. 63 is an enlarged top view of the portion of the delivery device in the sixth configuration, indicated by the region Z₈ in FIG. 61.
FIGS. 64-73 are various views of a delivery device according to an embodiment.
FIG. 74 is a flowchart describing a method of using a delivery device.

### Detailed Description

Apparatus and methods for inserting a device and/or pharmaceutical into a body cavity are described herein. In some embodiments, a delivery device includes a housing, an insertion member and a transfer member. The insertion member includes a distal end portion configured to be removably coupled to an implant. At least a portion of the insertion member is movably disposed within a passageway defined by the housing. The transfer member is configured to be coupled to the insertion member to transfer a force from an actuator to the insertion member such that the insertion member is moved relative to the housing. The transfer member includes a coupling portion configured to move relative to the insertion member when the force exerted by the actuator exceeds a threshold value.

In some embodiments, a delivery device includes a housing, an insertion member, a transfer member, and an actuator. The housing includes a contact surface configured to contact a surface associated with a target location. The insertion member includes a distal end portion configured to be removably coupled to an implant. At least a portion of the insertion member is movably disposed within a passageway defined by the housing. The transfer member is configured to be coupled to the insertion member to transfer a force from the actuator to the insertion member such that the insertion member is moved in a distal direction relative to the housing. The transfer member is further configured to limit the distal movement of the insertion member when the distal end portion of the insertion member contacts the target location.

In some embodiments, a delivery device includes a housing defining a passageway, an insertion member, and a guide member. The insertion member includes a distal end portion configured to be removably coupled to an implant. At least a portion of the insertion member is configured to move relative to the housing to deliver the implant to a target location. The guide member includes a first end portion that is movably coupled to the housing and a second end portion that is removably coupled to a portion of the implant. The guide member is configured to move the portion of the implant within the passageway of the housing when the guide member is moved relative to the housing.

In some embodiments, a delivery device includes a housing, an insertion member, a manipulator, and a transfer member. The housing defines a passageway configured to receive at least a portion of the insertion member. The insertion member includes a distal end portion configured to be removably coupled to a first portion of an implant. The manipulator is configured to manipulate a second end portion of the implant. The transfer member includes a first portion and a second portion. The first portion is coupled to the insertion member such that movement of the transfer member relative to the housing results in movement of the insertion member relative to the housing. The second portion is configured to actuate the manipulator when the transfer member is moved relative to the housing.

The delivery devices described herein can be a disposable, comprehensive unit that articulates with a cervix and facilitates insertion of an intrauterine device to a desired and/or predetermined position and/or orientation within the body. The embodiments described herein, can improve known procedures that employ up to five separate medical instruments by allowing same procedures to be completed using only one device. In doing so, the embodiments described herein can make the procedure of inserting an IUD significantly more intuitive and easy to perform, thus decreasing the amount of adverse events, mainly accidental expulsions, while also greatly expanding access to IUDs worldwide by providing any of the embodiments described herein that anyone can operate with minimal training. The delivery devices described herein can reduce or eliminate perforation of the tissue of the cervix and uterus by including any suitable mechanism(s) that limit forces applied during the insertion process. Also, the probability to place an IUD as close to the fundus of the uterus as possible will be significantly increased as compared to the placement of an IUD by the known inserters.

As used in this specification, the singular forms "a," "an" and "the" include plural referents unless the context clearly dictates otherwise. Thus, for example, the term "a member" is intended to mean a single member or a combination of members, "a material" is intended to mean one or more materials, or a combination thereof. Furthermore, the words "proximal" and "distal" refer to direction closer to and away from, respectively, an operator of the medical device. Thus, for example, the end of the medicament delivery device contacting the patient's body would be the distal end of the medicament delivery device, while the end opposite the distal end would be the proximal end of the medicament delivery device.

As used herein, the terms "about" and "approximately" generally mean plus or minus 10% of the value stated. For example, about 0.5 would include 0.45 and 0.55, about 10 would include 9 to 11, about 10000 would include 900 to 11000.

As used herein, the term "set" can refer to multiple features or a singular feature with multiple parts. For example, when referring to set of walls, the set of walls can be considered as one wall with multiple portions, or the set of walls can be considered as multiple, distinct walls. Thus, a monolithically constructed item can include a set of walls. Such a set of walls may include multiple portions that are either continuous or discontinuous from each other. For example, a monolithically constructed wall can include a set of detents can be said to form a set of walls. A set of walls can also be fabricated from multiple items that are produced separately and are later joined together (e.g., via a weld, an adhesive, or any suitable method).

As used herein, the term "parallel" generally describes a relationship between two geometric constructions (e.g., two lines, two planes, a line and a plane or the like) in which the two geometric constructions are substantially non-intersecting as they extend substantially to infinity. For example, as used herein, a line is said to be parallel to another line when the lines do not intersect as they extend to infinity. Similarly, when a planar surface (i.e., a two-dimensional surface) is said to be parallel to a line, every point along the line is spaced apart from the nearest portion of the surface by a substantially equal distance. Two geometric constructions are described herein as being "parallel" or "substantially parallel" to each other when they are nominally parallel to each other, such as for example, when they are parallel to each other within a tolerance. Such tolerances can include, for example, manufacturing tolerances, measurement tolerances or the like.

As used herein, the term "slope" generally describes a relationship between two geometric constructions in which the two geometric constructions are disposed at an angular orientation to each other. For example, an object's slope is related to an angle of a surface of the object relative to a neutral axis or plane. Furthermore, an object's slope is generally understood to be a change in height of the object along a given length of the neutral axis or plane. Thus, an object's slope forms an angle with the neutral axis referred to herein as "slope angle."

As used herein, the term "stiffness" is related to an object's resistance to deflection, deformation, and/or displacement that is produced by an applied force, and is generally understood to be the opposite of the object's "flexibility." For example, a wall of a tube with greater stiffness is more resistant to deflection, deformation and/or displacement when exposed to a force than a wall of a tube having a lower stiffness. Similarly stated, a tube having a higher stiffness can be characterized as being more rigid than a tube having a lower stiffness. Stiffness can be characterized in terms of the amount of force applied to the object and the resulting distance through which a first portion of the object deflects, deforms, and/or displaces with respect to a second portion of the object. When characterizing the stiffness of an object, the deflected distance may be measured as the deflection of a portion of the object different from the portion of the object to which the force is directly applied. Said another way, in some objects, the point of deflection is distinct from the point where force is applied.

Stiffness (and therefore, flexibility) is an extensive property of the object being described, and thus is dependent upon the material from which the object is formed as well as certain physical characteristics of the object (e.g., cross-sectional shape, length, boundary conditions, etc.). For example, the stiffness of an object can be increased or decreased by selectively including in the object a material having a desired modulus of elasticity, flexural modulus and/or hardness. The modulus of elasticity is an intensive property of (i.e., is intrinsic to) the constituent material and describes an object's tendency to elastically (i.e., non-permanently) deform in response to an applied force. A material having a high modulus of elasticity will not deflect as much as a material having a low modulus of elasticity in the presence of an equally applied stress. Thus, the stiffness of the object can be decreased, for example, by introducing into the object and/or constructing the object of a material having a relatively low modulus of elasticity.

The stiffness of an object can also be increased or decreased by changing a physical characteristic of the object, such as the shape or cross-sectional area of the object. For example, an object having a length and a cross-sectional area may have a greater stiffness than an object having an identical length but a smaller cross-sectional area. As another example, the stiffness of an object can be reduced by including one or more stress concentration risers (or discontinuous boundaries) that cause deformation to occur under a lower stress and/or at a particular location of the object. Thus, the stiffness of the object can be decreased by decreasing and/or changing the shape of the object.

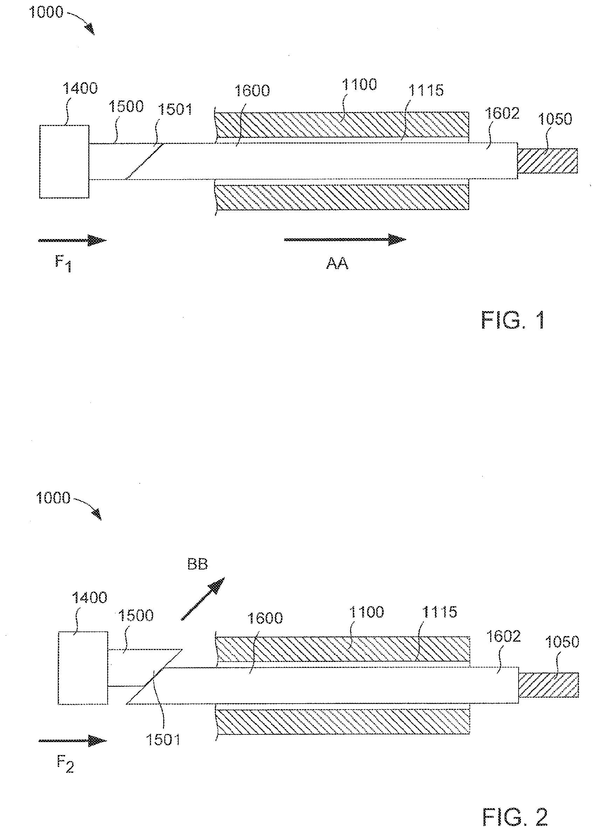

FIGS. 1 and 2 are schematic illustrations of a delivery device 10000 according to an embodiment, in a first configuration and a second configuration, respectively. The delivery device 1000 can deliver an implant 1050 to a target location within the body. For example, in some embodiments, the delivery device 1000 can be used to place an intrauterine device (IUD) in contact with the fundus of and/or within a uterus.

The delivery device 1000 includes a housing 1100, an actuator 1400, a transfer member 1500, and an insertion member 1600. The housing 1100 defines a passageway 1115 within which at least a portion of the insertion member 1600 is disposed. The housing 1100 can be any suitable shape, size, or configuration. For example, in some embodiments, at least a portion of the housing 1100 can be substantially cylindrical having an outer diameter suitable for insertion into a body orifice. In some embodiments, the housing 1100 can include a distal end portion configured to engage and/or contact a surface of a target location to at least temporarily couple the housing 1100 thereto, as described in further detail herein. While not shown in FIGS. 1 and 2, in some embodiments the housing 1100 can include a proximal end portion that can be engaged and/or manipulated by a user. For example, in some embodiments, the proximal end portion of the housing 1100 can form or include a handle or the like.

At least a portion of the insertion member 1600 is movably disposed within the passageway 1115 defined by the housing 1100. For example, in some embodiments, a distal end portion of the insertion member 1600 can extend beyond a distal end portion of the housing 1100, as shown in FIGS. 1 and 2. Moreover, the distal end portion 1602 of the insertion member 1600 can be removably coupled to the implant 1050 (e.g., an IUD). In this manner, upon delivery of the implant 1050 to a target location (not shown), the implant 1050 can be released and/or removed from the insertion member 1600. For example, in some embodiments, the distal end portion 1602 of the insertion member 1600 can be coupled to a portion of the implant 1050 via a friction fit, a snap fit, a press fit, a threaded fit, or the like. In some embodiments, at least a portion of the implant 1050 can be disposed within a portion of the insertion member 1600 (e.g., in such embodiments in which the portion of the insertion member 1600 defines a lumen or passageway that can receive at least a portion of the implant).

A portion of the insertion member 1600 is in contact (either directly, as shown, or indirectly) with a coupling portion 1501 of the transfer member 1500. For example, in some embodiments, a proximal end portion of the insertion member 1600 is placed in contact with the transfer member 1500. In other embodiments, the proximal end portion of the insertion member 1600 can be disposed in a proximal position relative to the transfer member 1500 (e.g., the transfer member 1500 can be in contact with a portion of the insertion member 1600 other than the proximal end portion).

The insertion member 1600 can be any suitable shape, size, or configuration. For example, in some embodiments, the insertion member 1600 can have an outer perimeter with a shape and size associated with the shape and size of the passageway 1115. In some embodiments, the insertion member 1600 can be a mechanism including any number of individual parts that are coupled together to perform any of the function of inserting an implant as described herein. In such embodiments, any of the individual parts of the mechanism can be moved relative to the other parts forming the mechanism. For example, in some embodiments, the insertion member 1600 can include a first insertion member and a second insertion member (not shown in FIGS. 1 and 2) configured to move relative to each other, as further described herein.

The transfer member 1500 is configured to transfer at least a portion of a force (e.g., a force F₁ and/or a force F₂ shown in FIGS. 1 and 2, respectively) to the insertion member 1600 to move the insertion member 1600 relative to the housing 1100. Moreover, as described below, the transfer member 1500 is configured to move relative to the insertion member (i.e., to "slip") when the force exceeds a threshold value. In this manner, the force with which the implant is delivered can be controlled. As described above, the transfer member 1500 includes a coupling portion 1501 that can be placed in contact with (either directly or indirectly) a portion of the insertion member 1600. The transfer member 1500 can be any suitable shape, size, or configuration. For example, while the coupling portion 1501 of the transfer member 1500 is shown as being angular, in other embodiments, the coupling portion 1501 can be substantially rounded. In other embodiments, the coupling portion 1501 of the transfer member 1500 can include a set of detents that can matingly receive the portion of the insertion member 1600. For example, in some embodiments, the insertion member 1600 can include a rounded protrusion configured to matingly couple the insertion member 1600 to the transfer member 1500.

The transfer member 1500 is operably coupled to the actuator 1400. For example, in some embodiments, the transfer member 1500 can be directly coupled to the actuator 1400. In other embodiments, the transfer member 1500 can be coupled to the actuator 1400 via an intervening structure such as, for example, a rack, a pinion, one or more linkages, a push rod, or the like. For example, in some embodiments, the actuator 1400 can include a trigger configured to pivot relative to the housing 1100 and can be coupled to the transfer member 1500 via a rack and pinion. In some embodiments, the transfer member 1500 can include a set of angular detents configured to be sequentially engaged by a push rod included in the actuator 1400, as described herein with respect to specific embodiments.

The actuator 1400 can be any suitable actuator 1400 configured to exert a force on at least the transfer member 1500. For example, in some embodiments, the actuator 1400 can be a trigger, a push button, a slide, a dial and/or a toggle configured to close an electrical circuit, or any other suitable energy source. As shown in FIG. 1, the actuator 1400 is configured to exert a force F₁ on the transfer member 1500 to move the transfer member 1500 relative to the housing 1100. More specifically, the actuator 1400 can exert the force F₁ to move the transfer member 1500 in the distal direction relative to the housing 1100, as indicated by the arrow **AA.** With the coupling portion 1501 of the transfer member 1500 in contact with the insertion member 1600, the transfer member 1500 can transfer at least a portion of the force F₁ to the insertion member 1600 to move the insertion member 1600 and/or the implant 1050 relative to the housing 1100 in the direction of the arrow **AA.** Thus, the insertion member 1600 can be moved to place the implant 1050 (e.g., and IUD) at a desired target location (e.g., a fundus of a uterus) within the body.

In certain circumstances, the coupling portion 1501 of the transfer member 1500 can move relative to the insertion member 1600. In particular, the coupling portion 1501 can move relative to the insertion member 1600 when a force F₂ exceeds a threshold value, as indicated by the arrow **BB** in FIG. 2. In this manner, the transfer member 1500 and/or the insertion member 1600 "slip" during an insertion event to limit the force of insertion. Expanding further, in certain circumstances during an insertion event, the distal end portion 1602 of the insertion member 1600 and/or a portion of the implant 1050 can be placed in contact with the target location and/or other surrounding tissue (e.g., fibroid tissue or the like) such that further actuation of the actuator 1400 increases the force applied to the transfer member 1500. The increased force during such an event is represented by the force F₂ in FIG. 2. As shown, at least the coupling portion 1501 of the transfer member 1500 can move relative to the insertion member 1600 to limit the amount of force transferred to the insertion member 1600 and/or the implant 1050, thereby preventing an undesirable amount of force from being applied to the target location (e.g., the fundus of a uterus). For example, in some embodiments, a portion of the transfer member 1500 can slide relative to a portion of the insertion member 1600 as indicated by the arrow **BB.** Similarly stated, the force F₂ exerted by the actuator 1400 can be sufficiently large to overcome a friction force maintaining the coupling portion 1501 relative to the insertion member 1600. Thus, the coupling portion 1501 of the transfer member 1500 can move relative to the insertion member 1600 while remaining in contact with at least a portion of the insertion member 1600. In this manner, only a portion of the force F₂ is transferred to the insertion member 1500 and/or implant. In some embodiments, a portion of the insertion mechanism 1600 and/or a portion of the transfer mechanism 1500 can be configured to deform (e.g., elastically or plastically) to allow the coupling member 1501 to move relative to the insertion member 1600.

In some embodiments, the transfer member 1500 can include a lock member (not shown in FIGS. 1 and 2) configured to engage a portion of the housing 1100 to limit distal movement of the transfer member 1500 and/or the insertion member 1600 relative to the housing 1100. For example, in some embodiments, when the coupling portion 1501 of the transfer member 1500 is moved a maximum distance relative to the insertion member 1600 (e.g., a "maximum slip" condition), the lock member is moved (e.g., via a spring or the like) into contact with the portion of the housing 1100. In this manner, the lock member and the housing 1100 can prevent further distal movement of the transfer member 1500 relative to the housing 1100 to prevent an undesirable amount of force applied to the target location.

When the distal end portion 1602 of the insertion member 1600 and/or the implant 1050 is placed adjacent to the target location (not shown in FIGS. 1 and 2), the insertion member 1600 can be decoupled from the implant 1050. For example, in some embodiments, the delivery device 1000 can include a second insertion member (not shown in FIGS. 1 and 2) configured to move relative to the insertion member 1600 to decouple the insertion member 1600 from the implant 1050.

Although transfer member 1500 is shown as maintaining contact with the insertion member 1600 (both during a no-slip condition and a slip condition), in other embodiments, the transfer member 1500 can be spaced apart from the insertion member 1600 during portions of an insertion event. For example, in some embodiments, the actuator 1400 can be configured to exert a force in a first direction (e.g., the direction of the arrow **AA**) and a force in a second direction, opposite the first. For example, in some embodiments, the actuator 1400 can include a bias member that is configured to return the actuator 1400 to a first configuration (e.g., a non-actuated configuration) after the actuator 1400 has been actuated. In such embodiments, the transfer member 1500 can be coupled to the actuator 1400 such that the transfer member 1500 is moved in a reciprocating motion relative to the housing 1100. In this manner, the transfer member 1500 can move concurrently with the actuator 1400 and the insertion member 1600 when moved in the first direction (e.g., a distal direction) and can move concurrently with the actuator 1400 and relative to the insertion member 1600 when moved in the second direction (e.g., a proximal direction). In such embodiments, the transfer member 1500 can be spaced apart from the insertion member 1600 when the transfer member 1500 is moved in the second direction.

Although not shown in FIGS. 1 and 2, in some embodiments a portion of the insertion member 1600 can be placed in contact with a portion of the housing 1100 (e.g., a wall or feature defining a portion of the passageway 1115) to limit proximal movement of the insertion member 1600 relative to the housing 1100. For example, in some embodiments, the insertion member 1600 can include a pawl configured to engage a set of detents and/or sloped teeth defined by a portion of the housing 1100. Expanding further, the pawl and the detents and/or teeth can be arranged to allow the insertion member 1600 to be moved in a distal direction relative to the housing 1100 while limiting movement of the insertion member 1600 in the proximal direction. Thus, in embodiments where the transfer member 1500 is arranged for reciprocating motion relative to the housing 1100, the transfer member 1500 can move in the proximal direction without substantially moving the insertion member 1600 in the proximal direction.

Although the coupling portion 1501 is shown in FIG. 2 as translating relative to the insertion member 1600, in other embodiments, a transfer member 1500 can include a coupling portion that is configured to deform when the force exerted by an actuator exceeds a threshold value. For example, in some embodiments, a coupling portion can include one or more stress concentration risers. In such embodiments, the stiffness of the transfer member can be reduced at the location of the one or more stress concentration risers. Thus, the transfer member can deform at the location of the one or more stress concentration risers to limit the force transferred to the insertion member and/or to allow the transfer member to move relative to the insertion member.

Although not shown in FIGS. 1 and 2, the delivery device 1000 can include any suitable feature, system, assembly, or subassembly configured to facilitate the placement of the implant 1050 into the target location. For example, in some embodiments, the delivery device 1000 can include a loading assembly configured to load an implant into the delivery device 1000, a vacuum assembly configured to temporarily couple the delivery device to a contact surface associated with the target location, and/or any other suitable feature.

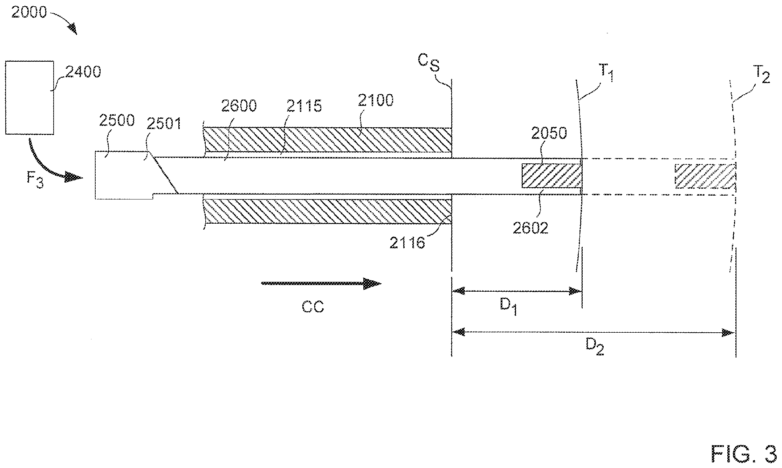

Although not shown in FIGS. 1 and 2, in some embodiments, a delivery device can be configured to move a distal end portion of an insertion member relative to a distal end portion of a housing by a variable distance such that the delivery device can accommodate patients having a wide variation in anatomical dimensions. For example, FIG. 3 is a schematic illustration of a delivery device 2000 according to an embodiment. The delivery device 2000 is configured to deliver an implant 2050 to a target location within the body. For example, in some embodiments, the delivery device 2000 can be used to place an intrauterine device (IUD) in contact with the fundus of and/or within a uterus.

The delivery device 2000 includes a housing 2100, an actuator 2400, a transfer member 2500, and an insertion member 2600. The housing 2100 includes a contact surface 2116 configured to contact a surface Cₛ associated with a target location T₁ and T₂, and defines a passageway 2115 configured to receive at least a portion of the insertion member 2600. The housing 2100 can be any suitable shape, size, or configuration. For example, in some embodiments, at least a portion of the housing 2100 can be substantially cylindrical with an outer diameter suitable for insertion into a body orifice. In some embodiments, the housing 2100 can be substantially similar to the housing 1100 described above with reference to FIGS. 1 and 2, and any other of the housings described herein. Thus, similar portions of the housing 2100 are not described in further detail herein.

At least a portion of the insertion member 2600 is movably disposed within the passageway 2115 defined by the housing 2100. For example, in some embodiments, a distal end portion 2602 of the insertion member 2600 can extend beyond a distal end portion of the housing 2100, as shown in FIG. 3. Moreover, the distal end portion 2602 of the insertion member 2600 can be removably coupled to the implant 2050 (e.g., an IUD). In this manner, upon delivery of the implant 2050 to the target location T₁ and/or T₂, the implant 2050 can be released and/or removed from the insertion member 2600. For example, in some embodiments, the insertion member 2600 can be coupled to a portion of the implant 2050 via a friction fit, a snap fit, a press fit, a threaded fit, or the like. In some embodiments, at least a portion of the implant 2050 can be disposed within a portion of the insertion member 2600 (e.g., a lumen or passageway defined by the insertion member 2600 that can receive at least a portion of the implant). The insertion member 2600 can be substantially similar to the insertion member 1600 described above with reference to FIGS. 1 and 2, thus, portions of the insertion member 2600 are not described in further detail herein.

A portion of the insertion member 2600 is in contact with (either directly, as shown, or indirectly) a coupling portion 2501 of the transfer member 2500. For example, in some embodiments, a proximal end portion of the insertion member 2600 is in contact with the transfer member 2500. In other embodiments, the proximal end portion of the insertion member 2600 can be disposed in a proximal position relative to the transfer member 2500 (e.g., the transfer member 2500 is in contact with a portion of the insertion member 2600 other than the proximal end portion).

The transfer member 2500 is configured to transfer at least a portion of a force F₃ to the insertion member 2600 to move the insertion member 2600 relative to the housing 2100. Moreover, as described below, the transfer member 2500 can limit movement of the insertion member 2600 in the distal direction when the distal end portion 2602 of the insertion member 2600 contacts the target location T₁ and/or T₂ and/or any other desired object. In this manner, the distance through which the insertion member 2500 moves during an insertion event can be varied to accommodate anatomical differences between patients. As described above, the transfer member 2500 includes a coupling portion 2501 that is in contact with a portion of the insertion member 2600. The transfer member 2500 can be any suitable shape, size, or configuration. For example, while the coupling portion 2501 of the transfer member 2500 is shown as being angular, in other embodiments, the coupling portion 2501 can be substantially rounded. In some embodiments, the transfer member 2500 can be similar in form and function as the transfer member 1500 described above with reference to FIGS. 1 and 2 and/or any of the other transfer members described herein.

As shown in FIG. 3, the transfer member 2500 is at least operably coupled to the actuator 2400. In some embodiments, the transfer member 2500 can be directly coupled to the actuator 2400. In other embodiments, the transfer member 2500 can be coupled to the actuator 2400 via an intervening structure. The actuator 2400 can be any suitable actuator 2400 configured to exert a force on at least the transfer member 2500. In some embodiments, the actuator 2400 can be substantially similar in form and function as the actuator 1400 described above with reference to FIGS. 1 and 2 and/or any of the other actuators described herein. Thus, similar portions of the actuator 2400 are not described in further detail herein.

As shown in FIG. 3, the actuator 2400 is configured to exert a force F₃ on the transfer member 2500 to move the transfer member 2500 relative to the housing 2100. More specifically, the actuator 2400 can exert the force F₃ to move the transfer member 2500 in the distal direction relative to the housing 2100, as indicated by the arrow **CC.** With the coupling portion 2501 of the transfer member 2500 in contact with the insertion member 2600, the transfer member 2500 can transfer at least a portion of the force F₃ to the insertion member 2600 to move the insertion member 2600 and/or the implant 2050 relative to the housing 2100 in the direction of the arrow **CC.** Thus, the insertion member 2600 can be moved to place the implant 2050 (e.g., and IUD) at a desired target location.

As shown in FIG. 3, in certain circumstances, a target location T₁ can be located at a first distance D₁ from the contact surface Cₛ (e.g., a surface of the cervix). For example, in some embodiments, the first distance D₁ can be approximately five centimeters. Thus, during an insertion event, the insertion member 2600 can extend and/or be moved the first distance D₁ from the contact surface 2116 of the housing 2100 to place the implant 2050 at the target location T₁. When the distal end portion 2602 of the insertion member 2600 is placed in contact with the target location T₁, at least the coupling portion 2501 of the transfer member 2500 can limit further movement of the insertion member 2600 in the direction of the arrow **CC.** Thus, the implant 2050 can be placed at the target location T₁ without moving past and/or applying an undesired amount of force on the target location T₁. In some embodiments, the transfer member 2500 can move relative to the insertion member 2600 to limit further movement of the insertion member 2600.

In other circumstances, a target location T₂ can be located at a second distance D₂ from the contact surface Cₛ (e.g., because the anatomy of a second patient is different from the anatomy of a first patient). For example, in some embodiments, the second distance D₂ can be as great as 13 centimeters. Thus, during an insertion event, the insertion member 2600 can extend and/or move the second distance D₂ from the contact surface 2116 of the housing 2100 to place the implant 2050 at the target location T₂. When the distal end portion 2602 of the insertion member 2600 is placed in contact with the target location T₂, at least the coupling portion 2501 of the transfer member 2500 can limit further distal movement of the insertion member 2600 in the direction shown by the arrow **CC.** Thus, the implant 2050 can be placed at the target location T₂ without moving past and/or applying an undesired amount of force on the target location. Thus, the same device 2000 can be used to place the implant 2050 through a distance of between D₁ and D₂.

While not described herein in detail, the delivery device 2000 can function similarly to the delivery device 1000 described in detail with reference to FIGS. 1 and 2 and/or any of the other delivery devices shown and described herein. Moreover, while not shown in FIG. 3, the delivery device 2000 can include any suitable feature, system, assembly, or subassembly configured to facilitate the placement of an implant at a target location.

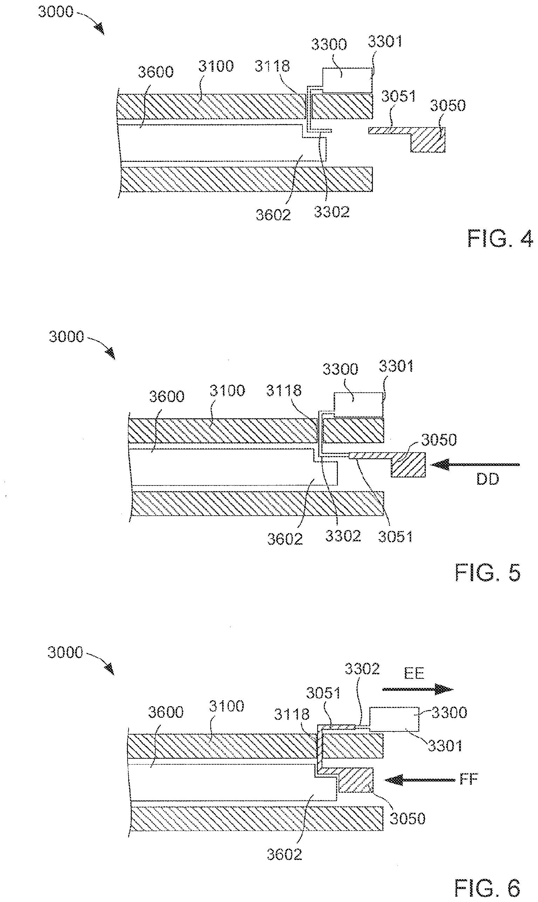

FIGS. 4-6 show a delivery device 3000 according to an embodiment. The delivery device 3000 is configured to deliver an implant 3050 to a target location (not shown) within a body. For example, in some embodiments, the delivery device 3000 can be used to place an intrauterine device (IUD) in contact with the fundus and/or within of a uterus. The delivery device 3000 includes a housing 3100, a guide member 3300, and an insertion member 3600. The housing 3100 defines a passageway 3118 configured to receive at least a portion of the guide member 3300 and/or a portion of the implant 3050, as described in further detail below. The housing 3100 can be any suitable shape, size, or configuration. For example, in some embodiments, at least a portion of the housing 3100 can be substantially cylindrical with an outer diameter suitable for insertion into a body orifice. In some embodiments, the housing 3100 can be substantially similar to the housing 1100 described above with reference to FIGS. 1 and 2 and/or any of the other housings described herein. Thus, similar portions of the housing 3100 are not described in further detail herein.

At least a portion of the insertion member 3600 is movably disposed within the housing 3100. For example, in some embodiments, the insertion member 3600 can be operably coupled to an actuator and/or a transfer member (not shown in FIGS. 4-6) configured to move the insertion member 3600 relative to the housing 3100. Thus, the insertion member 3600 can be moved in a distal direction to deliver the implant 3050 to a target location, as described in detail above.

As shown, the insertion member 3600 includes a distal end portion 3602 that can be removably coupled to the implant 3050 (see e.g., FIG. 6). For example, in some embodiments, the insertion member 3600 can be coupled to a portion of the implant 3050 via a friction fit, a snap fit, a press fit, or the like. In some embodiments, at least a portion of the implant 3050 can be disposed within a portion of the insertion member 3600 (e.g., a lumen or passageway defined by the insertion member 3600 that can receive at least a portion of the implant). The insertion member 3600 can be substantially similar in form and/or function as the insertion member 1600 described above with reference to FIGS. 1 and 2 and/or any of the other insertion members described herein. Thus, portions of the insertion member 3600 are not described in further detail herein.

The guide member 3300 of the delivery device 3000 is configured to move relative to the housing 3100 to couple the implant 3050 to the insertion member 3600. The guide member 3300 includes a first portion 3301 that is movably coupled to the housing 3100 and a second portion 3302 that is removably coupled to the portion 3051 of the implant 3050. For example, in some embodiments, at least a feature (e.g., a tab, a protrusion, a flange, etc.) of the first portion 3301 can be disposed within an opening (not shown in FIGS. 4-6) defined by the housing 3100 such that the guide member 3300 can be moved along a length of the housing 3100. In other embodiments, at least the first portion 3301 of the guide member 3300 can be disposed within a portion of the housing 3100, and can be configured to move away from (i.e., spaced apart from) a portion of the housing 3100. For example, in some embodiments, the first portion 3301 of the guide member 3300 can be moved in a perpendicular direction relative to a longitudinal centerline of the housing 3100. In other embodiments, the first portion 3301 can be moved in the proximal or distal direction beyond a proximal surface or distal surface, respectively, of the housing 3100

The second portion 3302 of the guide member 3300 is configured to be movably disposed, at least temporarily, within the passageway 3118 defined by the housing 3100. The second portion 3302 can include any suitable feature or mechanism configured to removably couple the second portion 3302 to the portion 3051 of the implant 3050. For example, in some embodiments, the second portion 3302 of the guide member 3300 can include a snare that can be moved between an open (or expanded) configuration and a closed (or collapsed) configuration to couple the portion 3051 of the implant 3050 thereto.

The guide member 3300 can be any suitable shape, size, or configuration. For example, in some embodiments the guide member 3300 can be monolithically constructed. In other embodiments, the guide member 3300 can be formed from more than one part such that at least one part can move relative to one or more other parts. For example, in some embodiments, a portion of the guide member 3300 can be moved between a first configuration and a second configuration to couple the portion 3051 of the implant 3050 thereto.

As shown in FIG. 5, the implant 3050 can be moved in the direction of the arrow DD to place the portion 3051 of the implant 3050 in contact with the second portion 3302 or the guide member 3300. For example, in some embodiments, the implant 3050 can be an IUD having a filament portion (e.g., the portion 3051). In such embodiments, the second portion 3302 of the guide member 3300 can be placed in contact with the filament portion and the guide member 3300 (e.g., the first portion 3301 and/or the second portion 3302) can be manipulated to couple the implant 3050 thereto. For example, in some embodiments, the guide member 3300 can include and/or be operably coupled to a bias member that biases or urges the second portion 3302 to move from an open configuration to a closed configuration (e.g., as described above). In this manner, the second portion 3302 of the guide member 3300 can be at least temporarily coupled to the portion 3051 of the implant 3050.

When the implant 3050 is coupled to the guide member 3300, the first portion 3301 of the guide member 3300 can be manipulated to move the guide member 3300 relative to the housing 3100, as indicated by the arrow **EE** in FIG. 6. The movement of the guide member 3300 is such that the implant 3050 moves relative to the housing 3100 to be removably coupled to the distal end portion 3602 of the insertion member 3600, as indicated by the arrow **FF** in FIG. 6. More specifically, the movement of the guide member 3300 in the **EE** direction moves the second portion 3302 of the guide member within the passageway 3118 defined by the housing 3100 and pulls the portion 3051 of the implant 3050 at least partially through and/or into the passageway 3118.

While not shown in FIGS. 4-6, in some embodiments, the delivery device 3000 can further include a manipulator configured to engage and/or manipulate the portion 3051 of the implant 3050 when the guide member 3300 is moved relative to the housing 3100. For example, in some embodiments, the manipulator can be an assembly or mechanism configured to cut the portion 3051 of the implant 3050 when the portion 3051 is moved within, through and/or outside of the passageway 3118. Expanding further, in some embodiments, the manipulator can be at least operably coupled to the guide member 3300 such that when the guide member 3300 is moved relative to the housing 3100, the manipulator is moved relative to the portion 3051 of the implant 3050 to sever the portion 3051. Thus, the portion 3051 of the implant 3050 can be decoupled from the guide member 3050. After the implant 3050 is decoupled from the guide member 3300, the delivery device can be manipulated to deliver the implant 3050 to a target location in any suitable manner, as described herein. For example, in some embodiments, the implant 3050 can be an IUD that includes a filament (e.g., the portion 3051) that is cut to a desired length by the manipulator such that the filament can be accessed after the IUD is placed in or at the fundus of the uterus.

While the guide member 3300 is shown being disposed at a distal end portion of the housing 3100, in other embodiments, the guide member 3300 can be disposed at any position along a length of the housing 3100. Accordingly, the passageway 3118 defined by the housing 3100 can be disposed at any position along the length of the housing 3100 such that the second portion 3302 of the guide member 3300 can be disposed therein. Furthermore, while the passageway 3118 is shown as being substantially linear, in other embodiments, a housing can define a passageway that is substantially nonlinear. In such embodiments, the second portion 3302 of the guide member 3300 can be sufficiently flexible to be moved through the passageway 3118. Similarly, the portion 3051 of the implant 3050 can be sufficiently flexible to be moved through the passageway 3118.

Although not described herein in detail, the delivery device 3000 can include any suitable feature, system, assembly, or subassembly configured to facilitate the placement of an implant at a target location. For example, in some embodiments, the delivery device 3000 can include an actuator assembly that can actuate the guide member 3300 and/or the insertion member 3600, and/or a vacuum assembly configured to temporarily couple the delivery device to a contact surface associated with the target location.

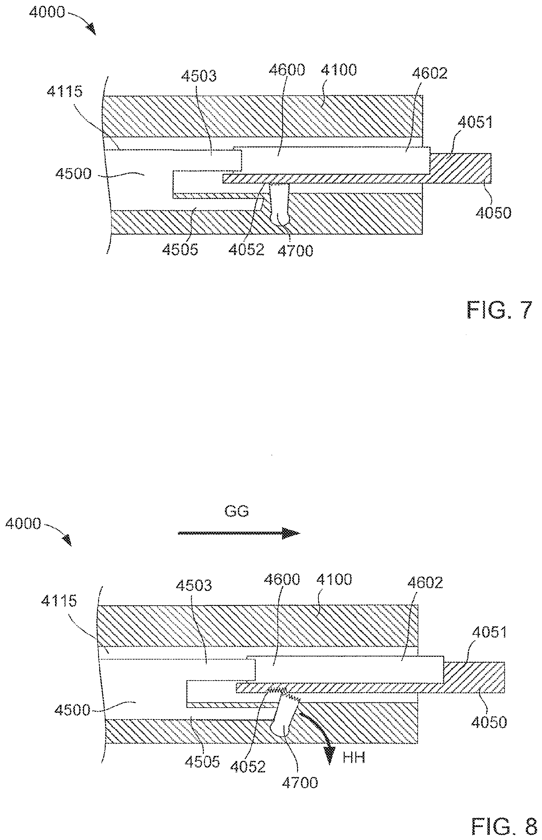

FIGS. 7 and 8 are schematic illustrations of a delivery device 4000 according to an embodiment. The delivery device 4000 is configured to deliver an implant 4050 to a target location (not shown) within a body. For example, in some embodiments, the delivery device 4000 can be used to place an intrauterine device (IUD) in contact with the fundus of a uterus and/or within a uterus. The delivery device 4000 includes a housing 4100, a transfer member 4500, an insertion member 4600, and a manipulator 4700. The housing 4100 defines a passageway 4115 configured to receive at least a portion of the insertion member 4600. The housing 4100 can be any suitable shape, size, or configuration. For example, in some embodiments, at least a portion of the housing 4100 can be substantially cylindrical with an outer diameter suitable for insertion into a body orifice. In some embodiments, the housing 4100 can be substantially similar to the housing 1100 described above with reference to FIGS. 1 and 2 and/or any of the housings described herein. Thus, similar portions of the housing 4100 are not described in further detail herein.

At least a portion of the insertion member 4600 is movably disposed within the passageway 4115 defined by the housing 4100. For example, in some embodiments, a distal end portion 4602 of the insertion member 4600 can extend beyond a distal end portion of the housing 4100, as shown in FIG. 3. Moreover, the distal end portion 4602 of the insertion member 4600 can be removably coupled to the implant 4050 (e.g., an IUD). In this manner, upon delivery of the implant 4050 to the target location, the implant 4050 can be released and/or removed from the insertion member 4600. For example, in some embodiments, the insertion member 4600 can be coupled to a first portion 4051 of the implant 4050 via a friction fit, a snap fit, a press fit, a threaded fit, or the like. In some embodiments, at least a portion of the implant 4050 can be disposed within a portion of the insertion member 4600 (e.g., a lumen or passageway defined by the insertion member 4600 that can receive at least a portion of the implant). The insertion member 4600 can be substantially similar to the insertion member 1600 described above with reference to FIGS. 1 and 2 and/or any insertion member described herein, thus, portions of the insertion member 4600 are not described in further detail herein.

A portion of the insertion member 4600 is coupled to and/or in contact with a first portion 4503 of the transfer member 4500. For example, in some embodiments, a proximal end portion of the insertion member 4600 is in contact with the transfer member 4500. In other embodiments, the proximal end portion of the insertion member 4600 can be disposed in a proximal position relative to the transfer member 4500 (e.g., the first portion 4503 of the transfer member 4500 is in contact with a portion of the insertion member 4600 other than the proximal end portion).

The transfer member 4500 includes the first portion 4503 (that can be placed in contact with the insertion member 4600, as described above) and a second portion 4505 that can be placed in contact with the manipulator 4700, as described further detail herein. The transfer member 4500 can be any suitable shape, size, or configuration. For example, although the transfer member 4500 is shown as being substantially U-shaped, in other embodiments, the second portion 4505 can extend from a surface of the first portion 4503 or vice-versa. Although not shown in FIGS. 7 and 8, the transfer member 4500 can be at least operably coupled to an actuator. For example, in some embodiments, the transfer member 4500 can be directly coupled to the actuator. In other embodiments, the transfer member 4500 can be coupled to the actuator via an intervening structure. The actuator can be any suitable mechanism configured to exert a force on at least the transfer member 4500. In this manner, the actuator can be manipulated to move the transfer member 4500, the insertion member 4600 and/or the implant 4050 relative to the housing 4100, as further described herein.

The manipulator 4700 can be any suitable feature or mechanism that is moved by the second portion 4505 of the transfer member 4500 to engage and/or manipulate a second portion 4052 of the implant 4050. For example, in some embodiments, the manipulator 4700 can be an assembly or mechanism configured to cut the second portion 4052 of the implant 4050. In other embodiments, the manipulator 4700 can be engage the second portion 4052 of the implant 4050 to form stress concentration risers along the second portion 4052. In still other embodiments, the manipulator 4700 can engage the second portion 4052 of the implant 4050 to strip an insulating surface from the second portion 4052.

As shown in FIG. 8, the delivery device 4000 can be placed in a desired position relative to a target location (not shown) and the transfer member 4500 can be moved in the distal direction, as indicated by the arrow **GG.** For example, in some embodiments, a user can manipulate an actuator such that the actuator exerts a force to move the transfer member 4500 in the **GG** direction (e.g., as described detail above with reference to FIGS. 1 and 2). In this manner, the first portion 4503 of the transfer member 4500 moves the insertion member 4600 (and the implant 4050) in the direction of the arrow **GG.** As shown in FIG. 8, the second portion 4505 of the transfer member 4500 actuates the manipulator 4700 such that the manipulator 4700 is moved in the direction of the arrow **HH.** In this manner, the manipulator 4700 can engage and/or manipulate the second portion 4052 of the implant 4050. For example, in some embodiments, the implant 4050 can be an IUD that includes a filament (e.g., the second portion 4052). In such embodiments, the manipulator 4700 can be moved to cut the filament to a desired length such that the filament can be accessed when the IUD is placed in or at the fundus of the uterus. The delivery device 4000 can be further manipulated in any suitable way, as described herein, to deliver the implant 4050 to the target location (not shown in FIGS. 7 and 8).

Although the manipulator 4700 is shown in FIG. 8 as being rotated about an axis to engage the second portion 4052 of the implant 4050, in other embodiments, the transfer member 4500 can be placed in contact with the manipulator 4700 to move the manipulator 4700 in any suitable manner. For example, in some embodiments, a portion of the manipulator 4700 can be disposed in a track (not shown) defined by a portion of the housing 4100 that can define a path along which the manipulator can be moved (e.g., a linear path, a curvilinear path, ctc.). In some embodiments, the manipulator 4700 can be configured to sever the second portion 4052 of the implant at a desired position. In other embodiments, the manipulator 4700 can form one or more stress concentration risers at which the second portion 4052 can break (e.g., the manipulator indirectly severs the second portion 4052).

While not described herein in detail, the delivery device 4000 can include any suitable feature, system, assembly, or subassembly configured to facilitate the placement of an implant at a target location. For example, in some embodiments, the delivery device 3000 can include an actuator assembly configured to actuate the transfer member 4500, a vacuum assembly configured to temporarily couple the delivery device to a contact surface associated with the target location, and/or a guide member configured to releasably couple the implant 4050 to the delivery device 4000.

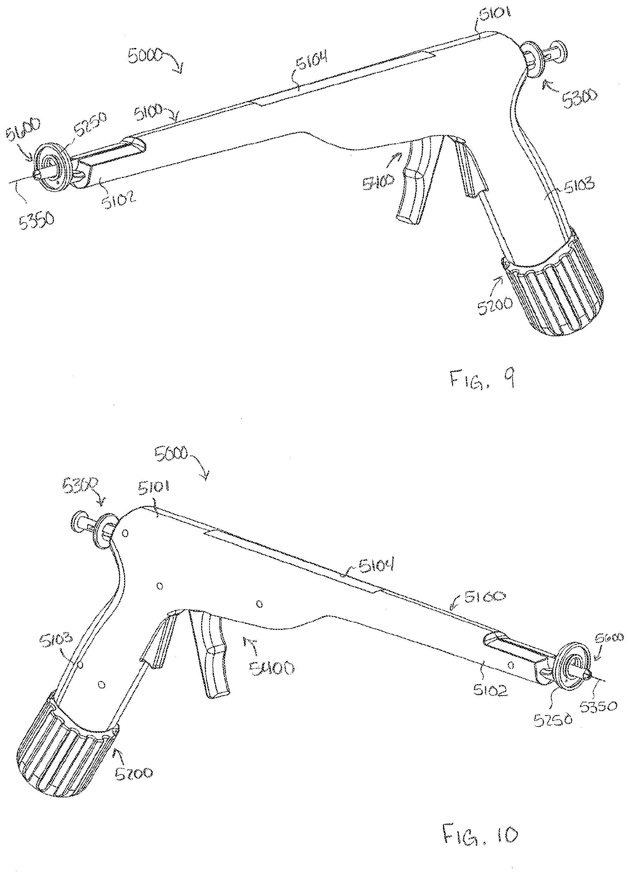

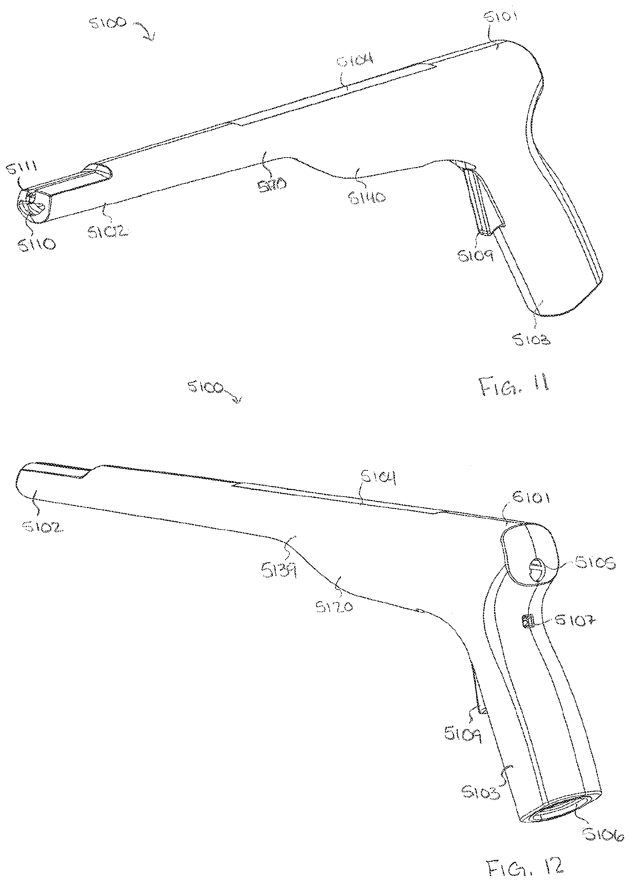

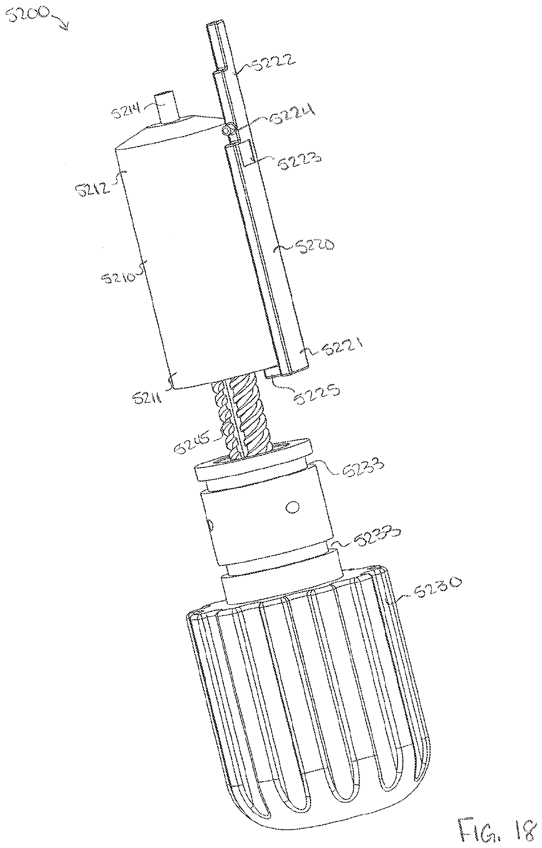

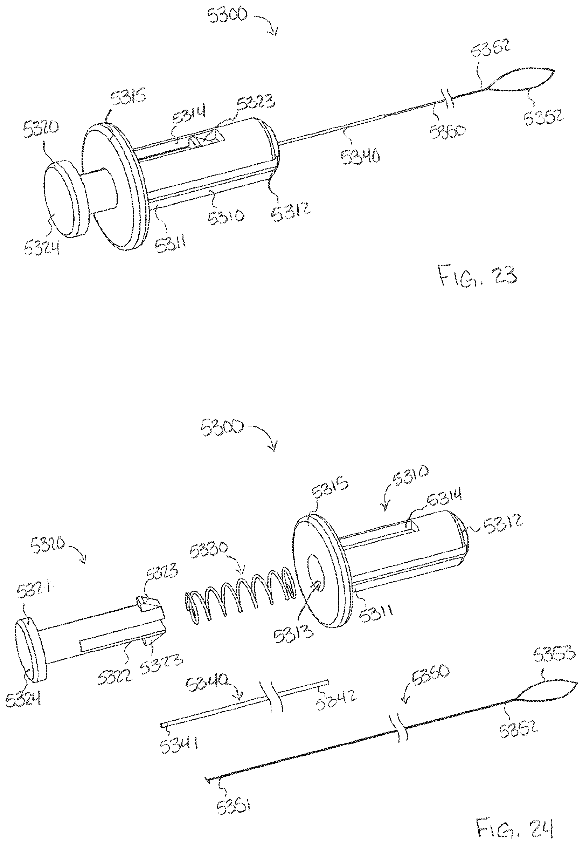

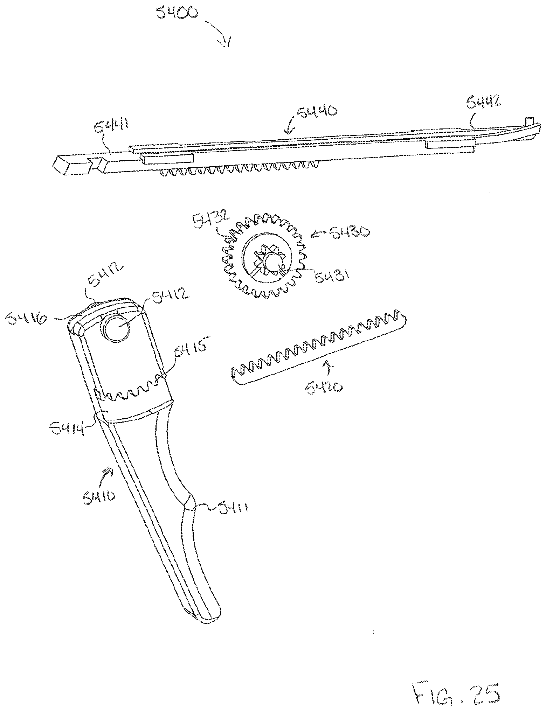

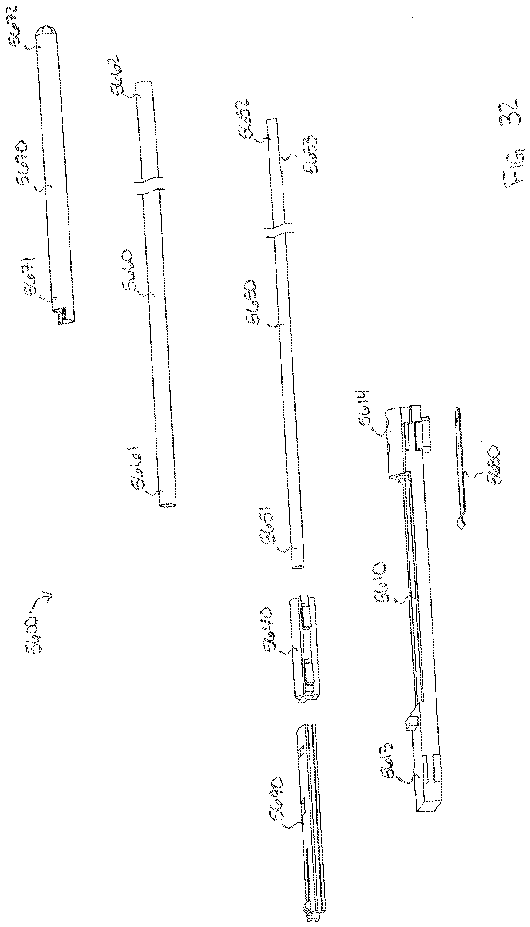

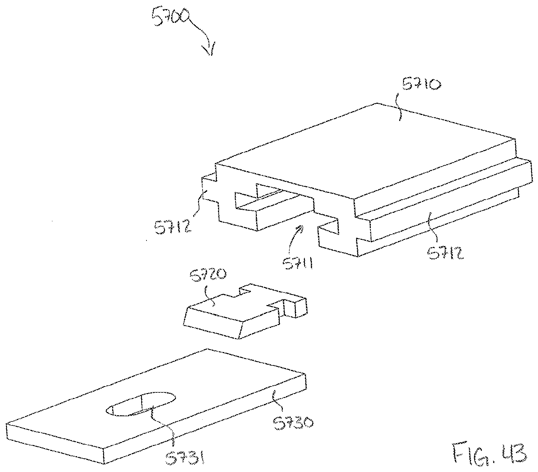

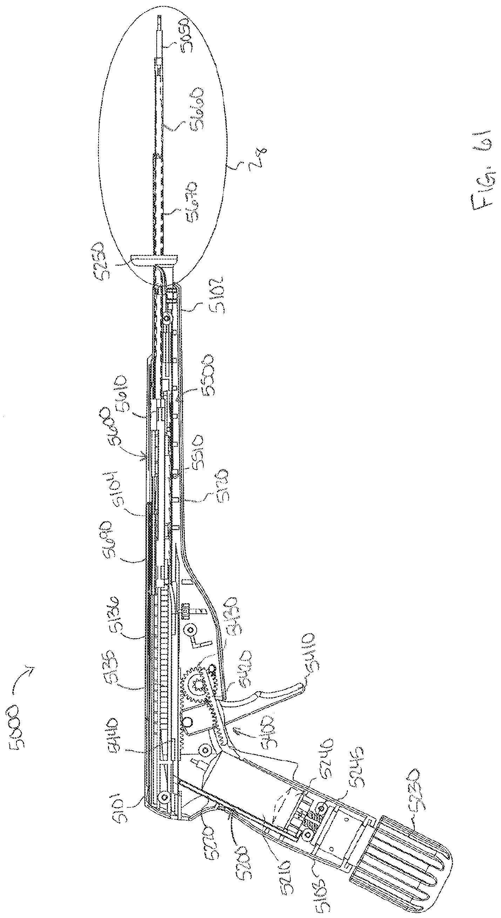

FIGS. 9-63 show a delivery device 5000 according to an embodiment. The delivery device 5000 can deliver an implant 5050 (see e.g., FIGS. 61-63) to a target location within the body. For example, in some embodiments, the delivery device 5000 can be used to place an intrauterine device (IUD) in contact with the fundus of and/or within a uterus. FIGS. 9 and 10 are perspective views of the delivery device 5000 in a first configuration (i.e., prior to use). The delivery device 5000 includes a housing 5100 (see e.g., FIGS. 11-15), a vacuum assembly 5200 (see e.g., FIGS. 18-22), a guide mechanism 5300 (see e.g., FIGS. 23 and 24), an actuator assembly 5400 (see e.g., FIGS. 25-27), a transfer mechanism 5500 (see e.g., 28-31), an insertion assembly 5600 (see e.g., FIGS. 32-42), and a cutter assembly 5700 (see e.g., FIG. 43). A discussion of the components of the delivery device 5000 will be followed by a discussion of the operation of the delivery device 5000.

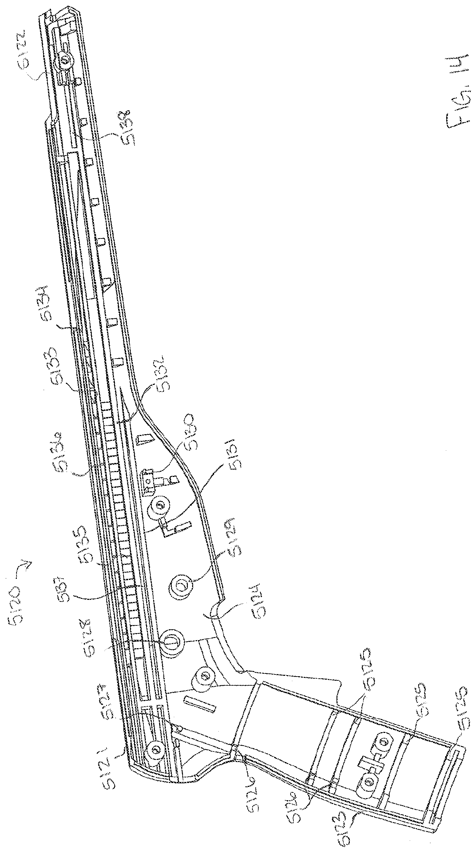

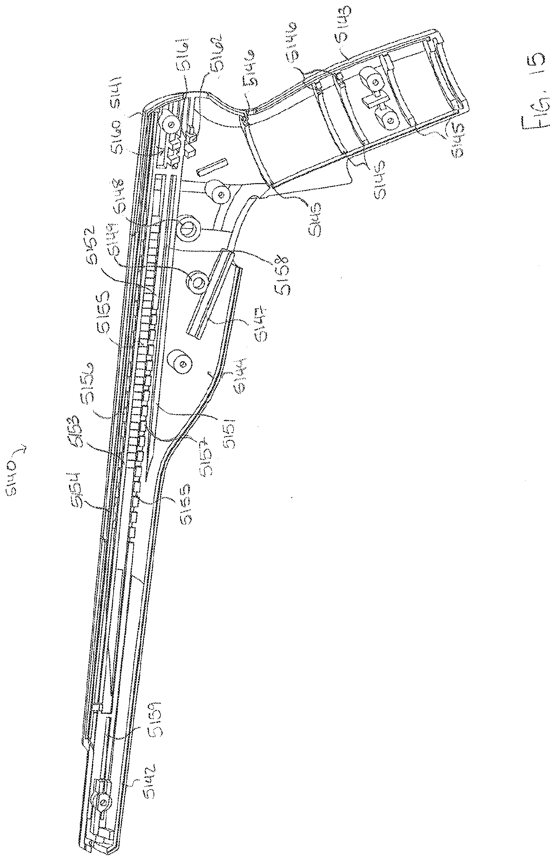

As shown in FIGS. 11-15, the housing 5100 includes a first housing member 5120 (FIG. 14) and a second housing member 5140 (FIG. 15) that are coupled together to form the housing 5100 and collective features thereof. The housing 5100 has a proximal end portion 5101, a distal end portion 5102, and a handle portion 5103. While shown in FIGS. 11-15 as having a specific shape, in other embodiments, the housing 5100 can have any suitable shape, size, or configuration. The housing 5100 defines a status window (or opening) 5104 and an actuator stop 5109. The status window 5104 can allow an operator to monitor the status and/or position of at least a portion of the insertion assembly 5600 contained within the housing 5100. For example, by visually inspecting the status window 5104 an operator (e.g., a technician, physician, nurse, etc.) can determine whether the insertion assembly 5600 has been partially actuated prior to use. In other embodiments, the status window 5104 can provide a visual indication of the distance that a distal end portion of the insertion assembly 5600 has traveled beyond a distal surface of the housing 5100. The actuator stop 5109 can be placed in contact with a portion of the actuator assembly 5400 during use to limit a movement of a portion of the actuator assembly 5400.

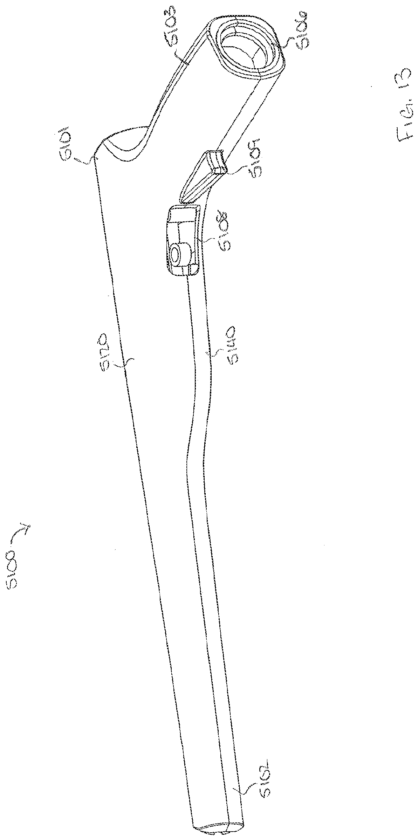

The housing 5100 defines a guide mechanism opening 5105 (see FIG. 12), a vacuum assembly opening 5106, a lock status window 5107 (see FIG. 12), and actuator opening 5108 (see FIG. 13), a vacuum tip opening 5110 (see FIG. 11), and an insertion assembly opening 5111 (see e.g., FIG.). The guide mechanism opening 5105 movably receives a portion of the guide mechanism 5300, the vacuum assembly opening 5106 movably receives a portion of the vacuum assembly 5200, the lock status window 5107 provides an opening to visually inspect the position of a portion of the vacuum assembly 5200, the actuator opening 5108 movably receives a portion of the actuator assembly 5400, the vacuum tip opening 5110 receives a portion of a vacuum tip member 5250, and the insertion assembly opening 5111 movably receives a portion of the insertion assembly 5600.

As shown in FIG. 14, the first housing member 5120 includes an outer surface 5139 and an inner surface 5124, and a proximal end portion 5121, a distal end portion 5122, and a handle portion 5123. The outer surface 5139 (FIG. 12) is substantially smooth surface. In some embodiments, the outer surface 5139 can include any suitable texture, finish, surface, etc. configured to enhance the ergonomics of the delivery device 5000. For example, in some embodiments, the outer surface 5139 at the handle portion 5123 can include a textured finish to provide grip for a user. The outer surface 5139 can also define any number of apertures or openings that can receive mounting hardware (e.g., screws or the like) used to couple the first housing member 5120 to the second housing member 5140.

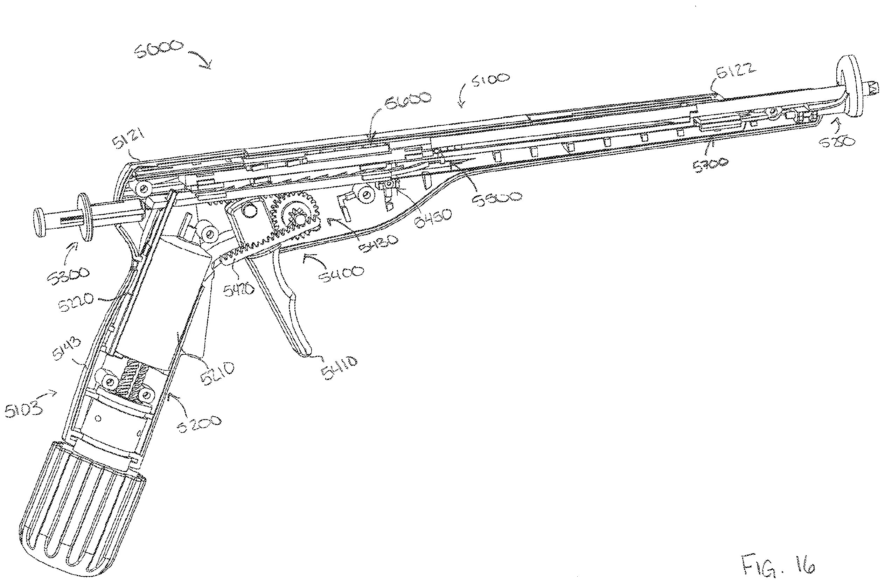

The inner surface 5124 of the first housing member 5120 includes a set of mounting ribs 5125 disposed along the handle portion 5123. More specifically, the mounting ribs 5125 are arranged perpendicularly to a longitudinal centerline (not shown) defined by the handle portion 5123. In this manner, the mounting ribs 5123 can be placed in contact with a portion of the vacuum assembly 5200 (see e.g., FIG. 16) to retain the portion of the vacuum assembly 5200 relative to the handle portion 5123 of the housing 5100. As shown in FIG. 14, the mounting ribs 5125 collectively define at least a portion of a channel and/or series of openings 5126 configured to receive a lock rod 5220 included in the vacuum assembly 5200, as described in further detail herein. More particularly, the mounting ribs 5125 and the corresponding mounting ribs 5145 of the second housing member 5140 (described below with reference to FIG. 15) collectively define the channel.

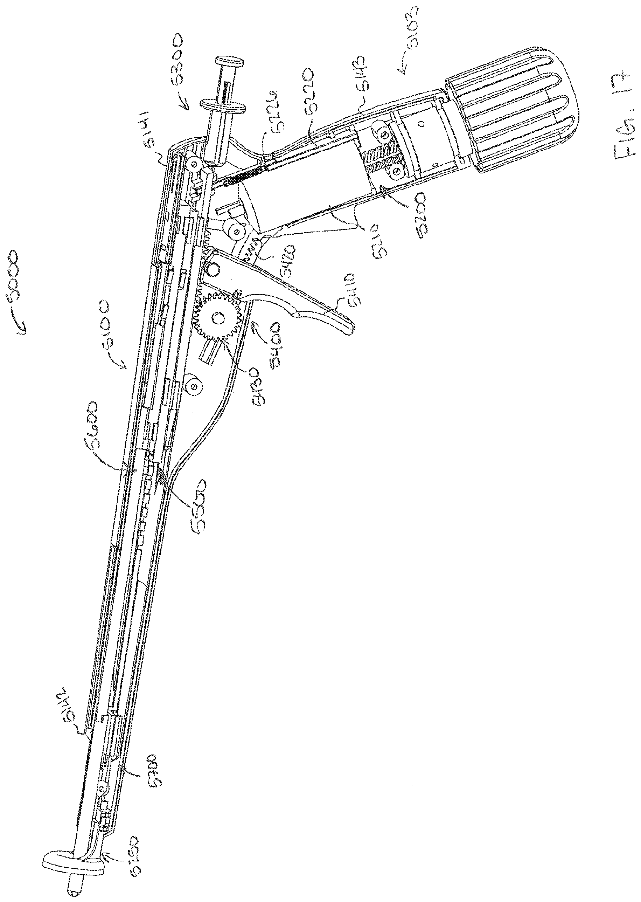

The inner surface 5124 also includes a spring protrusion 5127, a trigger protrusion 5128, a gear protrusion 5129, a pawl mount 5130, a first guide rail 5131, a second guide rail 5132, a third guide rail 5133, a fourth guide rail 5134, a transfer rack 5135, and an insertion rack 5136. The spring protrusion 5127 extends from the inner surface 5124 to receive a portion of a lock rod spring 5226 (see e.g., FIG. 17). The trigger protrusion 5128 is an annular protrusion that extends from the inner surface 5124 to movably receive a pivot protrusion 5412 of a trigger 5410 included in the actuator assembly 5400. Similarly stated, the pivot protrusion 5412 of the trigger 5410 is disposed within an aperture defined by the annular shape of the trigger protrusion 5128. In this manner, the trigger 5410 can pivot about the pivot protrusion 5412 disposed within the trigger protrusion 5128, as further described herein. Similarly, the gear protrusion 5129 is an annular protrusion that extends from the inner surface 5124 to movably receive a portion of a gear member 5430 included in the actuator assembly 5400. In this manner, the gear assembly 5430 can rotate about the portion disposed within the gear protrusion 5128. The pawl mount 5130 is disposed in a distal position relative to the gear protrusion 5129 and is coupled to a pawl 5460. More specifically, the pawl 5460 is movably coupled to the pawl mount 5130 such that the pawl 5460 can pivot relative to the pawl mount 5130. Although not shown in FIG. 14, the pawl 5460 can also be coupled to a spring (e.g., a rotational spring) configured to resist the pivoting motion of the pawl 5460 (e.g., in either a clockwise or counterclockwise direction). Thus, the pawl 5460 can remain in a first position until a force is applied to pivot the pawl 5460, as described in further detail herein.

The first guide rail 5131, the second guide rail 5132, the third guide rail 5133, and the fourth guide 5134 extend from the inner surface 5124 and are each substantially parallel to a longitudinal centerline of the first housing member 5120 between the proximal end portion 5121 and the distal end portion 5122. In this manner, the first guide rail 5131, the second guide rail 5132, the third guide rail 5133, and the fourth guide 5134 are arranged to control movement of various components within the housing 5100. Moreover, the first guide rail 5131, the second guide rail 5132, the third guide rail 5133, and the fourth guide 5134 correspond to and/or interact with the first guide rail 5151, the second guide rail 5152, the third guide rail 5153 and the fourth guide rail 5154, respectively, to control movement of various components within the housing 5100. For example, the first guide rail 5131 can guide the movement of a portion of the actuator assembly 5400, the second guide rail 5132 can guide the movement of a portion of the actuator assembly 5400 and a portion of the transfer mechanism 5500, and the third guide rail 5133 and the fourth guide rail 5134 can guide the movement of a portion of the insertion assembly 5600.

The transfer rack 5135 is disposed between the second guide rail 5132 and the third guide rail 5133 and includes a set of teeth configured to engage a portion of the transfer mechanism 5500 to limit and/or control movement of the transfer mechanism 5500 within the housing 5100. Expanding further, the teeth included in the transfer rack 5135 are substantially uniform, with each tooth being asymmetrical. For example, each tooth included in the transfer rack 5135 includes a first surface having a first slope and a second surface having a second slope much greater than the first slope. The arrangement of each tooth in the transfer rack 5135 is such that the slope angle of the first surface forms an acute (e.g., less than 90°) with the inner surface 5123, whereas the slope angle of the second surface forms a greater angle (e.g., approximately 90°) with the inner surface 5123. Moreover, the slope of each tooth is such that the height of each tooth increases from a first height at a first position to a second height at a second position, distal to the first position. Thus, as described in more detail herein, the transfer rack 5135 can allow a distal movement of at least a portion of the transfer mechanism 5500 relative to the housing 5100 while substantially limiting a proximal movement of at least the portion of the transfer mechanism 5500 relative to the housing 5100.

In some embodiments, the movement of the transfer mechanism 5500 along the length of the transfer rack 5135 (e.g., the amount of movement, the force required to initiate movement, etc.) can be controlled by changing the slope of the first surface and/or the slope of the second surface of each tooth. For example, the slope angles of each tooth included in transfer rack 5135 can be selected to further control movement of the transfer mechanism along the surface of the rack. For example, the force that is exerted to move the transfer mechanism 5500 along a first surface of a tooth with a smaller slope angle is less than the force that is exerted to move the transfer mechanism 5500 along a first surface of a tooth with a larger slope angle. Moreover, the transfer rack 5135 can be configured to selectively allow a movement of the transfer mechanism 5500 along a second surface of a tooth by increasing the second slope angle of the tooth and/or by rounding a leading edge of the tooth (e.g., formed by the intersection of the first surface and the second surface). For example, a second slope angle of a tooth included in transfer rack 5135 can be obtuse (e.g., greater than 90°), thereby allowing for a sequential movement of an object along the second surfaces of each tooth included in the transfer rack 5135. Although described with respect the transfer rack 5135, any of the racks and/or ratchets included in the delivery device 5000 can be modified and/or selected to control a movement of a component along a surface of the rack and/or ratchet.

The insertion rack 5136 is disposed between the third guide rail 5133 and the fourth guide rail 5134 and includes a set of teeth configured to engage a portion of the insertion assembly 5600. As described above with reference to the transfer rack 5135, the insertion rack 5136 can be configured to allow a distal movement of at least the portion of the insertion assembly 5600 relative to the housing 5100 while substantially limiting a proximal movement of at least the portion of the insertion assembly 5600 relative to the housing 5100. In this manner, the device 5000 is configured such that certain portions of the insertion assembly 5600 can reciprocate within the housing 5100, while other portions of the insertion assembly 5600 can move in a single direction.

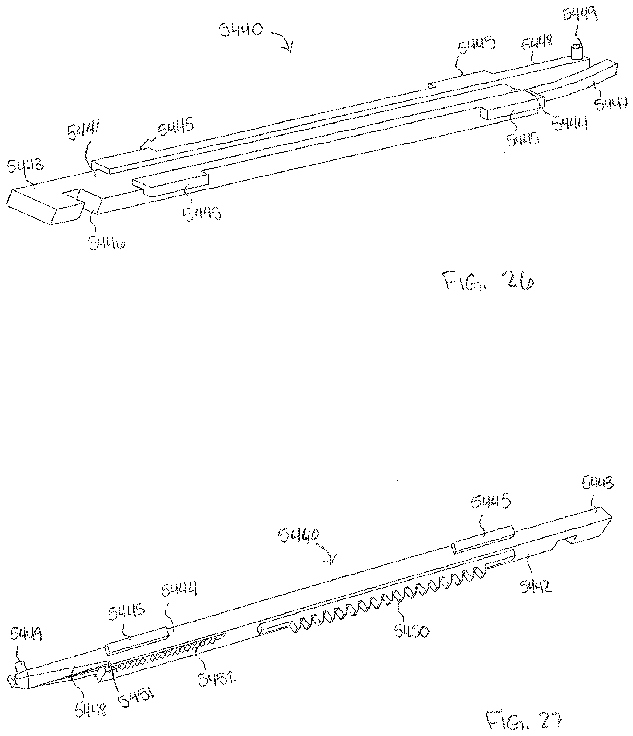

The inner surface 5124 of the first housing member 5120 also defines a drive channel 5137 and a cutter channel 5138. The drive channel 5137 is defined between the first guide rail 5131 and the second guide rail 5132 and can be a substantially smooth channel (e.g., the drive channel 5137 is devoid of a rack, detent or the like). The drive channel 5137 slidably receives a set of guide protrusions 5445 of a drive member 5440 included in the actuator assembly 5400 (see e.g., FIGS. 26 and 27). In this manner, the drive channel 5137 can define a linear path between the first guide rail 5131 and the second guide rail 5132 within which the drive member 5440 can travel, as described in further detail herein. In a similar manner, the cutter channel 5138 slidably receives a guide protrusion 5712 of a cutter housing 5710 included in the cutter assembly 5700 (see e.g., FIG. 43). Thus, the cutter channel 5138 can define a linear path within which the cutter housing 5710 can travel.

As shown in FIG. 15, the second housing member 5140 includes an outer surface 5170 and an inner surface 5144, and a proximal end portion 5141, a distal end portion 5142, and a handle portion 5143. The outer surface 5170 (FIG. 11) is substantially smooth surface. In some embodiments, the outer surface 5170 can include any suitable texture, finish, surface, etc. that can enhance the ergonomics of the delivery device 5000. For example, in some embodiments, the outer surface 5170 at the handle portion 5143 can include a textured finish to provide grip for a user. The outer surface 5170 can also define any number of apertures or openings that can receive mounting hardware (e.g., screws or the like) used to couple the second housing member 5140 to the first housing member 5120.

The inner surface 5144 of the second housing member 5140 includes a set of mounting ribs 5145 disposed along the handle portion 5143. More specifically, the mounting ribs 5145 are arranged perpendicularly to a longitudinal centerline (not shown) defined by the handle portion 5143. In this manner, the mounting ribs 5143 can be placed in contact with a portion of the vacuum assembly 5200 (see e.g., FIG. 16) to retain the portion of the vacuum assembly 5200 relative to the handle portion 5143 of the housing 5100. As shown in FIG. 15, the mounting ribs 5145 define at least a portion of a channel and/or a series of openings 5146 configured to receive a lock rod 5220 included in the vacuum assembly 5200, as described in further detail herein. More particularly, the mounting ribs 5145 and the corresponding mounting ribs 5125 of the first housing member 5120 (described above with reference to FIG. 14) collectively define the channel.

The inner surface 5144 also includes a rack guide 5147, a trigger protrusion 5148, a gear protrusion 5149, a first guide rail 5151, a second guide rail 5152, a third guide rail 5153, a fourth guide rail 5154, a transfer rack 5155, and an insertion rack 5156. The rack guide 5147 extends from the inner surface 5144 and can receive a portion of a drive rack 5420 included in the actuator assembly 5400 (see e.g., FIG. 17). In this manner, the rack guide 5147 can support the drive rack 5420 to provide a path along which and/or within which the drive rack 5420 can move. The trigger protrusion 5148 is an annular protrusion that extends from the inner surface 5144 to movably receive a pivot protrusion 5412 of a trigger 5410 included in the actuator assembly 5400. Similarly stated, the pivot protrusion 5412 of the trigger 5410 is disposed within an aperture defined by the annular shape of the trigger protrusion 5148. In this manner, the trigger 5410 can pivot about the pivot protrusion 5412 disposed within the trigger protrusion 5148, as further described herein. Similarly, the gear protrusion 5149 is an annular protrusion that extends from the inner surface 5144 to movably receive a portion of a gear member 5430 included in the actuator assembly 5400. In this manner, the gear assembly 5430 can rotate about the portion disposed within the gear protrusion 5148.

The first guide rail 5151, the second guide rail 5152, the third guide rail 5153, and the fourth guide 5154 extend from the inner surface 5144 and are each substantially parallel to a longitudinal centerline of the second housing member 5140 between the proximal end portion 5141 and the distal end portion 5142. In this manner, the first guide rail 5151, the second guide rail 5152, the third guide rail 5153, and the fourth guide 5154 are arranged to control movement of various components within the housing 5100. Moreover, the first guide rail 5151, the second guide rail 5152, the third guide rail 5153, and the fourth guide 5154 correspond to and/or interact with the first guide rail 5131, the second guide rail 5132, the third guide rail 5133 and the fourth guide rail 5134, respectively, to control movement of various components within the housing 5100. For example, the first guide rail 5151 can guide the movement of a portion of the actuator assembly 5400, the second guide rail 5152 can guide the movement of a portion of the actuator assembly 5400 and a portion of the transfer mechanism 5500, and the third guide rail 5153 and the fourth guide rail 5154 can guide the movement of a portion of the insertion assembly 5600.

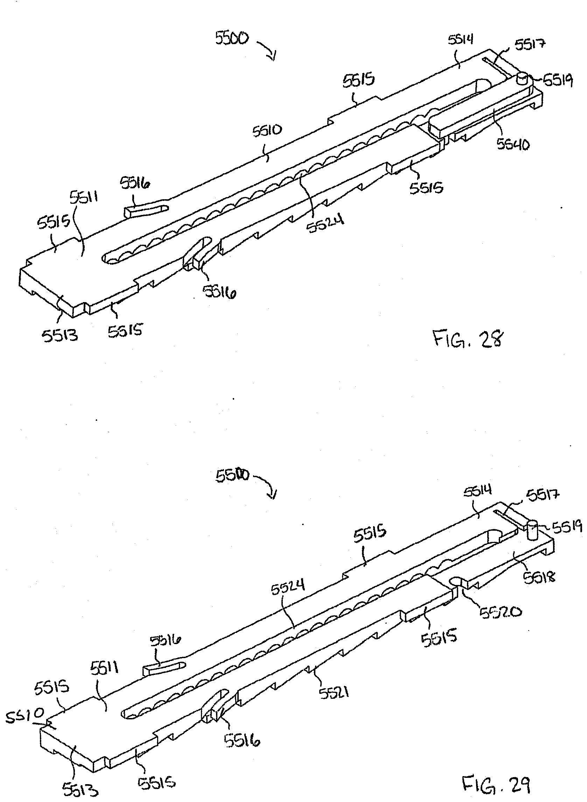

As shown in FIG. 15, the second guide rail 5152 includes a set of lock out detents 5157 that extend along a length of the second guide rail 5152 from a proximal position to a distal position. More specifically, each lock out detent is distinct and independent from the other lock out detents included in the set of lock out detents 5157. The set of lock out detents 5157 can selectively receive a portion of a lock out member 5540 included in the transfer mechanism 5500 (see e.g., FIG. 28). For example, in certain circumstances, the lock out member 5540 can be actuated during a delivery event to prevent injury of the patient, as further described herein. More particularly, when actuated, one of the lock out detents included in the set of lock out detents 5157 can receive a portion of the lock out member 5540 to prevent movement of the transfer mechanism 5500 relative to the housing 5100, as further described herein.

The transfer rack 5155 is disposed between the second guide rail 5152 and the third guide rail 5153 and includes a set of teeth that engage a portion of the transfer mechanism 5500 to limit and/or control movement of the transfer mechanism 5500 within the housing 5100. Moreover, the transfer rack 5155 corresponds to and/or cooperatively functions with the transfer rack 5135 to limit and/or control movement of the transfer mechanism 5500. Expanding further, the teeth included in the transfer rack 5155 are substantially uniform and each tooth has an asymmetric shape. For example, each tooth included in the transfer rack 5155 includes a first surface having a first slope and a second surface having a second slope much greater than the first slope. The arrangement and function of the transfer rack 5155 is similar to the arrangement and function of the transfer rack 5135 included in the first housing member 5120. Therefore, the transfer rack 5155 of the second housing member 5140 is not described in further detail herein.

The insertion rack 5156 is disposed between the third guide rail 5153 and the fourth guide rail 5154 and includes a set of teeth configured to engage a portion of the insertion assembly 5600. Moreover, the insertion rack 5156 corresponds to and/or cooperatively functions with the insertion rack 5136 to limit and/or control movement of the a portion of the insertion assembly 5600. As described above with reference to the transfer rack 5155, the insertion rack 5156 can be configured to allow a distal movement of at least the portion of the insertion assembly 5600 relative to the housing 5100 while substantially limiting a proximal movement of at least the portion of the insertion assembly 5600 relative to the housing 5100. In this manner, the device 5000 is configured such that certain portions of the insertion assembly 5600 can reciprocate within the housing 5100, while other portions of the insertion assembly 5600 can move in a single direction.

The inner surface 5144 of the second housing member 5140 also defines a drive channel 5158 and a cutter channel 5159. The drive channel 5158 is defined between the first guide rail 5151 and the second guide rail 5152 and can be a substantially smooth channel (e.g., does not include a rack or the like). The drive channel 5158 slidably receives a set of guide protrusions 5445 of the drive member 5440 included in the actuator assembly 5400. In this manner, the drive channel 5158 can define a linear path between the first guide rail 5151 and the second guide rail 5152 within which the drive member 5440 can travel, as described in further detail herein. In a similar manner, the cutter channel 5159 slidably receives a guide protrusion 5712 of the cutter housing 5710 included in the cutter assembly 5700. Thus, the cutter channel 5138 can define a linear path within which the cutter housing 5710 can travel.

The inner surface 5144 of the second housing member 5140 includes a set of lock protrusions 5160 that are disposed at the proximal end portion of the housing 5100 and that define a drive slot 5161 and a lock rod slot 5162. More specifically, the drive slot 5161 is substantially parallel to the drive channel 5158 and can receive a proximal end portion 5441 of the drive member 5440 included in the actuator assembly 5400 (see e.g., FIG. 17). Similarly, the lock rod slot 5162 is substantially parallel to the channels 5146 defined by the mounting ribs 5145 and can receive a distal end portion 5222 of the lock rod 5220 included in the vacuum assembly 5200. Furthermore, the lock rod 5220 can be configured to engage the drive member 5440 when the lock rod 5220 is disposed in the lock rod slot 5162 and when the drive member 5440 is disposed in the drive slot 5161 (see e.g., FIG. 16), as further described herein.

As shown in FIGS. 16 and 17, a portion of the vacuum assembly 5200 is disposed within or at the handle portion 5103 of the housing 5100 and the vacuum tip 5250 is disposed at the distal end portion 5102 of the housing 5100. As shown in FIGS. 18-22, the vacuum assembly 5200 includes a vacuum cylinder 5210, the lock rod 5220, an engagement member 5230, a threaded insert 5235, a plunger 5240, a threaded rod 5245, and the vacuum tip 5250. While not shown in FIGS. 9-63, the delivery device can include any suitable tubing configured to fluidically couple the vacuum tip 5250 to the vacuum cylinder 5210, as described in further detail herein.

The vacuum cylinder 5210 includes a proximal end portion 5211 and a distal end portion 5212 and defines an inner volume 5213 therebetween. The vacuum cylinder 5210 can be any suitable shape, size, or configuration. For example, in some embodiments, the vacuum cylinder 5210 can have a shape and size that substantially correspond to a space defined by the handle portion 5103 of the housing 5100. While the vacuum cylinder 5120 is shown as being substantially cylindrical (i.e., in shape), in other embodiments, the vacuum cylinder 5210 can be, for example, oblong, elliptical, or any suitable polygonal shape. The distal end portion 5212 of the vacuum cylinder 5210 is substantially closed and includes a port 5214 that can be coupled to the tubing (not shown) to fluidically couple the vacuum cylinder 5210 to the vacuum tip 5250. The proximal end portion 5213 of the vacuum cylinder 5210 is open such that the inner volume 5213 can receive at least a portion of the plunger 5240 and the threaded rod 5245.

The lock rod 5220 is coupled to the vacuum cylinder 5210 and can be moved between a first (or locked) position and a second (or unlocked) position relative the vacuum cylinder 5210, as described in further detail herein. The lock rod includes a proximal end portion 5221, a distal end portion 5222 and a status indicator 5223. The proximal end portion 5221 includes a tab 5225 that extends perpendicularly from the proximal end portion 5221 such that at least a portion of the tab 5255 is disposed about the inner volume 5213 of the vacuum cylinder 5120. The distal end portion 5222 of the lock rod includes a spring protrusion 5224 that can receive a portion of lock rod spring 5226 described above. In this manner, the lock rod spring 5226 can urge and/or at least temporarily retain the lock rod 5222 in its first (locked) position relative vacuum cylinder 5210. The distal end portion 5222 is further configured to be disposed within the lock rod slot 5162 when the lock rod 5220 is in its first position relative to the vacuum cylinder 5210. Moreover, when the lock rod 5220 is in its first position the status indicator 5223 is substantially aligned with the lock status window 5107 defined by the housing 5100. Thus, a user can visually inspect the status and/or position of the lock rod 5220.

The engagement member 5230 of the vacuum assembly 5200 includes an outer surface 5231 and an inner surface 5232. The outer surface 5231 defines a set of mounting slots 5233. As shown in FIGS. 16 and 17, each mounting slot 5233 can receive a mounting rib 5125 of the first housing member 5120 and a mounting rib 5145 of the second housing member 5140. Therefore, when the first housing member 5120 is coupled to the second housing member 5140, the mounting ribs 5125 and 5145 are disposed within the mounting slots 5233 and selectively retain the engagement member 5230 relative to the housing 5100. More specifically, the mounting ribs 5125 and 5145 can collectively limit a movement of the engagement member 5230 in a direction parallel to a longitudinal centerline of the handle portion 5103 while allowing the engagement member 5330 to be rotated relative to the housing 5100, as described in further detail herein.

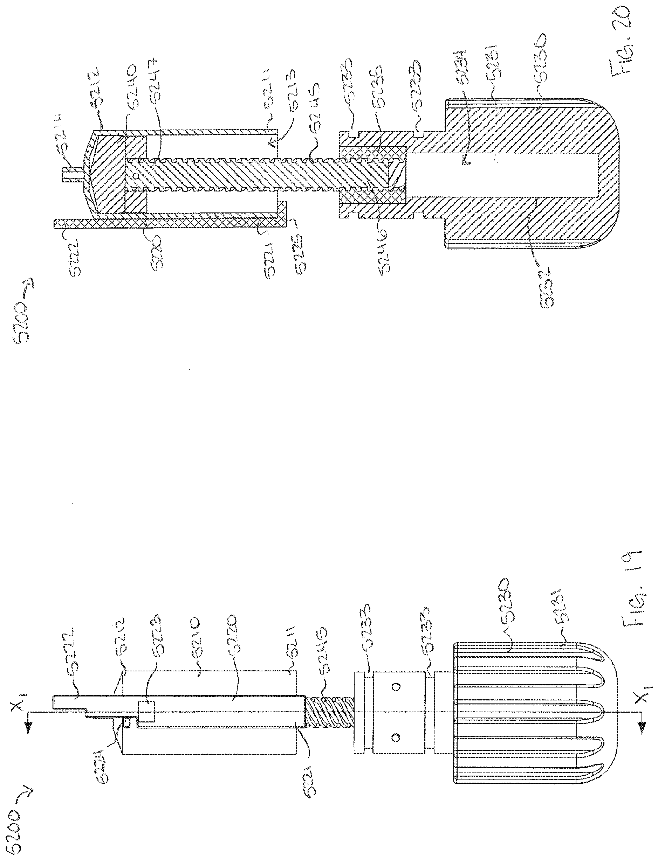

The inner surface 5232 of the engagement member 5230 defines a channel 5234 that receives the threaded insert 5235 and at least a portion of the threaded rod 5245. As shown in FIG. 20, the threaded insert 5235 can be disposed within the channel 5234 and can form a press fit with the inner surface 5232 of the engagement member 5230. Similarly stated, threaded insert 5235 can be fixedly disposed within a portion of the channel 5234. Furthermore, the threaded rod 5245 includes a proximal end portion 5246 that is at least temporarily disposed and/or threadedly engaged within the threaded insert 5235 such that the threads of the threaded insert 5235 engage the threads of the threaded rod 5245. The threaded rod 5245 further includes a distal end portion 5247 that is coupled to the plunger 5240, as described in further detail herein.

The plunger 5240 can be any suitable shape, size, or configuration. For example, in some embodiments, the plunger 5240 can have a diameter that is directly related and/or corresponding to the inner diameter of the vacuum cylinder 5210. In such embodiments, the diameter of the plunger 5240 can be slightly larger than the inner diameter of the vacuum cylinder 5210. In this manner, the sides of the plunger 5240 can engage the inner surface of the vacuum cylinder 5210 to define a fluid tight and/or hermetic seal. In some embodiments, an outer surface of the plunger 5240 can include a set of grooves that define a void such that the side of the plunger 5240 can deform (e.g., be flattened) to occupy a portion of the void when disposed within or moved within the vacuum cylinder 5210. Similarly stated, the grooves can allow the sides of the plunger 5240 to deform such that the diameter can be reduced to be substantially similar to the inner diameter of the vacuum cylinder 5210. Thus, the plunger 5240 can form a substantially fluid tight and/or hermetic seal with the inner surface of the vacuum cylinder 5210.

As described above, the plunger 5240 is coupled to the distal end portion 5247 of the threaded rod 5245. More specifically, the distal end portion 5247 of the threaded rod 5245 can be fixedly coupled to the plunger 5240. In this manner and as described in further detail herein, the engagement member 5230 can be rotated relative to the vacuum cylinder 5210 (rotated relative to the housing 5100 when disposed therein) to move the plunger 5240 within the vacuum cylinder 5210 in a proximal direction. With the plunger 5240 forming a substantially fluid tight and/or hermetic seal with the inner surface of the vacuum cylinder 5210, the movement of the plunger 5240 can produce a negative pressure within the vacuum cylinder 5210 and thus, exerts a suction force on the port 5214, as described in further detail herein. In addition, when the plunger 5240 is moved in the proximal direction relative to the vacuum cylinder 5210 through a predetermined distance, a portion of the plunger 5240 is placed in contact with the tab 5225 of the lock rod 5220. Accordingly, further movement in the proximal (or downward, as shown in the drawings) direction moves the lock rod 5220 from its first position to its second position relative to the vacuum cylinder 5210.

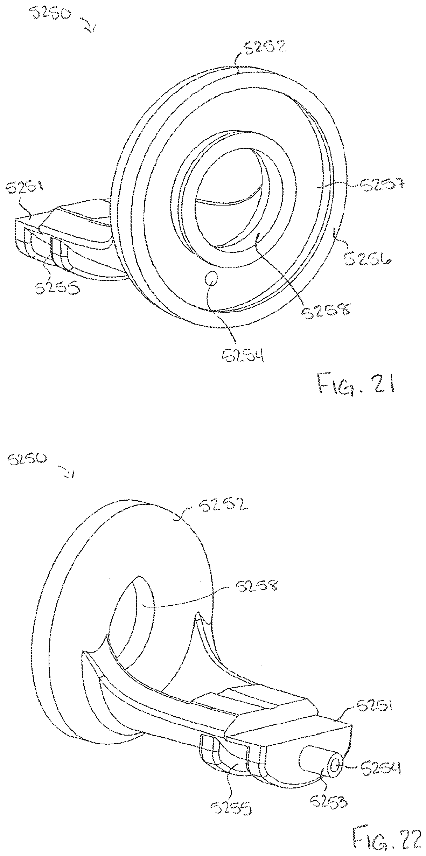

As described above, the vacuum tip 5250 is disposed at a distal end portion 5102 of the housing 5100 and is in fluid communication with the vacuum cylinder 5210 via one or more tubes (or other lumen-defining member). As shown in FIGS. 21 and 22, the vacuum tip 5250 includes a proximal end portion 5251 and a distal end portion 5252. The proximal end portion 5251 of the vacuum tip 5250 includes a port 5253 that can be coupled to the tubing (not shown) to fluidically couple the vacuum tip 5250 to the vacuum cylinder 5250. The proximal end portion 5251 also defines a mounting slot 5255 that can be placed in contact with a distal surface of the housing 5100 to couple the vacuum tip 5250 thereto. The distal end portion 5252 of the vacuum tip 5250 is substantially annular and defines an insertion member opening 5258 that can movably receive a portion of the insertion assembly 5600. The distal end portion 5252 includes a distal surface 5256 that defines a vacuum channel 5257 that substantially circumscribes the insertion member opening 5258. As shown, the vacuum tip 5250 further defines a lumen 5254 that extends through the port 5253 and the distal surface 5256 such that the port 5253 is in fluid communication with the vacuum channel 5257. In this manner, the vacuum cylinder 5210 can be in fluid communication with the vacuum channel 5257 such that when the plunger is moved in the proximal direction, a negative pressure (i.e., suction) is applied within the vacuum channel 5257. Thus, the distal surface 5256 can be placed in contact with a surface within the body and can transfer at least a portion of the suction force on the surface to couple the delivery device 5000 thereto, as described in further detail herein.

FIGS. 23 and 24 show the guide mechanism 5300. The guide mechanism 5300 includes a base 5310, an activator 5320, a bias member 5330 (e.g., a compression spring), a sheath 5340, and a filament 5350. The guide mechanism 5300 is movably disposed, at least partially, within the housing 5100. More specifically, the base 5310 is movably disposed within the guide mechanism opening 5105 of the housing 5100 (see e.g., FIGS. 16 and 17). In addition, a portion of the filament 5350 movably disposed within the housing 5100 such that the distal end portion 5232 of the filament 5350 can at least temporarily extend beyond a distal end portion 5102 of the housing 5100 (see c.g., FIGS. 9 and 10) to be coupled to the implant 5050, as described in further detail herein.

The base 5310 of the guide mechanism 5310 includes a proximal end portion 5311 and a distal end portion 5312 and defines a set of slots 5314 that can receive a portion of the activator 5320. The proximal end portion 5311 of the base 5310 includes an engagement flange 5315 and defines an opening 5313. The engagement flange 5315 can be manipulated by a user to move the base 5310 relative to the housing 5100. The opening 5313 can movably receive the bias member 5330 and a portion of the activator 5320 such that the bias member 5330 and the activator can move between a first configuration (placing the distal end portion 5322 in the closed or collapsed configuration) and a second configuration (placing the distal end portion 5322 in the opened or expanded configuration). Moreover, the opening 5313 can extend through the distal end portion 5312 with a diameter that is smaller than the diameter of the opening 5313 at the proximal end portion 5311. In this manner, a portion of the filament 5350 can pass through the distal end portion 5312 of the base 5310 to couple to the activator 5320. This arrangement also provides a shoulder within the distal end portion 5312 against which the bias member 5330 is in contact when the bias member 5330 is disposed within the opening 5313.

The activator 5320 includes a proximal end portion 5321 and a distal end portion 5322. The proximal end portion 5321 includes an engagement flange 5324 that can be manipulated by a user to move the activator 5320 relative to the base 5310. The distal end portion 5322 of the activator 5320 is bifurcated and includes a set of tabs 5323 that can be movably disposed within the slots 5314 defined by the base 5310. Expanding further, by bifurcating the distal end portion 5322 of the activator 5320, the distal end portion 5322 can deform to allow at least the distal end portion 5322 to be inserted into the opening 5313. When the tabs 5323 are disposed within the slots 5314 of the base 5310, the distal end portion 5322 can return to its undeformed configuration, thereby retaining the activator 5320 within the base 5310. This arrangement prevents the activator 5320 from being separated from and/or moved out of the base 5310 when the force from the bias member 5330 is exerted upon the activator 5320. In addition, a proximal surface of the tabs 5233 can be placed in contact with a distal surface of the engagement flange to limit the movement of the activator 5320 relative to the base 5310.

The sheath 5340 includes a proximal end portion 5341 and a distal end portion 5342. The sheath 5340 can be disposed about at least a portion of the filament 5350 such that the filament 5350 can move relative to the sheath 5340. The proximal end portion 5341 of the sheath 5340 is coupled to the distal end portion 5322 of the base 5310. The distal end portion 5342 of the sheath 5340 can be configured to manipulate and/or act upon a portion of the filament 5350 when the filament 5350 is moved relative to the sheath 5340, as described in further detail herein.

The filament 5350 includes a proximal end portion 5351 and a distal end portion 5232. The filament 5350 can be formed from any suitable material. In this manner, the filament 5350 can be sufficiently flexible to be disposed within a tortuous path defined by the housing 5100 and/or the insertion assembly 5600. For example, in some embodiments, the filament 5350 can be disposed within a passageway (not shown) defined by the housing 5100 that is non-linear and can further be threaded through and/or adjacent a portion of the cutter assembly 5700 and/or the insertion assembly 5600. The proximal end portion 5351 of the filament 5350 is coupled to the activator 5320 of the guide mechanism 5300. Thus, the filament 5350 can be moved with the activator 5320 relative to the housing 5100 and/or the sheath 5340. The distal end portion 5352 of the filament 5350 can extend, at least temporarily beyond the distal end portion of the housing 5100 and a distal end portion of the insertion assembly 5600 to be coupled to a portion of the implant 5050. For example as shown in FIGS. 23 and 24, the distal end portion 5352 of the filament 5350 forms a snare 5353 (e.g., a loop) that can receive a portion of the implant 5050 (e.g., a retraction filament of an IUD). Expanding further, a user can manipulate the activator 5320 to move the activator 5320 in a distal direction, thereby placing the bias member 5330 in a compressed configuration. In addition, the distal movement of the activator 5320 moves the filament 5350 distally relative to the sheath 5340 such that the snare 5353 can move beyond the distal end portion 5342 of the sheath 5340 and into an open (or expanded) configuration. When the portion of the implant 5050 is disposed within the snare 5353, the user can disengage the activator 5320 and the bias member 5330 can move to its uncompressed configuration. Thus, the filament 5350 moves in a proximal direction relative to the sheath 5340 and the distal end portion 5342 of the sheath 5340 can engage the snare 5353 to move the snare 5353 to a closed (or collapsed) configuration, thereby coupling the portion of the implant 5050 to the filament 5350, as described in further detail herein.

FIGS. 25-27 show the actuator assembly 5400. The actuator assembly 5400 includes the trigger 5410, the drive rack 5420, the gear member 5430, and the drive member 5440. The actuator assembly 5400 can be manipulated by a user to exert a force on the transfer member 5500, thereby actuating the device 5000 (as described herein). More specifically, the actuator assembly 5400 is disposed within the housing 5100 (see e.g., FIG. 16 and 17) such that the drive member 5440 is in contact with a portion of the transfer member 5500 and such that a portion of the trigger 5410 extends through the actuator opening 5108 defined by the housing 5100 (see e.g., FIGS. 9, 10 and 13). Thus, the user can engage the trigger 5410 to actuate the actuator assembly 5400 and move the transfer member 5500 relative to the housing 5100, as described in further detail herein.

The trigger 5410 of the actuator assembly 5400 includes an engagement portion 5411 and the pivot protrusions 5412, and defines a drive rack channel 5414 and a slot 5416. The trigger 5410 is partially disposed within the housing 5100 such that the engagement portion 5411 extends outside of the housing 5100 through the actuator opening 5108. As described above, the pivot protrusions 5412 can be rotatably disposed within the aperture defined by the trigger protrusion 5128 of the first housing member 5120 and the aperture defined by the trigger protrusion 5148 of the second housing member 5140. In this manner, the trigger protrusion 5128 and the trigger protrusion 5148 can collectively limit linear movement of the trigger 5410, and can collectively allow a pivoting movement of the trigger 5410.

With the engagement portion 5411 disposed substantially outside of the housing 5100, the user can engage the engagement portion 5411 of the trigger 5410 to actuate the actuator assembly 5400. Although not shown in FIG. 25, the engagement portion 5411 of the trigger 5410 can include any suitable texture, finish, surface, etc. configured to enhance the ergonomics of the trigger 5410. Similarly, the engagement portion 5411 can be any suitable shape configured to enhance the ergonomics of the trigger 5410. For example, as shown in FIG. 25, the engagement portion 5411 can define one or more recesses, detents and/or contours that can correspond to a placement of a user's fingers when the user grips the trigger 5410.

The drive channel 5414 of the trigger 5410 movably receives the drive rack 5420. Similarly stated, the drive rack 5420 can move relative to the trigger 5410 when disposed within the drive channel 5414. The drive channel 5414 can be any suitable configuration. For example, the drive channel 5414 can be substantially arced with a radius of curvature that is sufficiently large to allow the drive rack 5410 to move in a linear path along a surface of the rack guide 5147 included in the second housing member 5140 (see e.g., FIGS. 15 and 17) when the trigger 5410 is moved.

The trigger 5410 also includes a gear segment 5415 that extends into and/or at least partially defines the drive channel 5414. The gear segment 5415 is an arced segment and includes a set of teeth that are substantially uniform, with each tooth being substantially symmetrical. In use, the gear segment 5415 engages a portion of the drive rack 5420 to advance the drive rack 5420 along the linear path defined by the rack guide 5147. For example, as shown in FIG. 25, the drive rack 5420 includes a set of teeth that are substantially uniform, with each tooth being substantially symmetrical. In this manner, the drive rack 5420 can be disposed within the drive channel 5414 such that the teeth of the drive rack 5420 mesh (e.g., at least one tooth of the drive rack 5420 is disposed within a space defined between adjacent teeth of the gear segment 5415) with the teeth of the gear segment 5415. Thus, when the user manipulates the trigger 5410 to pivot the trigger 5410 about the pivot protrusions 5412, the gear segment 5415 sequentially engages the teeth of the drive rack 5420 to advance the drive rack 5420 along the linear path defined by the surface of the rack guide 5147.

Although not shown in FIG. 25, the trigger 5410 can include a protrusion or hook that can be coupled to a trigger spring which, in turn, is coupled to a portion of the housing 5100. In this manner, the trigger spring can be configured to pivot the trigger 5410 within and/or about the trigger protrusions 5128 and 5148 (described above) after the trigger 5410 has been manipulated by the user. For example, the user can manipulate the trigger 5410 by exerting a force on the engagement portion 5411 (e.g., squeezing the trigger 5410) such that the trigger 5410 pivots form a first position toward a second position (e.g., toward the trigger stop 5109 of the housing 5100), thereby placing the trigger spring in tension (e.g., moving the trigger spring to an extended configuration). After the trigger 5410 is placed in contact with the trigger stop 5109, the user can remove at least a portion of the force (e.g., by squeezing with less force or releasing the trigger 5410) to allow the trigger spring to exert a force (e.g., the kinetic energy of the trigger spring moving from the extended configuration to a resting, compressed configuration) on the trigger 5410 that pivots the trigger 5410 away from the trigger stop 5109 toward the first position. Thus, the trigger 5410 can be repeatedly manipulated to actuate the actuator assembly 5400, as described in further detail below.

The gear member 5430 includes a rack pinion 5431 configured to engage the drive rack 5420 and a drive pinion 5432 configured to engage the drive member 5440. As described above, a portion of the gear member 5430 is movably disposed within the apertures defined by the gear protrusion 5129 of the first housing member 5120 and the gear protrusion 5149 of the second housing member 5140 such that the rack pinion 5431 is in contact with the drive rack 5420 and the drive pinion 5432 is in contact with the drive member 5440. The rack pinion 5431 includes a set of teeth that are substantially uniform, with each tooth being substantially symmetrical. Similarly, the drive pinion 5432 includes a set of teeth that are substantially uniform, with each tooth being substantially symmetrical. Moreover, as shown in FIG. 25, the arrangement of the gear member 5430 can be such that a diameter of the rack pinion 5431 is smaller than a diameter of the drive pinion 5432. Accordingly, the number of teeth of the rack pinion 5431 is less than the number of teeth of the drive pinion 5432 (e.g., the gear member 5430 defines a gear reduction wherein the corresponding linear motion for a full rotation of the rack gear 5431 results in a lesser amount of linear motion for the corresponding full rotation of the drive gear 5432). Thus, the output torque of the gear member 5430 and/or the ratio of linear motion of the drive rack 5420 to the drive member 5440 can be controlled by increasing or decreasing the ratio of diameters between the rack pinion 5431 and the drive pinion 5432 (and, therefore, increasing or decreasing the number of teeth included in rack pinion 5431 and/or the drive pinion 5432).

As described above, the rack pinion 5431 engages the drive rack 5420. More specifically, the teeth of the rack pinion 5431 are configured to mesh with the teeth of the drive rack 5431 such that as the drive rack 5420 is moved along the linear path defined by the rack guide 5147, the teeth of the rack pinion 5431 are sequentially advanced along the teeth of the drive rack 5420. Thus, the movement of the drive rack 5420 rotates the gear member 5430 within and/or about an axis defined by the apertures defined by the gear protrusion 5129 and the gear protrusion 5149. Furthermore, with the drive pinion 5432 in contact with a portion of the drive member 5440, the drive pinion 5432 is rotated along a surface of the drive member 5440, as described in further detail herein.

The drive member 5440 of the actuator assembly 5400 is configured to move within the housing 5100 between a first position (e.g., a proximal position) and a second position (e.g., a distal position). Similarly stated, the drive member 5440 is configured to reciprocate within the housing 5100. As shown in FIG. 26 and 27, the drive member 5440 has a first side (or surface) 5441 and a second side (or surface) 5442, and a proximal end portion 5443 and a distal end portion 5444. The first side 5441 (e.g., a top side) includes the set of guide protrusions 5445. The guide protrusions 5445 are configured to be movably disposed within the drive channel 5137 of the first housing member 5120 and the drive channel 5158 of the second housing member 5140. Thus, movement of the drive member 5440 can be substantially limited to a path defined by the drive channel 5137 and the drive channel 5158. Similarly stated, the first guide rail 5131 and the second guide rail 5132 of the inner surface 5124 and the first guide rail 5151 and the second guide rail 5152 of the inner surface 5144 engage the guide protrusions 5445 to allow the drive member 5440 to move in a proximal direction and a distal direction while substantially limiting a movement in any other direction.

As shown in FIG. 27, the second side 5442 (e.g., a bottom side) includes an actuator rack 5450 and an engagement rack 5452. The actuator rack 5450 includes a set of teeth that are substantially uniform, with each tooth being substantially symmetrical. The teeth of the actuator rack 5450 are configured to mesh with the teeth of the drive pinion 5432. Thus, when the trigger 5410 is manipulated by a user, the drive rack 5420 is advanced along the linear path defined by the rack guide 5147 to rotate the gear member 5430, which, in turn, is advanced along and/or rotated within the actuator rack 5450. With the gear member 5430 disposed within the apertures defined by the gear protrusion 5129 and the gear protrusion 5149, the rotation of the drive pinion 5432 moves the drive member 5440 relative to the gear member 5430. Furthermore, the slot 5416 defined by the trigger 5410 is arranged to provide a space through which the actuator rack 5450 can move. Therefore, when the trigger 5410 is moved from its first position towards its second position (e.g., towards the trigger stop 5109 of the housing 5100), the drive member 5440 is advanced in the distal direction. When the trigger is moved from its second position towards its first position, the drive member 5440 is moved in a proximal direction, as described in further detail herein.

The engagement rack 5452 is disposed within a recess 5451 defined by the second side 5442 of the drive member 5440. The engagement rack 5452 includes a set of teeth that are substantially uniform, with each tooth being substantially symmetrical. The teeth of the engagement rack 5452 can be placed in contact with the pawl 5460 (described above) such that when the drive member 5440 is moved relative to the housing 5100, the engagement rack 5452 is moved relative to the pawl 5460. In this manner, the pawl 5460 and the engagement rack 5452 can be configured to collectively control a movement of the drive member 5440 relative to the housing 5100, as described in further detail herein.

The proximal end portion 5443 of the drive member 5440 defines a lock rod slot 5446. The lock rod slot 5446 receives the distal end portion 5222 of the lock rod 5220 when the drive member 5440 is in its first position and when the lock rod 5220 is in its first position relative to the vacuum cylinder 5210, as described above. More specifically, the proximal end portion 5443 of the drive member 5440 is disposed within the drive slot 5161 defined by the second housing portion 5140 such that the lock rod slot 5446 of the drive member 5440 is aligned with the lock rod slot 5162 defined by the second housing portion 5140. Thus, the lock rod 5220 can retain the drive member 5220 in its first position when the lock rod 5220 is in its first position relative to the vacuum cylinder 5210 (see e.g., FIGS. 16 and 17). In this manner, movement of the drive member 5440 is limited and/or prevented when the vacuum cylinder 5210 has not been fully actuated (such that the lock rod 5220 is in its second position).

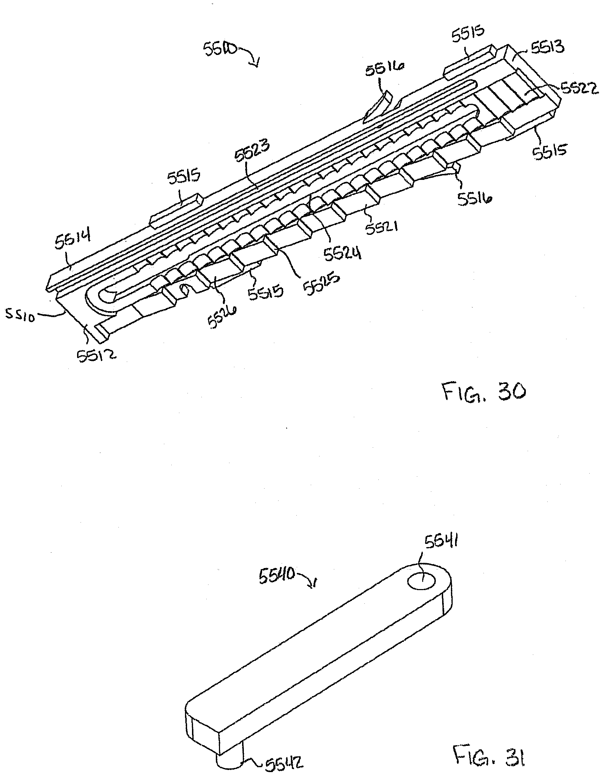

The distal end portion 5444 of the drive member 5440 includes a push arm (or pawl) 5447 and an engagement arm 5448. The push arm 5447 can be placed in contact with a portion of the transfer mechanism 5500 to move the transfer mechanism 5500 in a distal direction, as described in further detail herein. The engagement arm 5448 includes an engagement protrusion 5449 that is movably disposed within a engagement slot 5523 defined by a portion of the transfer mechanism 5500 (see e.g., FIG 30). In this manner, the engagement protrusion 5449 can be placed in contact with a set of walls defining the engagement slot 5523 to limit the distal movement of the transfer mechanism 5500 relative to the drive member 5440 and/or to move the transfer mechanism 5500 in a proximal direction relative to the housing 5100, as described in further detail herein.

FIGS. 28-31 show the transfer mechanism 5500. The transfer mechanism 5500 is disposed within the housing 5100 (see e.g., FIG. 16 and 17) and can be moved by the drive member 5440 of the actuator assembly 5400 when the actuator assembly 5400 is actuated. In this manner, the transfer mechanism 5500 can transfer at least a portion of the force exerted by the actuator assembly 5400 to the insertion assembly 5600 to facilitate the delivery of the implant 5050 to the target location. The transfer mechanism (or assembly) 5500 includes a transfer member 5510 and a lock out member 5540. Although shown as being constructed from two components that are separately constructed, in other embodiments, a transfer mechanism can be a single and/or monolithically constructed part.

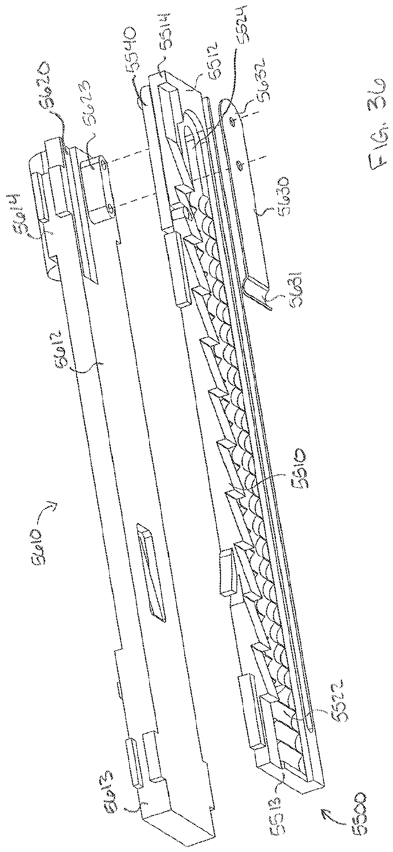

As shown in FIGS. 29 and 30, the transfer member 5510 has a first side 5511 and a second side 5512, a proximal end portion 5513 and a distal end portion 5514, and defines a channel 5524. As described in further detail herein, a carrier 5610 included in the insertion assembly 5600 is disposed on and/or in contact with the first side 5511 of the transfer member 5510 such that the carrier can move concurrently with the transfer member 5510. Furthermore, the carrier 5610 includes a mount protrusion 5623 that is movably disposed within the channel 5524 (see e.g., FIG. 36).

The first side 5511 (e.g., a top side) includes a set of guide protrusions 5515, a set of retraction protrusions 5516, and a mounting protrusion 5519. The first side 5511 also defines a spring slot 5517, a recessed portion 5518, and a notch 5520. The guide protrusions 5515 are configured to be movably disposed on a surface (e.g., a top surface) of the second guide rail 5132 of the first housing member 5120 and a surface (e.g., a top surface) of the second guide rail 5152 of the second housing member 5140. Thus, movement of the drive member 5440 can be substantially limited to a path along the surface of the second guide rail 5132 and the surface of the second guide rail 5152.

The retraction protrusions 5516 extend from opposite lateral sides (e.g., left and right sides) of the transfer member 5510, and are in contact with the transfer rack 5135 of the first housing member 5120 and the transfer rack 5155 of the second housing member 5120. As described above, the transfer rack 5135 and the transfer rack 5155 each include a set of asymmetrical teeth that can contact the retraction protrusions 5516 to limit proximal movement of the transfer member 5510 relative to the housing 5100. For example, the transfer racks 5135 and 5155 can limit the proximal movement of the transfer member 5510 within the housing 5100 until a force is applied that is sufficiently large to overcome the friction force and/or engagement between the retraction protrusions 5516 and the second surface (described above) of the transfer racks 5135 and 5155. In a similar manner, a force can be applied that is sufficiently large to deform (e.g., elastically or plastically) the retraction protrusions 5516 such that the retraction protrusions 5516 are disengaged from the transfer racks 5135 and 5155. Thus, the amount force required to deform the retraction protrusions 5516 can be controlled by increasing or decreasing the flexibility of the retraction protrusions 5516 (e.g., increasing the cross-sectional area, adding a discontinuity, or forming the retractions protrusions 5516 from a material that is more or less stiff). This arrangement allows the transfer member 5510 to move within the housing 5100 distally (e.g., to insert the implant 5050), while limiting, at least over a range of motion, proximal movement of the transfer member 5510 within the housing 5100.

The mounting protrusion 5519 extends from the recessed surface 5518 of the first side 5511 of the transfer member 5510. The recessed surface 5518 and the mounting protrusion 5519 each receive and/or engage a portion of the lock out member 5540 (see, e.g., FIGS. 28 and 31). More specifically, the mounting protrusion 5519 is disposed within an aperture 5541 defined by the lock out member 5540, as described in further detail herein. The notch 5520 is disposed on a lateral side of the transfer member 5510 and receives a lock protrusion 5542 of the lock out member 5540, as shown in FIG. 28. The spring slot 5517 receives a portion of a biasing member (not shown) that is configured to move the lock out member 5440 between a first configuration and a second configuration when a maximum amount of "slip" occurs between the carrier 5610 of the insertion assembly 5600 and the transfer member 5510, as described in further detail herein. More particularly, the biasing member, which can be a leaf spring, exerts a force on the lock out member 5540 such that, under certain conditions, the lock out member 5440 rotates relative to the transfer member 5510 about the mounting protrusion 5519.

As shown in FIG. 30, second side 5512 (e.g., a bottom side) of the transfer member 5510 includes a drive portion 5521 and a slip surface 5522, and defines an engagement slot 5523. The drive portion 5521 includes a rack having a set of teeth that are substantially uniform, with each tooth being asymmetrical. Expanding further, each tooth included in the drive portion 5521 includes a first surface 5525 having a slope angle that is greater than a slope angle of a second surface 5526. For example, in some embodiments, the slope angle of the first surface 5525 can be approximately 90°, while the slope angle of the second surface 5526 is much less than 90° (but greater than zero). Moreover, the first surface 5525 can be disposed at a proximal position relative to the second surface 5526. Thus, each tooth included in the drive portion 5521 has a greater height at a proximal end portion than a height at a distal end portion. In this manner, the push arm 5447 of the drive member 5440 can be placed in contact with the first surface 5525 of a tooth included in the drive portion 5521 to move the transfer member 5510 in a distal direction when the drive member 5440 is moved distally, as described above. Thus, the slope of the first surface 5525 of each tooth included in the drive portion 5521 can be sufficiently large such that the push arm 5447 does not slip (i.e., maintains contact) when placed in contact with the first surface 5525. Moreover, the slope of the second surface 5526 can be sufficiently small such that when the drive member 5440 moves in the proximal direction (e.g., when the trigger 5410 is moving from its second position back to its first position), the push arm 5447 can move sequentially along the second surfaces of the teeth included in the drive portion 5521.

The engagement slot 5523 movably receives the engagement protrusion 5449 included in the engagement arm 5448 of the drive member 5440. In this manner, the engagement protrusion 5449 can move within the engagement slot 5523 when the transfer member 5500 is moved relative to the drive member 5440 (or vice versa) within a predetermined range. When the drive member 5540 moves proximally relative to the transfer member 5500 by a predetermined amount, a proximal wall defining the a portion of engagement slot 5523 is placed in contact with the engagement protrusion 5449 to selectively retain the transfer member 5510 relative to the drive member 5440, as described in further detail herein. Similarly stated, when the transfer member 5500 moves distally relative to the drive member 5540, the engagement protrusion 5449 maintains contact between the transfer member 5500 and the drive member 5540. In this configuration, proximal movement of the drive member 5540 results in proximal movement of the transfer member 5500.

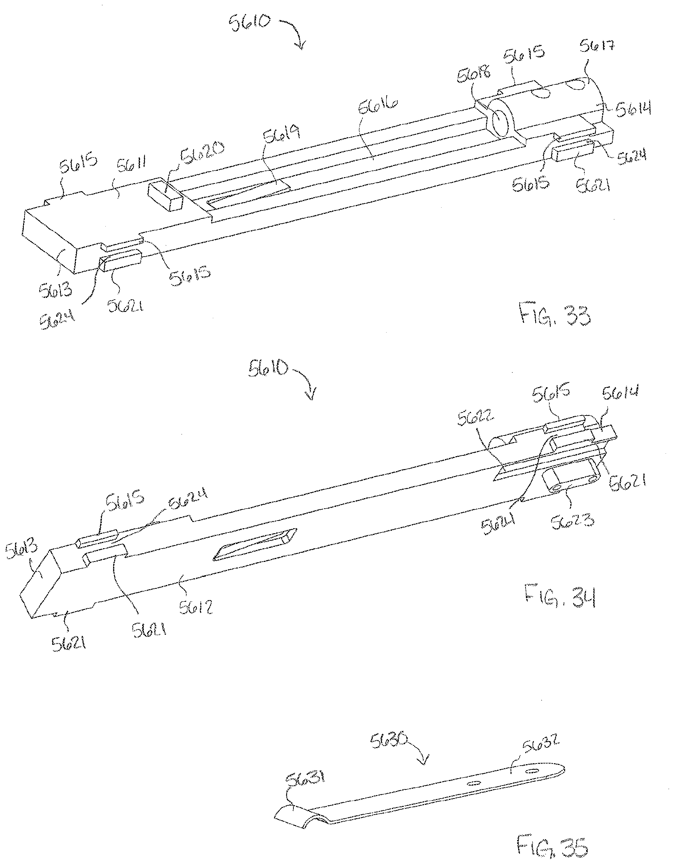

The slip surface 5522 of the transfer member 5510 is selectively placed in contact with a slip member 5630 included in the insertion assembly 5600 (see FIGS. 35 and 36). This arrangement can limit the amount of force transferred from the drive member 5540 to the insertion assembly 5600 and can allow selective relative movement between the transfer member 5510 and the insertion assembly 5600. As shown in FIG. 30, the slip surface 5522 includes a set of detents. The detents can be any suitable configuration. For example, as shown, the detents can be semicircular. In such embodiments, the radius of curvature of the detents and/or the radius of curvature of the edge between adjacent detents can be increased or decreased to control the amount of force exerted to cause the slip member 5630 to move along the slip surface 5522, as described in further detail herein. Although shown as being semicircular, in other embodiments, the detents can be arranged in a similar manner as the drive portion 5521 (e.g., arranged as a rack). In such embodiments, the slope angle of the teeth can be increased or decreased to control the amount of force exerted to cause the slip member 5630 to move along the slip surface 5522.

FIGS. 32-42 show the insertion assembly 5600. At least a portion of the insertion assembly 5600 is disposed within the housing 5100 (see e.g., FIGS. 16 and 17). Moreover, the transfer mechanism 5500 can engage a portion of the insertion assembly 5600 to move the insertion assembly 5600 and/or portions included therein in the distal direction to deliver the implant 5050 to the target location, as described in further detail herein. The insertion assembly 5600 includes the carrier 5610, the slip member 5630, an engagement member 5640, a push rod 5650, a push rod tube 5660, a distal sheath 5670, and a status member 5690.

As shown in FIGS. 33 and 34, the carrier 5610 has a first side 5611 and a second side 5612, and a proximal end portion 5613 and a distal end portion 5614. The first side 5611 (e.g., a top side) includes a set of guide protrusions 5615, an indicator protrusion 5620, and a coupling portion 5617. The first side 5611 also defines a channel 5616 that within which a tab 5619 is disposed. The second side 5612 (e.g., a bottom side) includes a set of guide protrusions 5621 and the mount protrusion 5623 and defines a recess 5622.

As shown, the carrier 5610 is arranged such that the guide protrusions 5615 of the first side 5611 are aligned with the guide protrusions 5621 of the second side 5612 to define a guide slot 5624 therebetween. In this manner, the carrier 5610 can be disposed within the housing 5100 such that the third guide rail 5133 of the first housing member 5120 and the third guide rail 5153 of the second housing member 5140 are disposed within the guide slots 5624. Similarly stated, the third guide rails 5133 and 5153 can be disposed within the guide slots 5624 such that the guide protrusions 5615 of the first side 5611 are disposed on a first side of the third guide rails 5133 and 5153, and the guide protrusions 5621 of the second side 5612 are disposed on a second side of the third guide rails 5133 and 5153. In this manner, carrier 5610 can move in a proximal and distal direction while being substantially limited in any other direction.

The coupling portion 5617 of the first side 5611 defines an opening 5618 that receives a portion of the push rod 5650 and a portion of the push rod tube 5660. More specifically, a proximal and portion 5661 of the push rod tube 5660 is fixedly disposed within the opening 5618 defined by the coupling portion 5617 and a proximal end portion 5651 of the push rod 5650 can extend through the coupling portion 5617 and the proximal end portion 5661 of the push rod tube 5660 to couple to the engagement member 5640, as described in further detail herein. The channel 5616 is configured to receive the proximal end portion 5651 of the push rod 5650 and the engagement member 5640 such that the push rod 5650 and the engagement member 5640 can be moved between a proximal position and a distal position, as described in further detail herein. The tab 5619 extends from a surface of the channel 5616 to selectively engage the engagement member 5640, as described in further detail herein. The indicator protrusion 5620 is configured to allow a user to visualize the status of the insertion assembly 5600 (e.g., the indicator protrusion 5620 can be an identifiable color such as, for example, green). For example, when the insertion assembly 5600 is moved into a final position to deliver the implant 5050, the indicator protrusion 5620 can be aligned with the status member 5690 to provide a visual indication to the user (e.g., through the status window 5104 described above).

The mount portion 5623 is configured to extend from the second side 5612 of the carrier 5610 and is coupled to a coupling portion 5632 of the slip member 5630. Moreover, when the carrier 5610 is disposed on the transfer member 5510, the mount portion 5623 is configured to extend through the channel 5524 defined by the transfer member 5510. For example, as shown in FIG. 36, the carrier 5610 is disposed on the first side 5511 of the transfer member 5510 and the slip member 5630 is disposed on the second side 5512 of the transfer member 5510. In this manner, the mount protrusion 5623 can extend through the channel 5524 defined by the transfer member 5510 to be coupled to the coupling portion 5632 of the slip member 5630. Furthermore, the mount protrusion 5623 can move within the channel 5624 during a "slip" condition. The slip member 5631 includes a radius portion 5631 that is in contact with the slip surface 5522 of the transfer member 5520. In this manner, when a force is exerted on the transfer mechanism 5500 (i.e., from the drive member 5440) that exceeds a threshold value, the transfer mechanism 5500 can move relative to the carrier 5610 and the slip member 5630 such that only a portion of the force (or no force) is transferred from the drive member 5440 and/or the transfer mechanism 5500 to the insertion assembly 5600. The force threshold value can be controlled and/or adjusted by increasing or decreasing the radius of the detents defined by the slip surface 5522 and/or of the radius portion 5632 of the slip member 5630 (e.g., a larger radius corresponds with a lower force threshold value). In some embodiments, the force threshold value can be controlled by increasing or decreasing the stiffness of the slip member 5530 (e.g., a stiffer slip member 5630 corresponds with a larger force threshold value). Therefore, an undesirable amount of force can be prevented from being applied to the target location.

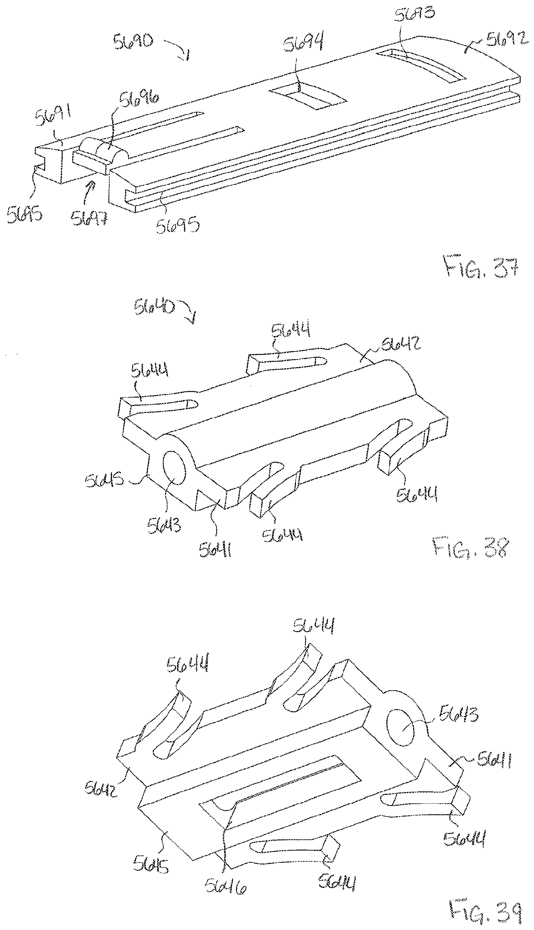

As shown in FIG. 37, the status member 5690 has a proximal end portion 5691 and a distal end portion 5692 and includes a first status portion 5693, a second status portion 5694, and a retention protrusion 5696. The status member 5690 can be movably disposed on the first side 5611 of the carrier 5610. More specifically, the status member 5690 defines a channel 5697 that is configured to be disposed about the indicator protrusion 5620 when the status member 5690 is disposed on the first side 5611 of the carrier 5610. Furthermore, the status member 5690 defines a set of guide slots 5695 that can be disposed about the fourth guide rail 5134 of the first housing member 5120 and the fourth guide rail 5154 of the second housing member 5140. In this manner, the fourth guide rails 5134 and 5154 can allow the status member 5690 to move in the proximal direction and in the distal direction while limiting a movement of the status member 5690 in any other direction. Moreover, the retention tab 5696 can engage a surface of the housing 5100 to resist movement relative to the housing 5100. For example, the retention tab 5696 can exert a force on the surface of the housing 5100 such that a frictional force exists therebetween. Therefore, to move the status member 5690 relative to the housing 5100, a sufficiently large force can be exerted to overcome the friction force between the retention tab 5696 and the surface of the housing 5100.

The first status portion 5693 is configured to allow a user to visualize the status of the insert assembly 5600. More specifically, the first status portion 5693 provides a visual indication that the insertion assembly 5600 is not in a final position to deliver the implant 5050. For example, the first status portion 5693 can be an identifiable color such as black or red. The second status portion 5694 of the status member 5690 can be a substantially clear portion that is configured to be aligned with the indicator protrusion 5620 when the insertion assembly 5600 is moved to the final position. In this manner, the indicator protrusion 5620 can be visualized through the second status portion 5694 and through the status window 5104. Furthermore, when the insertion assembly 5600 is in the final configuration, the first status portion 5693 can be positioned relative to the status window 5104 such that the first status window 5193 is not visible.

As shown in FIGS. 38 and 39, the engagement member 5640 has a proximal end portion 5641 and a distal end portion 5641 and defines and opening 5643 therethrough. The opening 5643 is configured to receive the proximal end portion 5651 of the push rod 5650. For example, in some embodiments, the push rod 5650 and a set of walls defining the opening 5643 can form a friction fit such that the push rod 5650 is fixedly coupled to the engagement member 5640. In this manner, the engagement member 5640 can be operative in moving the push rod 5650 relative to the push rod tube 5660, as described in further detail herein.

The engagement member 5640 further includes a set of retraction protrusions 5644 and a sled portion 5645 that defines a recess 5646. The retraction protrusions 5644 extend from opposite sides of the engagement member 5640 to be in contact with the insertion rack 5136 of the first housing member 5120 and the insertion rack 5156 of the second housing member 5120. As described above, the insertion rack 5136 and the insertion rack 5156 each include a set of asymmetrical teeth that can contact the retraction protrusions 5644 to limit a proximal movement of the engagement member 5640 relative to the housing 5100. For example, the insertion racks 5136 and 5156 can limit the proximal movement of the engagement member 5640 until a force is applied that is sufficiently large to overcome the friction force between the retraction protrusions 5644 and the second surface (described above) of the insertion racks 5136 and 5156. In a similar manner, a force can be applied that is sufficiently large to deform (e.g., elastically or plastically) the retraction protrusions 5644 such that the retraction protrusions 5644 are disengaged from the insertion racks 5136 and 5156. Thus, the force required to deform the retraction protrusions 5644 can be controlled by increasing or decreasing the flexibility of the retraction protrusions 5644 (e.g., increasing the cross-sectional area, adding a discontinuity, or forming the retractions protrusions 5644 from a material that is more or less stiff).

The sled portion 5645 is disposed within the channel 5616 defined by the carrier 5610 such that the tab 5619 is at least temporarily disposed within the recess 5646 of the sled portion 5645. Moreover, the engagement member 5640 can move relative to the carrier 5610 such that the sled portion 5645 moves within the channel 5616. For example and as described in further detail herein, the drive member 5410 can be configured to move the transfer mechanism 5510 and the carrier 5610 in a proximal direction. With the retraction protrusions 5644 in contact with the insertion racks 5136 and 5156, the engagement member 5640 can be retained a fixed position relative to the housing 5100 while the transfer mechanism 5500 and the carrier 5610 are moved in a proximal direction relative to the housing 5100. In such instances, the sled portion 5645 of the engagement member 5640 is moved to a distal position relative to the tab 5619 such that when the carrier 5610 is again moved in the distal direction, the tab 5619 can engage a surface of the sled portion 5645 to move the engagement member 5640 in the distal direction with the carrier 5610. Thus, the engagement member 5640 can be moved to a second position relative to the carrier 5610, as described in further detail herein.

The push rod 5650 includes the proximal end portion 5651 and a distal end portion 5652 (see e.g., FIG. 32). The push rod 5650 can be any suitable shape, size, or configuration. For example, in some embodiments, the push rod 5650 can be a substantially solid rod. In other embodiments, the push rod 5650 can be hollow. Furthermore, the push rod 5650 can be formed from any suitable material such as, for example, a biocompatible metal or polymer. In this manner, the stiffness of the push rod 5650 can be controlled by increasing or decreasing the cross-sectional area of the push rod 5650 and/or by forming the push rod 5650 from a material with a greater or lesser stiffness. In some embodiments, for example, the push rod 5650 and/or the push rod tube 5660 are formulated and/or constructed to bend and/or follow a curved path within the housing 5100 and/or the vacuum tip 5250 during the insertion process. The proximal end portion 5651 of the push rod 5650 is fixedly coupled to the engagement member 5640, as described above. The distal end portion 5652 includes a notch 5653 and can be placed in contact with an implant 5050 to deliver the implant 5050 to the target location (e.g., the fundus and/or within the uterus). The notch 5653 can be configured to provide clearance for an access opening 5663 within the push rod tube 5660, as described in further detail herein.

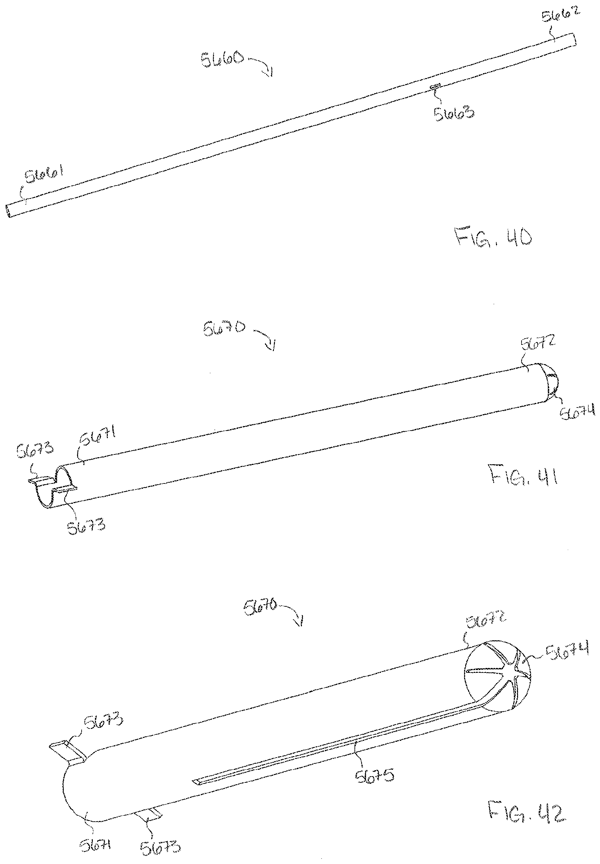

As shown in FIG. 40, the push rod tube 5660 has the proximal end portion 5661 and a distal end portion 5662, and defines a lumen therethrough and the access opening 5663. The push rod tube 5660 is configured to circumscribe at least a portion of the push rod 5650 such that the push rod 5650 can move within the push rod tube 5660. Furthermore, the push rod tube 5660 is configured to house, at least temporarily, the implant 5050, as described in further detail herein. The proximal end portion 5661 of the push rod tube 5660 is disposed within the opening 5618 of the coupling portion 5618 of the carrier 5610. More specifically, the push rod tube 5660 can form a friction fit with the coupling portion 5618 such that the push rod tube 5660 is fixedly coupled thereto. The distal end portion 5662 can be movably disposed within the distal sheath 5670, as described in further detail herein. The access opening 5663 can receive the distal end portion 5352 of the filament 5350 such that at least a portion of the filament 5350 of the guide assembly 5300 is disposed within the push rod tube 5660 while another portion of the filament 5350 is disposed outside of the push rod tube 5660. In this manner, the filament 5350 can be coupled to a portion of the implant 5050, as described above.

As shown in FIGS. 41 and 42, the distal sheath 5670 has a proximal end portion 5671 and a distal end portion 5672. A portion of the distal sheath 5670 is movably disposed within the distal end portion 5102 of the housing 5100. Furthermore, the distal end portion 5662 of the push rod tube 5660, a distal end portion 5651 of the push rod 5650, and the implant 5050 can be movably disposed within the distal sheath 5670. The proximal end portion 5671 includes a set of tabs 5673 that can be placed in contact with a surface of the housing 5100 to limit the distal movement of the distal sheath 5670 relative to the housing 5100. The distal end portion 5672 includes a movable cover 5674. The movable cover 5674 can be moved between an closed configuration in which a surface of the distal cover 5674 forms a rounded tip and an open configuration through which the push rod tube 5660 and/or the push rod 5650 can move, as described in further detail herein. The distal sheath 5670 also defines a slot 5675 that can receive a portion of the filament 5350 and/or a portion of the implant 5050. For example, the filament 5350 can be inserted through a portion of the slot 5675 and into the access opening 5663 to be disposed within the push rod tube 5660. Moreover, a user can manipulate the guide mechanism 5300 to pull the distal end portion 5352 of the filament 5350 and a portion of the implant 5050 (e.g., the retraction filament of an IUD) through the slot 5675 such that the portion of the implant 5050 can be inserted into the cutter assembly 5700, as described in further detail herein.

FIG. 43 shows the cutter assembly 5700. The cutter assembly 5700 is movably disposed within the distal end portion 5102 of the housing 5100 (see e.g., FIGS. 16 and 17), and is configured to engage a portion of the implant 5050 (e.g., a retraction filament of an IUD). The cutter assembly 5700 includes a cutter housing 5710, a cutter 5720, and an anvil 5730. The cutter housing 5710 includes a set of guide protrusions 5712 that can be movably disposed within the cutter channel 5138 of the first housing member 5120 and the cutter channel 5158 of the second housing member 5150. In this manner, the cutter channels 5138 and 5158 can allow the cutter housing 5710 to move in a proximal direction and a distal direction while limiting a movement in any other direction, as described above. The cutter housing 5710 includes a slot 5711 that receives the cutter 5720 such that the cutter 5720 is fixedly coupled thereto. The cutter 5720 can be any suitable member configured to include a sharp edge suitable for cutting a material.

The anvil 5730 is fixedly coupled to the coupled to the housing 5100 and can be movably disposed within the slot 5711. The anvil 5730 includes an aperture 5731 that can receive at a portion of the filament 5350 of the guide mechanism 5300 and/or a portion of the implant 5050. In this manner, the housing 5710 and the cutter 5720 can be moved relative to the anvil 5730 to cut the portion of the implant 5050. More specifically, when the drive member 5420 moves the transfer mechanism 5500 in the distal direction, a distal surface of the transfer member 5510 can be placed in contact with the housing 5710 of the cutter assembly 5700 to move the cutter housing 5710 and the cutter 5720 relative to the housing 5100 (and, therefore, the anvil 5730). Thus, the cutter 5730 can be used to cut a portion of the implant 5050 that is disposed within the aperture 5731.

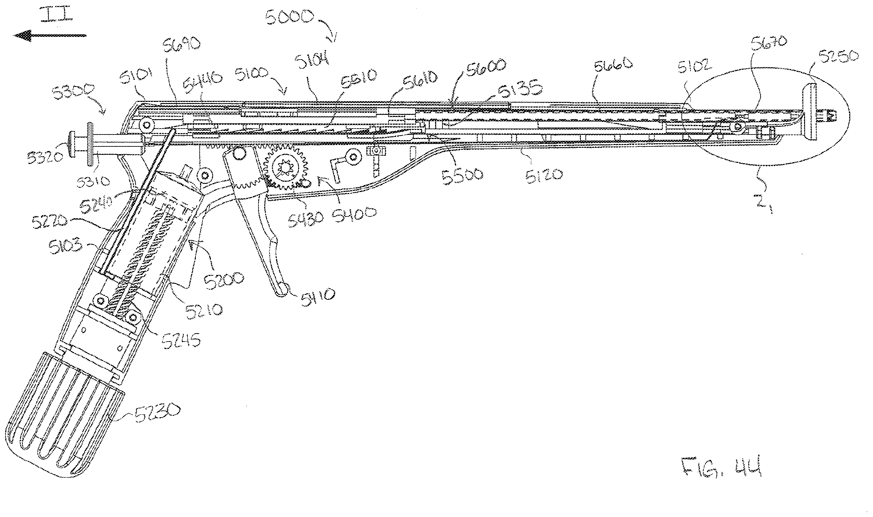

The delivery device 5000 is first enabled by moving the delivery device 5000 from a first configuration to a second configuration by releasably coupling a first portion 5051 of an implant 5050 (e.g., a retraction filament of an IUD) to the filament 5350 of the of the guide mechanism 5300. For example, a user can engage distal sheath 5670 to move the distal sheath 5670 in a proximal direction relative to the push rod tube 5660, as shown in FIG. 44. In this manner, the movable cover 5674 can move, at least partially, to its open configuration such that the distal end portion 5662 of the push rod tube 5660 is exposed. After the distal sheath 5670 is moved in the proximal direction, the user can engage the activator 5320 of the guide mechanism 5300 to move the activator 5320 in a distal direction relative to the base 5310 (e.g., the user can apply a force on the engagement flange 5324 of the activator 5320 in the distal direction). The distal movement of the activator 5320 moves the bias member 5330 (FIG. 34) to its compressed configuration and urges the snare 5353 disposed at the distal end portion 5352 of the filament 5350 to advance in the distal direction relative to the distal end portion 5342 of the sheath 5340. In this manner, the snare 5353 can move to an open (or expanded) configuration and can extend beyond the distal end portion 5662 of the push rod tube 5660.

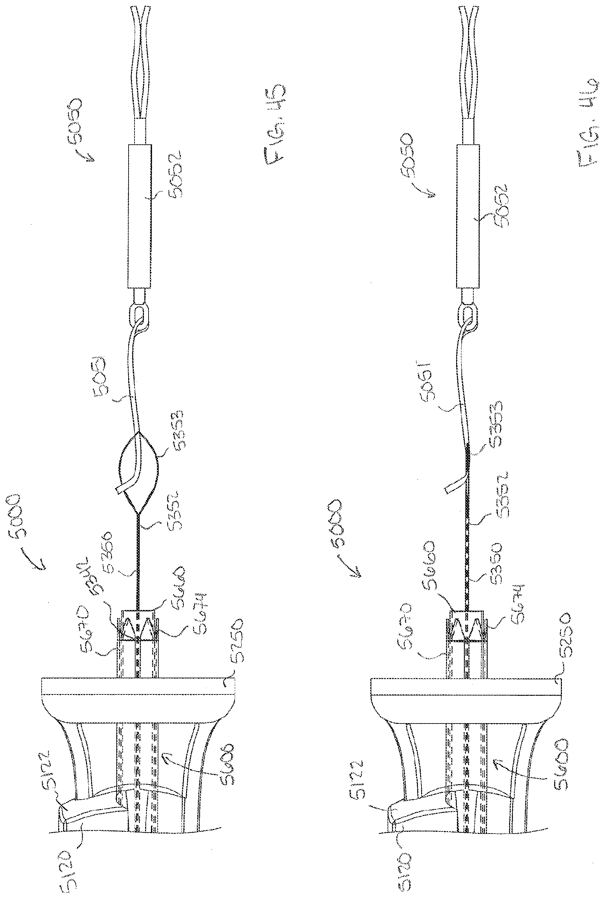

As shown in FIG. 45, the first portion 5051 of the implant 5051 can be inserted into the snare 5353. After the first portion 5051 of the implant 5050 is disposed within the snare 5353, the user can disengage the activator 5320 by removing the force applied on the engagement flange 5324. In this manner, the bias member 5330 can expand from its compressed configuration to exert a force on the activator 5320, thereby moving the activator 5320 in the proximal direction. The proximal movement of the activator 5320 moves the filament 5350 in the proximal direction relative to the sheath 5340 such that the distal end portion 5342 of the sheath 5340 engages a portion of the snare 5353 to move the snare 5353 from its open configuration to its closed (or collapsed) configuration, as shown in FIG. 46. Thus, the snare 5353 can collapse around the first portion 5051 of the implant 5050 to releasably couple the implant 5050 to the guide mechanism 5300 and/or the delivery device 5000.

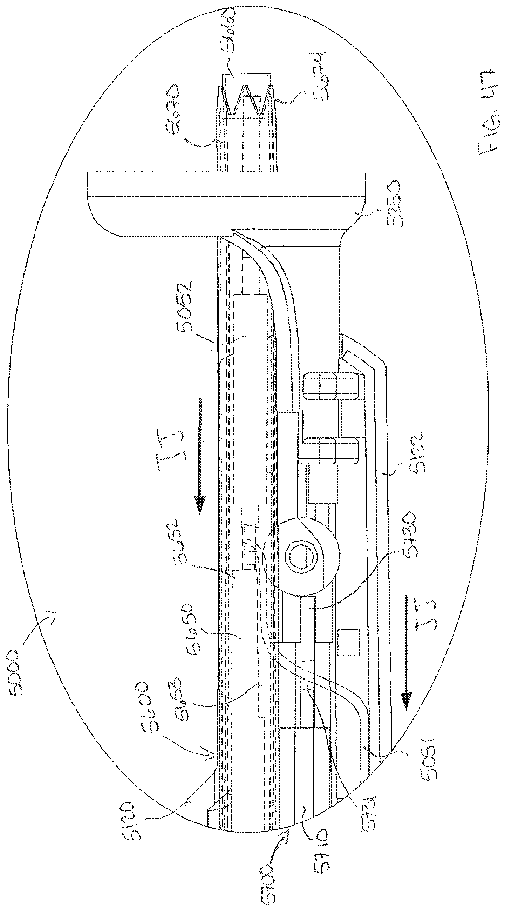

After the snare 5353 is moved to the closed configuration, the user can engage the base 5310 of the guide mechanism 5300 to collectively move the base 5310 and the activator 5320 in the proximal direction indicated by the arrow II in FIG. 44. The proximal movement of the base 5310 and the activator 5320 can urge the filament 5350 to move in the proximal direction. More specifically, when the guide mechanism 5300 is removed from the housing 5100, the filament 5350 moves in the proximal direction such that the distal end portion 5352 and the snare 5353 pass through the access opening 5663 defined in the push rod tube 5660, through the slot 5675 defined by the distal sheath 5670, and through the aperture 5731 defined by the anvil 5730 of the cutter assembly 5700, as indicated by the arrow JJ in FIG. 47. Thus, a distal end of the first portion 50 1 of the implant 5050 can be disposed within the aperture 5731 of the anvil 5730, as described in further detail herein.

After the first portion 5051 of the implant 5050 is moved through the aperture 5731, the user can insert the delivery device into a vagina of a patient such that the vacuum tip 5250 is disposed adjacent to the cervix (not shown). More specifically, the distal surface 5256 of the vacuum tip 5250 can be brought into contact with the cervix. In some embodiments, the vacuum tip can articulate (i.e., rotate) relative to the housing 5100 to enhance the user's ability to bring the vacuum tip 5250 into contact with the cervix. In such embodiments, the housing 5100 and the vacuum tip 5250 can collectively define a curved and/or non- linear passageway through which at least of the insertion assembly 5600 can be conveyed. In some embodiments, the vacuum tip 5250 can be an articulating head of the types shown and described in International Patent Application Publication No. WO 2012/054466.

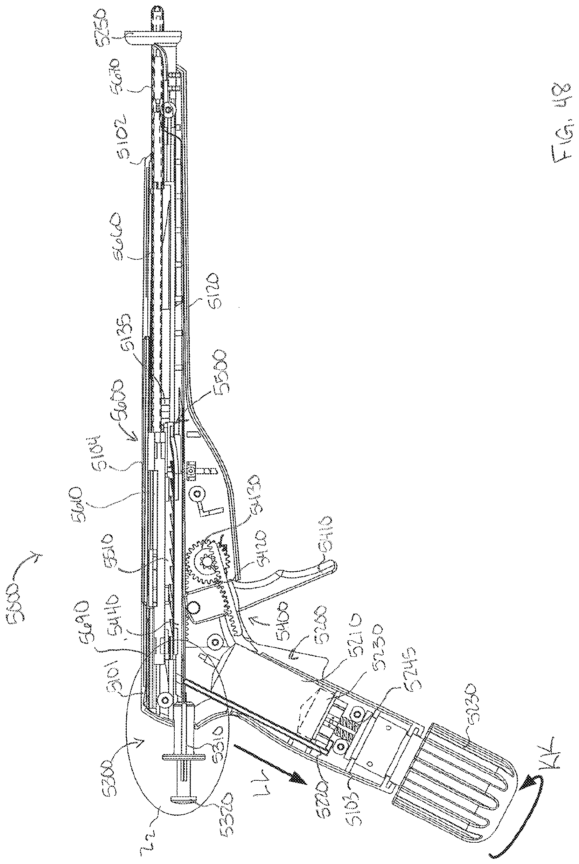

After the distal surface 5256 is placed in contact with the cervix, the user can manipulate the engagement portion 5230 of the vacuum assembly 5200 by rotating the engagement portion 5230 relative to the handle portion 5103 of the housing 5100, as indicated by the arrow **KK** in FIG. 48. In this manner, the threaded rod 5245 can be advanced within the threaded insert 5235 of the engagement portion 5230. The movement of the threaded rod 5245 can urge the plunger 5240 to move within the vacuum cylinder 5210, as indicated by the arrow **LL** in FIG. 48. The movement of the plunger 5240 within the vacuum cylinder 5210 can be such that a negative pressure is produced within the vacuum cylinder and transferred (via any suitable tubing, not shown) to the vacuum tip 5250. Thus, with the distal surface 5256 in contact with the cervix, a negative pressure can build within the vacuum channel 5257 of the vacuum tip 5250 and can exert a suction force on the cervix to couple, at least temporarily, the vacuum tip 5250 thereto.

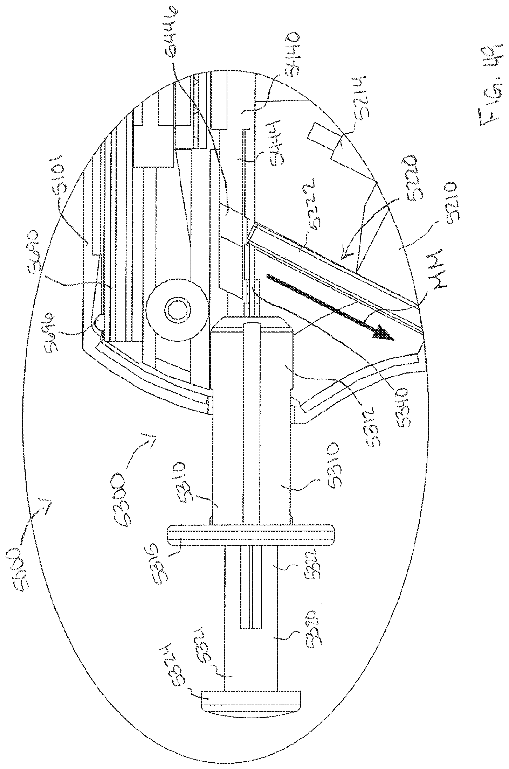

The movement of the plunger 5240 within the vacuum cylinder 5210 can be such that a portion of the plunger 5240 is placed in contact with the tab 5225 of the lock rod 5220. Thus, the lock rod 5220 can moved relative to the vacuum cylinder 5210. More specifically, the lock rod 5220 is moved such that the distal end portion 5222 is moved to a position that is substantially outside of the slot 5446 defined by the proximal end portion 5444 of the drive member 5440, as indicated by the arrow **MM** in FIG. 49. Therefore, the actuator assembly 5400 is moved from a locked configuration to an unlocked configuration. Furthermore, the movement of the lock rod 5220 can be such that the status indicator 5223 is brought into alignment with the lock status window 5107. In this manner, the status indicator 5223 can be seen through the lock status window 5107 to indicate to the user that the lock rod 5220 has been moved relative to the vacuum cylinder 5210.

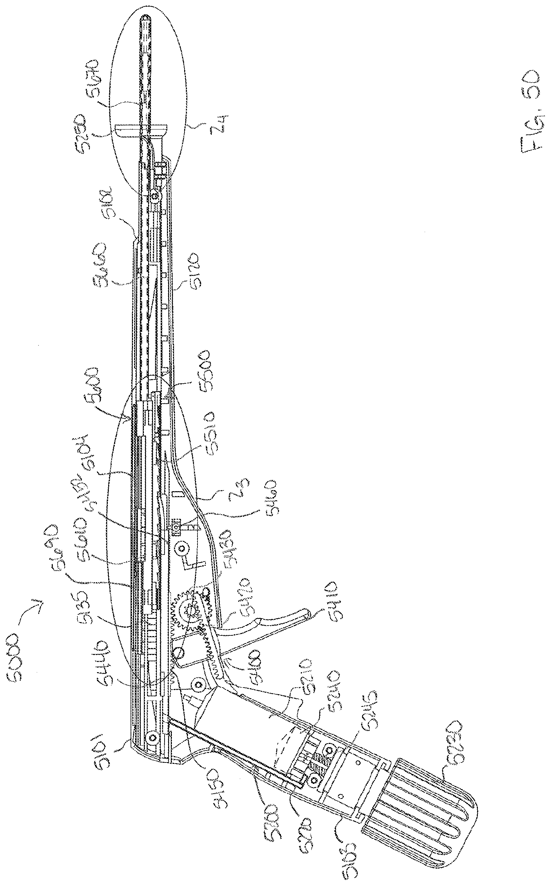

After the actuator assembly 5400 is moved to the unlocked configuration, the user can manipulate the engagement portion 5411 of the trigger 5410 to move the delivery device 5000 from a second configuration to a third configuration (FIG. 50). For example, the user can move the delivery device 5000 to the third configuration by squeezing the trigger 5410 and the handle portion 5103 of the housing 5100, thereby moving the trigger 5410 toward the trigger stop 5109. In this manner, the trigger 5410 can pivot within and/or about the apertures defined by the trigger protrusion 5128 of the first housing member 5120 and the trigger protrusion 5148 of the second housing member 5140. As described in detail above, the pivoting of the trigger 5410 can be such that the drive rack 5420 is advanced along the gear segment 5415. Thus, the drive rack 5420 is moved along the linear path defined by the rack guide 5147.

The movement of the drive rack 5420 urges the rack pinion 5431 of the gear member 5430 to advance along the teeth of the drive rack 5420 (described in detail above). With the gear member 5430 partially disposed in the apertures defined by the gear protrusion 5129 of the first housing member 5120 and the gear protrusion 5149 of the second housing member 5140, the meshing of the rack pinion 5431 with the drive rack 5420 rotates the gear member 5430 within the apertures. The drive pinion 5432 of the gear member 5430 is rotated concurrently with the rack pinion 5431 (e.g., at the same time but with a different circumferential displacement due to the larger diameter of the drive pinion 5432 relative to the rack pinion 5431). Moreover, with the drive pinion 5432 in contact with the actuator rack 5450, the rotation of the drive pinion 5432 advances the drive member 5440 in the distal direction. In this manner, the drive member 5440 can engage the transfer member 5510 to move the transfer mechanism 5500 in the distal direction and place the delivery device 5000 in the third configuration (FIG. 50).

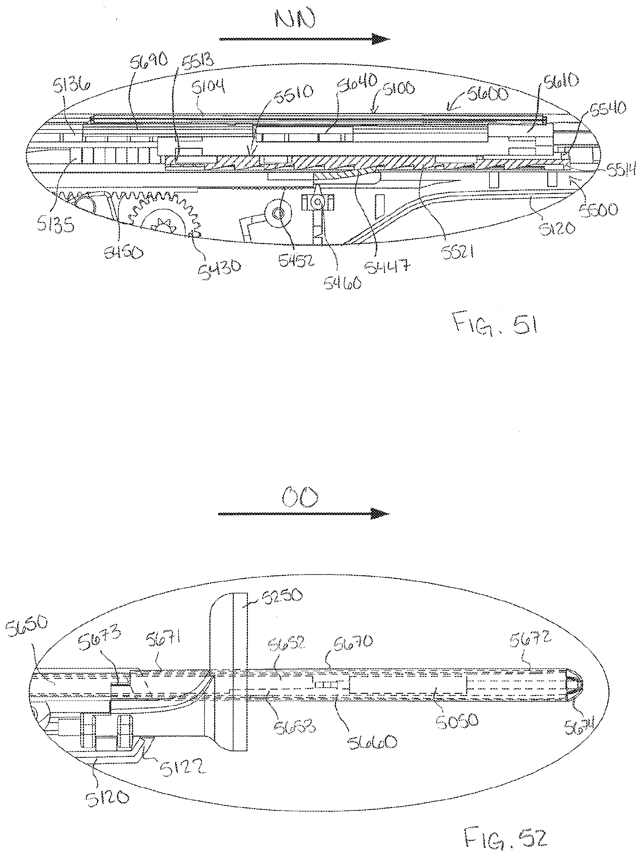

For example, as shown in FIG. 51, the push arm 5447 of the drive member 5440 is placed in contact with the drive portion 5521 of the transfer member 5500. In this manner, the push arm 5447 exerts a force on the first surface of a tooth (described above) included in the drive portion 5521. Furthermore, the teeth of the drive portion 5521 can be arranged such that the slope angle of the first surface is sufficiently large to substantially prevent the push arm 5447 from slipping relative to the first surface. Thus, the push arm 5447 exert the force on the first surface of a tooth of the drive portion 5521 to move the transfer mechanism 5500 in the distal direction, as indicated by the arrow **NN** in FIG. 51.

The distal movement of the transfer mechanism 5500 urges the insertion assembly 5600 to move in the distal direction. For example, with the carrier 5610 coupled to the transfer member 5510, the distal movement of the transfer member 5510 moves the carrier 5610 in the distal direction. The arrangement of the insertion assembly 5600 is such that as the carrier 5610 is moved in the distal direction, the slip member 5630, the engagement member 5640, the push rod 5650, the push rod tube 5660, the distal sheath 5670, and the status member 5690 are moved in the distal direction relative to the housing 5100, as shown in FIG. 51.

The distal movement of the distal sheath 5670 places the tabs 5673 of the distal sheath 5670 in contact with a surface of the distal end portion 5102 of the housing 5100, thereby limiting further distal movement of the distal sheath 5670, as described in further detail herein. Furthermore, a portion of the distal sheath 5670, the push rod 5650, the push rod tube 5660, and the implant 5050 are moved in the distal direction relative to the distal surface 5256 of the vacuum tip 5250 to pass through, for example, the cervical os (not shown), as indicated by the arrow **OO** in FIG. 52.

With the distal sheath 5670, the push rod 5650, the push rod tube 5660, and the implant 5050 in a desired position, the user can release the trigger 5410 to allow the trigger spring (not shown) to move the trigger 5410 towards its first position. In this manner, the trigger 5410 moves the drive rack 5420 in a proximal direction, which, in turn, rotates the rack pinion 5431 in a counterclockwise direction. Thus, the drive pinion 5432 moves the drive member 5440 in the proximal direction. Moreover, with the retraction protrusions 5516 of the transfer member 5510 in contact with the transfer rack 5135 and with the retraction protrusions 5644 of the engagement member 5640 in contact with the insertion rack 5136, the transfer mechanism 5500 and the insertion assembly 5600 can be at least temporarily retained in a fixed position relative to the housing 5100. Similarly stated, the retraction protrusions 5516 and 5644 and the transfer rack 5135 and the insertion rack 5136, respectively, limit the proximal movement of the transfer mechanism 5500 and the insertion assembly 5600 when the drive member 5440 is moved in the proximal direction. Thus, the drive member 5440 moves in the proximal direction relative to the transfer mechanism 5500 and the insertion assembly 5600.

The proximal movement of the drive member 5440 relative to the transfer member 5510 is such that the push arm 5447 moves along the second surface of the teeth included in the drive portion 5521 of the transfer member 5510. Expanding further, the slope angle of the second surface can be sufficiently small to allow the push arm 5447 to move in a proximal direction along the second surface of the teeth. Therefore, the actuator assembly 5400 can be returned to a non-actuated state.

Although not shown, the distal movement of the drive member 5440 places the engagement rack 5452 in contact with the pawl 5460. The pawl 5460 is then pivoted from its first configuration to its second configuration relative to the pawl mount 5460 and is sequentially advanced along a first surface of the teeth of the engagement rack 5452. Furthermore, with the pawl 5460 coupled to the spring (as described above), the spring exerts a force to retain the pawl 5460 in contact with the first surface of the teeth. Similarly stated, the pivoting of the pawl 5460 relative to the pawl mount 5130 moves the spring from an undeformed configuration having lower potential energy to a deformed configuration having a higher potential energy.

The arrangement of the pawl 5460 and the engagement rack 5452 is such that the pawl 5460 and the engagement rack 5453 prevent the trigger 5410 from being partly actuated. For example, if the trigger 5410 is partially moved toward its second position and a user removes the force (e.g., stops squeezing the trigger 5410), the pawl 5460 can move in a distal direction along a first surface of a tooth in the engagement rack 5452 until the pawl 5460 is placed in contact with a second surface of an adjacent tooth. In this manner, the second surface prevents a further distal movement of the pawl 5460 relative to the engagement rack 5452. Thus, the trigger 5410 is prevented from substantially moving to its first position, thereby alerting the user that the initial actuation was incomplete.

When the actuator assembly 5400 has been fully actuated (e.g., a user has moved the trigger 5410 to its second position) the pawl 5460 can be placed in a proximal position relative to the engagement rack 5452 and can be disposed within the recess 5451 defined by the drive member 5440. Similarly stated, the engagement rack 5452 is moved to a distal position relative to the pawl 5460 such that the engagement rack 5452 and the pawl 5460 are no longer in contact. In this manner, the spring can exert a force (e.g., by returning to its undeformed configuration) on the pawl 5460 to return the pawl 5460 to its first position relative to the pawl mount 5130 (e.g., the pawl 5460 is pivoted relative to the pawl mount 5460). When the drive member 5440 is moved in the proximal direction (e.g., when the trigger 5410 is moved towards its first position), the engagement rack 5452 is again placed in contact with the pawl 5460 and pivots the pawl 5460 in an opposite direction. In this manner, the pawl 5460 is sequentially advanced along the second surface of the teeth of the engagement rack 5452 until the engagement rack 5452 is moved to a distal position relative to the pawl 5460. Thus, a partial actuation step of the delivery device 5000 is prevented.

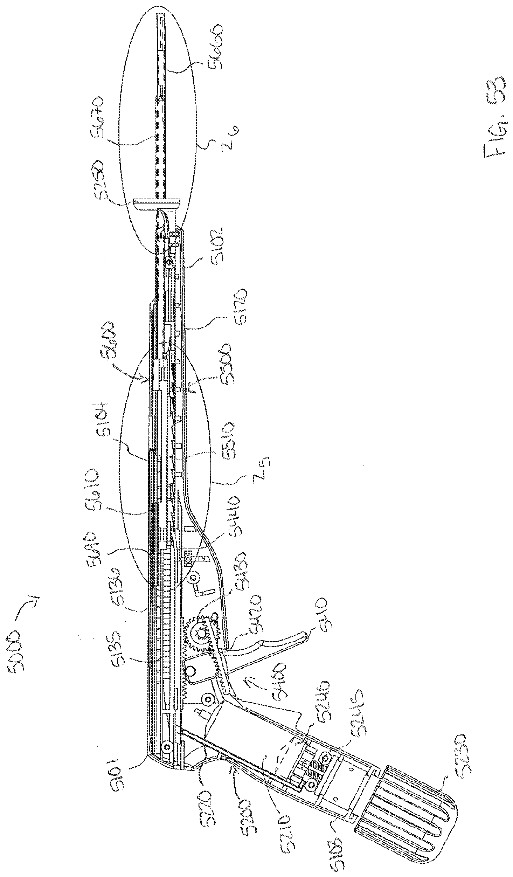

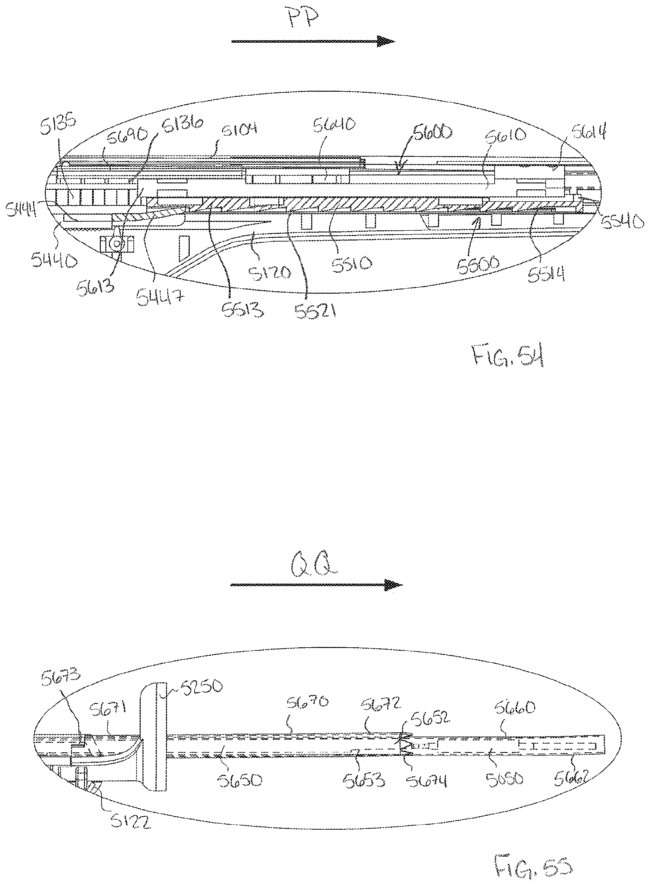

After the distal sheath 5670, the push rod 5650, the push rod tube 5660, and the implant 5050 have been advanced relative to the vacuum tip 5250 and after the trigger 5410 has been returned to its first position, the user can again squeeze the trigger 5410 to move the delivery device 5000 from the third configuration to a fourth configuration (FIG. 53). In this manner, the trigger 5410 moves the drive rack 5420 in a distal direction, which, in turn, rotates the rack pinion 5431 in a clockwise direction. Thus, the drive pinion 5432 moves the drive member 5440 in the distal direction (as described in detail above). The distal movement of the drive member 5440 is such that the push arm 5447 is again placed in contact with the drive portion 5521 of the transfer member 5500. Expanding further, with the position of the transfer mechanism 5500 at least temporarily retained, the push arm 5447 is placed in contact with the first surface of a tooth that is in a proximal position relative to the tooth previously engaged by the push arm 5447. In this manner, the push arm 5447 exerts a force on the first surface of the tooth to move the transfer mechanism 5500 in the distal direction, as indicated by the arrow **PP** in FIG. 54.

The distal movement of the transfer mechanism 5500 urges the insertion assembly 5600 to move in the distal direction, as described in detail above. For example, as the carrier 5610 is moved in the distal direction, the slip member 5630, the engagement member 5640, the push rod 5650, the push rod tube 5660, and the status member 5690 are moved in the distal direction relative to the housing 5100, as shown in FIG. 53. Furthermore, with the tabs 5673 of the distal sheath 5670 in contact with the surface of the distal end portion 5102 of the housing 5100, the push rod 5650, the push rod tube 5660, and the implant 5050 are advanced in the distal direction relative to the distal sheath 5670. In this manner, the push rod tube 5660 contacts the movable cover 5674 of the distal sheath 5670 and moves the movable cover 5674 to its open configuration. Thus, the distal end portion 5662 of the push rod tube 5660 and the implant 5050 are moved to a distal position relative to the movable cover 5674 of the distal sheath 5670 (i.e., the distal end portion 5662 of the push rod tube 5660 and the implant 5050 extend beyond the distal sheath 5670), as indicated by the arrow **QQ** in FIG. 55.

With the push rod 5650, the push rod tube 5660, and the implant 5050 in a desired position, the user can again release the trigger 5410 to allow the trigger spring (not shown) to move the trigger 5410 towards its first position. With the retraction protrusions 5516 of the transfer member 5510 the retraction protrusions 5644 of the engagement member 5640 in contact with the transfer rack 5135 and the insertion rack 5136, respectively, the transfer mechanism 5500 and the insertion assembly 5600 can be at least temporarily retained in a fixed position relative to the housing 5100. Thus, the drive member 5440 moves in the proximal direction relative to the transfer mechanism 5500 and the insertion assembly 5600 and the actuator assembly 5400 returns to the non-actuated state (as described in detail above).

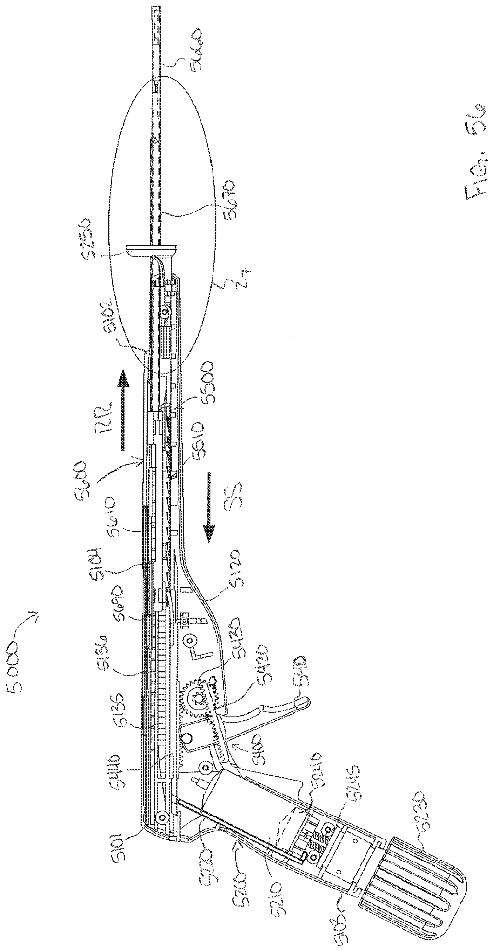

After the push rod 5650, the push rod tube 5660, and the implant 5050 have been advanced relative to the distal sheath 5670, and after the trigger 5410 has been returned to its first position, the user can again squeeze the trigger 5410 to move the delivery device 5000 from the fourth configuration to a fifth configuration (FIG. 56). In this manner, the trigger 5410 moves the drive rack 5420 in a distal direction, which, in turn, rotates the rack pinion 5431 in a clockwise direction. Thus, the drive pinion 5432 moves the drive member 5440 in the distal direction (as described in detail above). The distal movement of the drive member 5440 is such that the push arm 5447 is again placed in contact with the drive portion 5521 of the transfer member 5500. Expanding further, with the position of the transfer mechanism 5500 at least temporarily retained, the push arm 5447 is placed in contact with the first surface of a tooth that is in a proximal position relative to the tooth previously engaged by the push arm 5447. In this manner, the push arm 5447 exerts a force on the first surface of the tooth to move the transfer mechanism 5500 in the distal direction, as indicated by the arrow **RR** in FIG. 56.

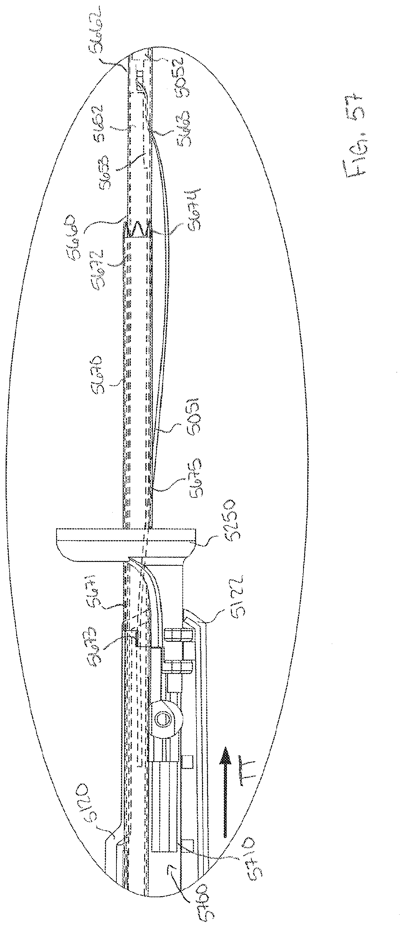

The distal movement of the transfer mechanism 5500 is such that a distal surface of the transfer member 5510 is placed in contact with a distal surface of the cutter housing 5700. In this manner, the transfer mechanism 5500 can move the cutter housing 5700, and the cutter 5720 disposed therein, in the distal direction relative to the anvil 5730, as indicated by the arrow **TT** in FIG. 57. Therefore, with the first portion 5051 of the implant 5050 disposed within the aperture 5731 defined by the anvil 5730, the cutter housing 5710 and the cutter 5720 are moved to a distal position relative to the aperture 5731 and the cutter 5720 cuts the first portion 5051 of the implant 5051 to a desired length, thereby moving the delivery device 5000 from the fourth configuration to a fifth configuration.

The distal movement of the transfer mechanism 5500 again urges the insertion assembly 5600 to move in the distal direction. For example, as the carrier 5610 is moved in the distal direction, the slip member 5630, the engagement member 5640, the push rod 5650, the push rod tube 5660, and the status member 5690 are again moved in the distal direction relative to the housing 5100, as shown in FIG. 56. Furthermore, with the tabs 5673 of the distal sheath 5670 in contact with the surface of the distal end portion 5102 of the housing 5100, the push rod 5650, the push rod tube 5660, and the implant 5050 are advanced in the distal direction relative to the distal sheath 5670. In this manner, the distal end portion 5662 of the push rod tube 5660, the distal end portion 5652 of the push rod 5650, and the implant 5050 are moved to a distal position relative to the movable cover 5674 of the distal sheath 5670.

In some instances, the distal end portion 5662 of the push rod tube 5660 can be placed in contact with a surface of the anatomy of a patient. For example, in some instances, the distal end portion 5662 can be placed in contact with a wall of the uterus (e.g., a target location). In such instances, the contact between the distal end portion 5662 of the push rod tube 5660 and the wall (e.g., the fundus of the uterus) can be such that the wall exerts a reaction force on the distal end portion 5662 of the push rod tube 5660. In this manner, the push rod tube 5660 resists further distal movement. Furthermore, with the user squeezing the trigger 5410 the force exerted on the transfer member 5510 increases due to the increasing reaction force exerted on the distal end portion 5662 of the push rod tube 5660 by the wall of the uterus.

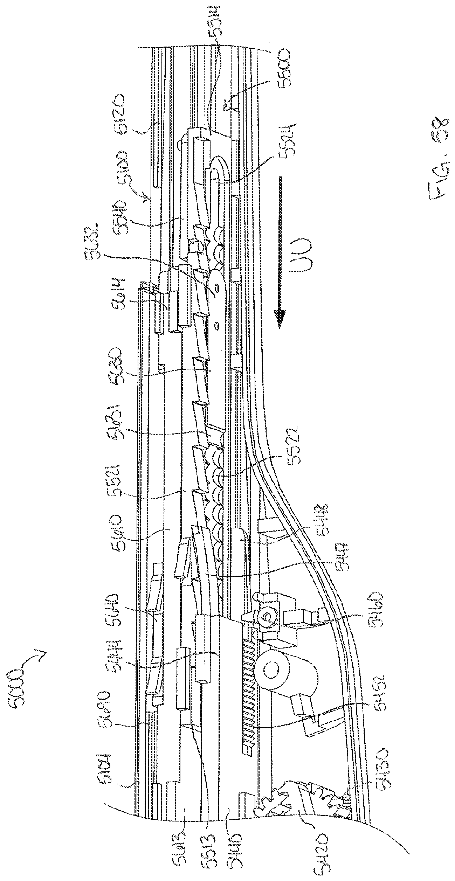

In such instances, the arrangement of the transfer member 5510, the carrier 5610 the slip member 5630 can be such that the transfer member 5510 can move relative to the carrier 5610 and the slip member 5630. Similarly stated, the carrier 5610 and the slip member 5630 can "slip" along the transfer member 5510. Expanding further, as described above with reference to FIG. 36, the mount portion 5623 of the carrier 5610 is disposed within the channel 5524 of the transfer member 5510 and coupled to the coupling portion 5631 of the slip member 5630 such that the radius portion 5631 of the slip member 5630 is in contact with the slip surface 5522. In this manner, the force exerted on the transfer member 5510 can be sufficiently large to cause the slip member 5630 to "slip" along the surface of the slip surface 5522, as indicated by the arrow **UU** in FIG. 58. Thus, the transfer mechanism 5500 transfers a portion of the force that would otherwise be transferred to the insertion assembly 5600 and an undesired amount of force exerted on the wall of the uterus can be prevented.

The "slipping" (e.g., force limiting) can be such that the delivery device 5000 can be used on patients with varying anatomical dimensions. For example, in some instances, a uterus of a first patient may be five centimeters deep while the uterus of a second patient may be up to 13 centimeters. In this manner, similar delivery devices 5000 can be used on each patient to deliver the implant 5050 (e.g., an IUD). Expanding further, the "slipping" of the transfer mechanism 5500 relative to the carrier 5610 and the slip member 5630 is such that a substantially equal amount of force can be applied to a wall of the uterus (e.g., the fundus) of both patients. Thus, the delivery device 5000 can be used to deliver an implant to patients with varying anatomical dimensions. Moreover, the "slipping" of the transfer mechanism 5500 can be such that the cutter assembly 5700 cuts the first portion 5051 of the implant 5050 to a length associated with the anatomical dimensions of the patient. For example, the transfer member 5510 can slip relative to the carrier 5610 and the slip member 5630 and can engage the cutter housing 5700 to move the cutter housing 5700 in the distal direction (as described in detail above).

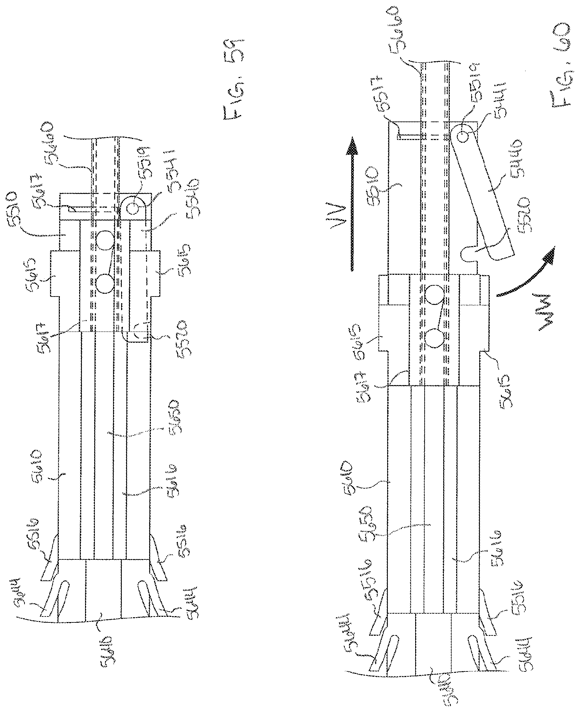

In some instances, the slip member 5630 can "slip" a maximum distance along the slip surface 5522 of the transfer member 5510. Similarly stated, the transfer member 5510 can move a maximum distance in the distal direction relative to the carrier 5610 and the slip member 5630. In such instances, prior to "slipping," the carrier 5610 is disposed relative to the transfer member 5510 such that the lock out member 5540 is in a restrained configuration, as shown in FIG. 59. More specifically, the lock out member 5540 is disposed within the recess 5622 (not shown in FIG. 59) defined by the second side 5612 of the carrier 5610. In this manner, the lock out member 5540 is retained relative to the transfer member 5510. Similarly stated, a set of walls defining the recess 5612 prevent the lock out spring (not shown) from expanding to its unrestrained configuration. Thus, when the lock out member 5540 is disposed within the recess 5612, the lock out spring in restrained configuration an includes a higher potential energy than when in its unrestrained configuration.

As shown in FIG. 60, when the transfer mechanism 5510 is moved in the distal direction relative to the carrier 5610 (e.g., into a maximum slip position), the lock out member 5540 can be moved to a distal position relative to the recess 5612, as indicated by the arrow VV in FIG. 60. In this manner, the lock out spring (not shown) can expand towards it unrestrained configuration to exert a force on the lock out member 5540. Thus, the lock out member 5540 pivots about the mounting protrusion 5519 and the lock protrusion 5542 (not shown in FIG. 60) moves from the notch 5520, as indicated by the arrow WW. With the lock out member 5540 moved to the unrestrained configuration, the lock protrusion 5542 is moved into a detent included in the set of lock out detents 5157 defined by the first guide rail 5151. Therefore, with the lock protrusion 5542 disposed within the lock out detent 5157, the transfer mechanism 5500 is prevented from moving. Furthermore, with the transfer mechanism 5500 constrained, the actuator 5400 is prevented from moving, thereby preventing injury of the patient and/or damage to the delivery device 500.

Referring back to FIG. 56, with the push rod 5650, the push rod tube 5660, and the implant 5050 in a desired position and with the transfer mechanism 5500 not is a maximum slip position, the user can again release the trigger 5410 to allow the trigger spring (not shown) to move the trigger 5410 towards its first position. In this manner, the drive member 5440 is moved in the proximal direction. Moreover, the distal movement of the transfer member 5510 moves the engagement protrusion 5449 of the engagement arm 5448 within the engagement slot 5523 defined by the transfer member 5510 to place the engagement protrusion 5449 with a proximal wall defining the engagement slot 5523. Thus, when the drive member 5440 moves in the proximal direction, the engagement protrusion 5449 exerts a force on the proximal wall defining a portion of the engagement slot 5523 to urge the transfer member 5500 to move in the proximal direction, as indicated by the arrow SS in FIG. 56. Expanding further, the engagement protrusion 5449 can exert a force on the proximal wall defining the portion of the engagement slot 5523 that is sufficiently large to move the retraction protrusions 5516 of the transfer member 5510 relative to the transfer racks 5135 and 5155. For example, in some instances the force is sufficiently large to overcome the friction force between the transfer racks 5135 and 5155 and the retraction protrusions 5516. In other instances, the force is sufficiently large to deform the retraction protrusions 5516 such that the retraction protrusions 5516 are removed from contact with the transfer racks 5135 and 5155. Thus, the transfer mechanism 5500 can be moved in the proximal direction (and can, therefore, reciprocate within the housing).

The proximal movement of the transfer mechanism 5500 moves the delivery device from the fifth configuration to a sixth configuration (FIG. 61). More specifically, the proximal movement of the transfer mechanism 550 moves the carrier 5610, the slip member 5630, and the push rod tube 5660 in the proximal direction. Expanding further, with the engagement protrusions 5644 of the engagement member 5640 in contact with the insertion racks 5136 and 5156, the carrier 5610, the slip member 5630, and the push rod tube 5660 move in the proximal direction relative to the engagement member 5640, as shown in FIG. 61.

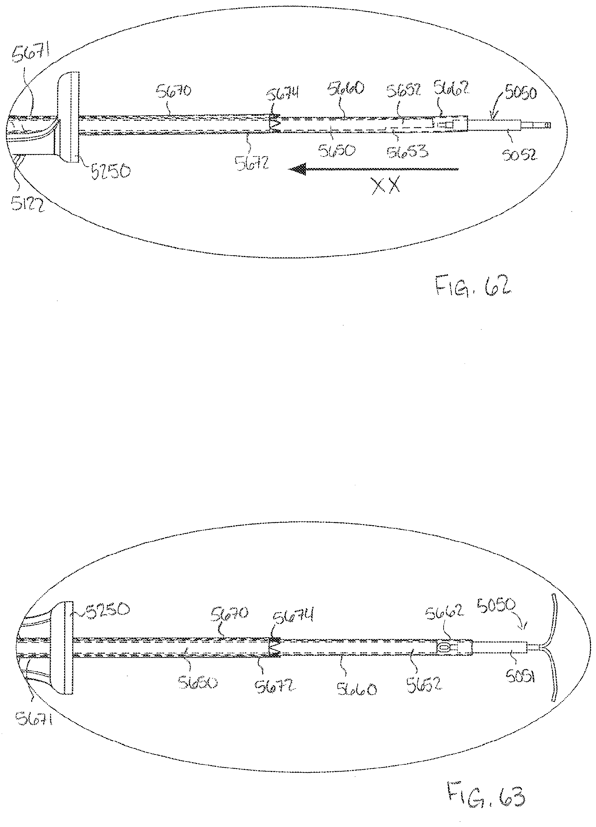

With the push rod tube 5660 fixedly coupled to the carrier 5610 and with the push rod 5650 fixedly coupled to the engagement member 5640, the proximal movement of the carrier 5610 moves the push rod tube 5660 in the proximal direction relative to the push rod 5650, as indicated by the arrow **XX** in FIG. 62. In this manner, a second portion 5052 of the implant 5050 can extend beyond the distal end portion 5662 of the push rod tube 5660. As shown in FIG. 63, in embodiments wherein the implant 5050 is an IUD, the proximal movement of the push rod tube 5660 can be such that the arms of the IUD can expand. In this manner, the IUD can be delivered to the target location (e.g., a wall of the uterus) and the delivery device 5000 can be removed. Expanding further, negative pressure within the vacuum assembly 5200 can be bled by a valve or the like (not shown) to allow the vacuum tip 5250 to be decoupled from, for example, the surface of the cervix. Moreover, with the first portion 5051 of the implant 5050 cut, the delivery device 5000 can be removed while the implant 5050 remains at or near the target location.

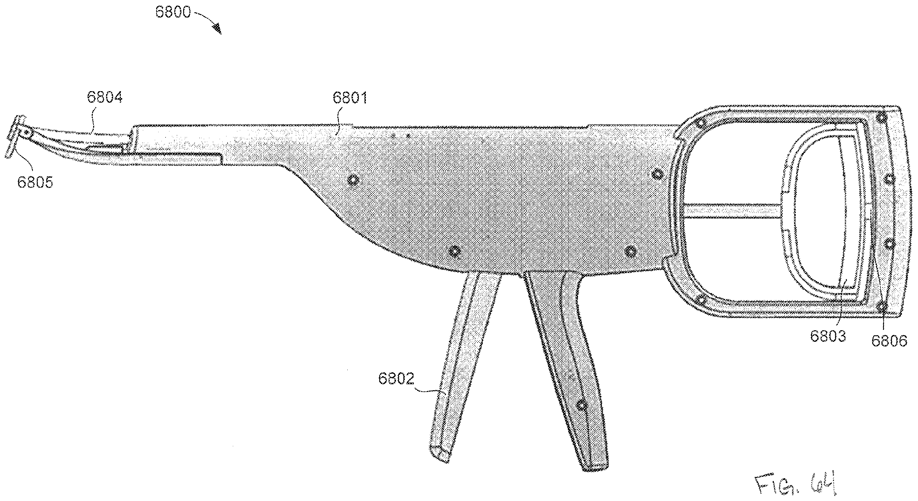

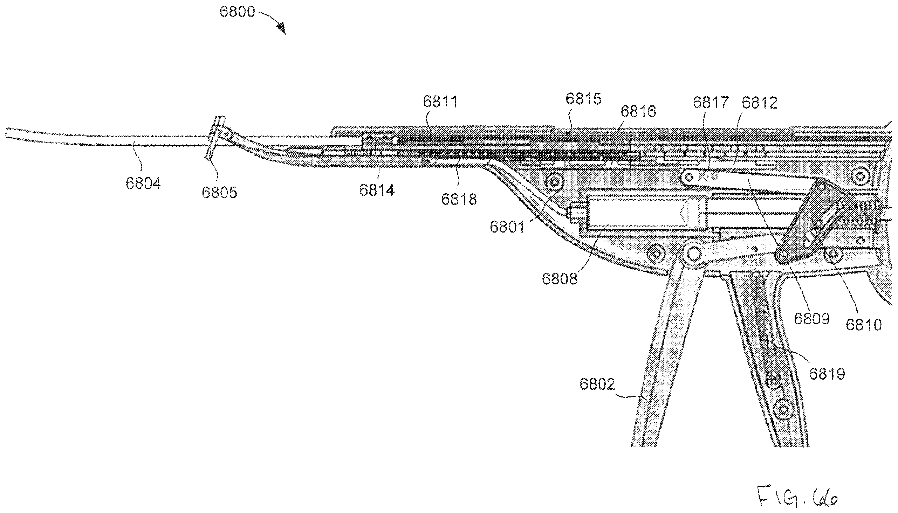

FIGS. 64-73 show a delivery device 6800 according to an embodiment. The delivery device 6800 includes a housing 6801 a cervical articulator 6805, which provides for attachment to the cervix so that the cervical canal can be straightened and/or repositioned with gentle traction. The attachment is made via suction/aspiration through one or more ports (not shown). The ports can be independent of one another or connected in parallel or in series. In this embodiment, the ports are coplanar on a horseshoe-shaped plate that is on a hinge to articulate with varying cervical/uterine orientations. The horseshoe design facilitates an open line of sight for the inserting health care provider. In other embodiments, the ports can be on a convex or concave plate, on separate plates, on a circular plate with an opening in the middle for easy view of the cervical os, or any other suitable orientation. The plate(s) may also be hinged, flexible by using a section of very thin material, ribbed, or constructed from a flexible material. The plate(s) can also be separated into two or more independent plates, with one plate for each individual port. The number, sizes and shapes of ports can vary depending on the most effective shape, size and number determined through scientific research. The ports may have a protruding flexible flap in distal of the plate to encourage grasping of the port on to the tissue. In some embodiments, suction is created via a syringe cylindrical vessel or other of some shape that is hollow. The vacuum is created via a plunger or plunger-like mechanism. An example is shown in this embodiment. Suction could also be created with a vacuum fitting or other aspiration source.

In some embodiments, the cervical articulator 6805 can be disconnected from the housing 6801 and/or remaining portions of the delivery device 6800 and can be used as a separate device. Thus, in some embodiments, the cervical articulator 6805 can function substantially independently to perform functions similar to those performed by the cervical tenaculum in other intrauterine procedures, including, but not limited to, artificial insemination (intrauterine semination), colcoscopy, dilation and curettage, manual vacuum aspiration, electric vacuum aspiration, endometrial biopsy, dilatation and evacuation, insertion of various contraceptive devices, uterine fibroid removal and certain abortion procedures.

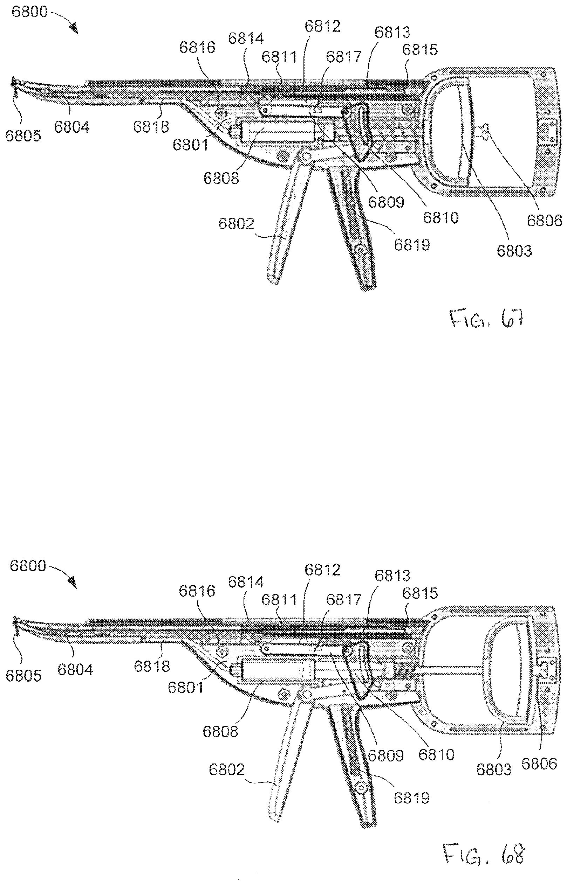

The delivery device 6800 includes a suction assembly, including but not limited to a handle 6803, a vacuum creating mechanism 6808 (e.g., a syringe), a tubing 6818, a handle lock 6806, and any suitable port or ports (not shown) disposed at a distal end of the delivery device 6800 to create suction with the tissue with which it comes in contact (see e.g., FIGS. 64-67). The suction will enable a user of the delivery device 6800 to pull traction on the tissue up to certain level of force. The user can move the handle 6803 in the proximal direction to actuate the vacuum creating mechanism 6808 (FIG. 68). The handle lock 6806 can retain the handle 6803 in the distal position (see e.g., FIG. 68).

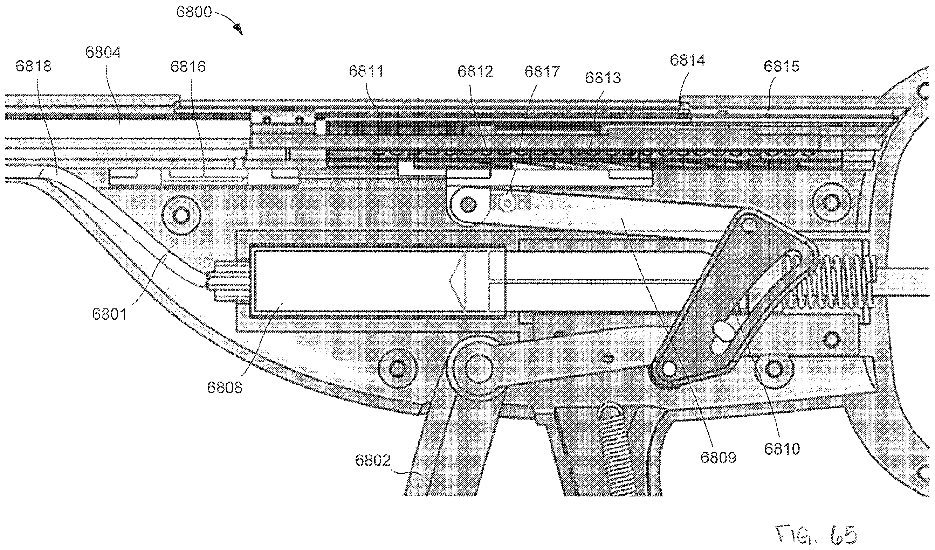

An insertion event is accomplished using a series of interconnected parts within the housing 6801. For example, the delivery device 6800 can include a trigger 6802, a four-bar linkage 6809 (also referred to herein as "linkage"), a drive plate 6812, a shuttle 6813, a carrier 6814, a deployment rod 6811 (also referred to herein as "rod"), and an insertion tube 6804 (also referred to herein as "delivery tube 6804" or "tube") (see e.g., FIGS. 65-73). The drive plate 6812 is moved by the linkage 6809, which then pushes the shuttle 6813 via a ratchet mechanism. The ratchet mechanism allows for a distal movement of the shuttle 6813 while limiting and/or preventing a proximal movement. The shuttle 6813, in turn, pushes the carrier 6814 via a second ratchet mechanism. The ratchet mechanism of the shuttle 6813 includes a set of notches that have rounded edges. The carrier 6814 has a rounded nodule that fits within the rounded ratchet track of the shuttle 6813. The carrier 6814 in turn, is attached to the deployment rod 6811 and the deployment tube 6804 which contains and/or is coupled to the IUD during the insertion.

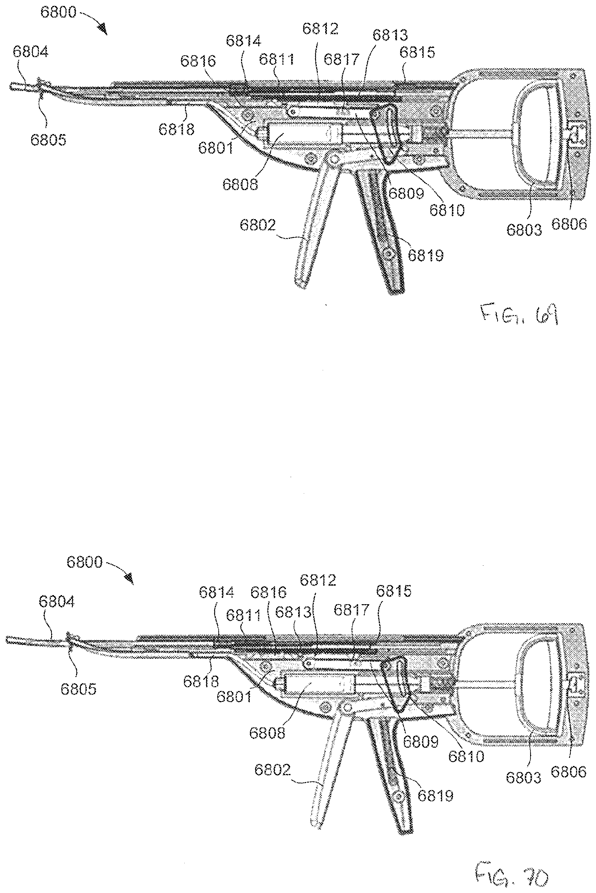

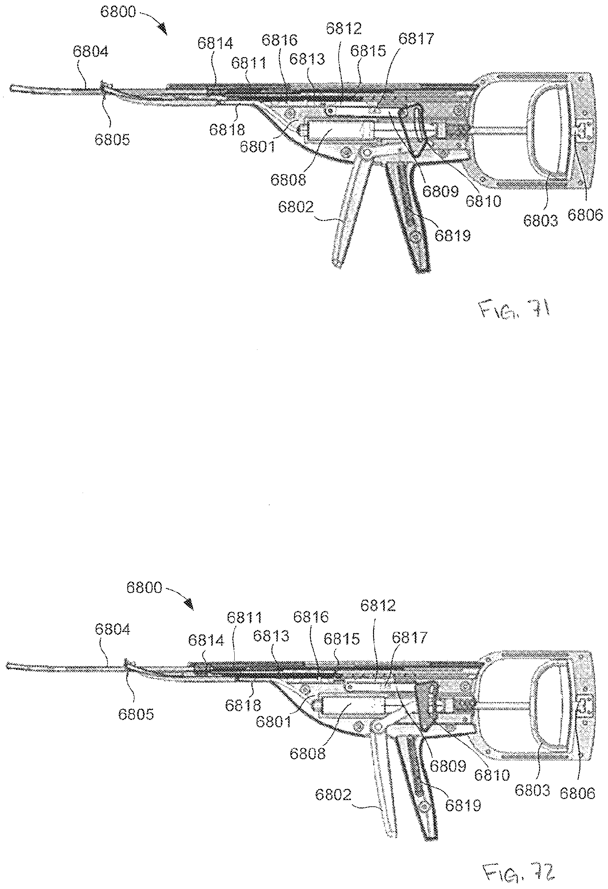

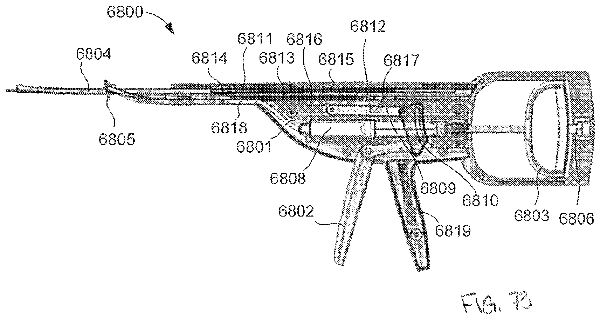

The delivery device 6800 can be configured to be actuated in a multi-actuation method. Similarly stated, the delivery device 6800 described herein can insert an IUD via several discrete actions and/or actuations. The first actuation moves the delivery device from a first drive plate 6812 distal three centimeters, pushing distal the shuttle 6813, the carrier 6814, the rod 6811, and the tube 6804 (FIG. 69). Upon release of the trigger 6802, the drive plate 6812 ratchets back three centimeters while the other parts (i.e., the shuttle 6813, the carrier 6814, the rod 6811 and the tube 6804) remain in the advanced position. Upon the next (second) actuation, the drive plate 6812 again moves distal three centimeters, moving everything else distal (i.e., the shuttle 6813, the carrier 6814, the rod 6811, and the tube 6804) as well (FIG. 70). During the release of the trigger 6802, the drive plate 6812 slides back once again. The third actuation (FIG. 71) is identical to the first two. On the fourth actuation, all of the parts again move distal three centimeters, however, upon the release of the handle, the deployment tube 6804 retracts proximal about 1.2 centimeters while the deployment rod 6811 remains in place in order to allow the arms of the IUD to unfold (FIG. 72). The amount of distal and proximal movement can be controlled by changing the appropriate dimensions of the shuttle 6813, carrier 6814, and/or the rod 6811. Additional mechanisms or parts can be added to produce a distal and a proximal motion with a single actuation. Upon the fifth and final actuation, the drive plate 6812 again pushed everything distal including the deployment tube 6804, which pushes the IUD from the underside of the T-arms (FIG. 73).

Although shown as including specific mechanisms (e.g., the ratchet mechanisms and/or the four bar linkage), in other embodiments, any suitable mechanism of action can be used to accomplish the motions set forth above. Examples of such alternative mechanisms include integrating some of the major parts to reduce the total number of moving parts, including another handle and/or linkage type, changing the number of actuations and/or the distance of travel for each actuation, or other functional modifications. The number of actuations can be changed. In this case five actuations are described. In the other embodiments, the number of actuations can range from 1 to infinity.

As described above, the delivery device 6800 is configured to include a force-limiting mechanism that prevents the distal end of the delivery device 6800 from exerting a force sufficiently large to perforate a uterus. This limiting force may be a constant or may be variable depending on the exact distance of travel. In some embodiments, this limit is regulated by the ratchet mechanism between the shuttle 6813 and carrier 6814. The amount of force is a function of the diameter of the divots (e.g., recesses, detents, radii, etc.) on the ratchet mechanism. To create the variability in force, the diameter of divots can be different in different locations of the ratchet mechanism. Other mechanisms can also be used to create variability in limiting force.

The delivery device 6800 can be a one-time use, disposable device, with features that limit the feasibility of reuse. For example, in some embodiments, the trigger 6802 locks out after the final actuation so that it will not return to its original position. In some embodiments, a wire clip can be attached to the delivery tube 6804 that can move in a distal direction but prevents substantially any movement of the delivery tube 6804 in the proximal direction. In some embodiments, there can also be several other spring clips that fit into specific channels upon certain events to prevent proximal movement of the delivery tube 6804. In other embodiments, these spring clips and channels/notches may be altered. In some embodiments, however, such use-limiting features need not be included. For example, some embodiments could include different materials allowing the delivery device 6800 to be used multiple times with the ability to be sterilized through different mechanism, including but not limited to autoclaving.

The delivery device also includes a depth indicator 6815 in order to give the user visual feedback as to the distance traveled by the IUD or other inserted medical product into the uterus or other body cavity. As shown in FIGS. 64-73, the depth indicator 6815 is disposed along a top plane of the delivery device 6800 so that it will be directly in the line of sight of the user.

In some embodiments, a sheath can be used to ease entry of an IUD or other object through the cervical os into the uterus. The distal end (first end to make contact with the cervix) may be tapered to simultaneously act as a cervical dilator or os finder, and will be hollow to allow the passage of an IUD or other medical product. The distal end may contain slits or it may be a simple taper with a small hole at the distal end comprised of a material that will expand to facilitate passage or other suitable materials or design. The sheath can be used on the distal end of the delivery device 6800 or separately as an add-on to other devices used for cervical penetration or insertion into other sphincters in the human body. A sheath may or may not be included as a part of the delivery device 6800.

Although not shown in FIG. 64-73, the tube 6804 that houses the IUD can be tapered at the distal end. The tube 6804 may also be split in four or more parts on the distal end to provide flexibility. The tapered distal end can help move through the cervical canal more easily than a non-tapered tube 6804 as well as act as an os finder and/or cervical dilator. A closed end tube 6804 with slits similar to the distal sheath 5670, described above with reference to FIGS. 41 and 42, can be easily moved through the cervical canal. The rounded end can reduce the chances of perforation as the surface area of the tube coming in contact with the fundus of the uterus increases, thereby reducing the pressure exerted for the same amount of force applied. The reduction in pressure is such that the likelihood of perforation is reduced.

An IUD can be loaded into the distal end of the delivery device 6800 by several different mechanisms. For example, an IUD can be inserted through the use of a separate tool that comes attached to the delivery device 6800 (not shown in FIGS. 64-73). The tool can include a metal loop through which the strings of the IUD would be threaded. This tool can be pulled away from the delivery device 6800, in turn threading the IUD strings through the delivery tube 6804 and/or sheath, through a defined channel, and through a cutter pathway. In other embodiments, a funneled distal end tool can be used to facilitate the ease of inserting the IUD and strings (or filaments) into the delivery device 6800. In yet other embodiments, the tube 6804 can define a cutout in a side such that the IUD could be placed into the tube 6804 horizontally as opposed to from the top of the delivery device 6800.

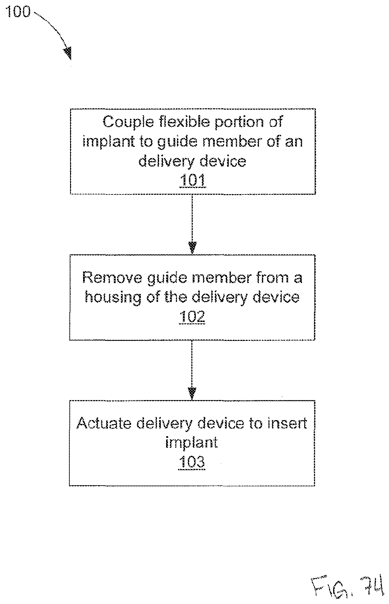

FIG. 74 is a flowchart illustrating a method 100 for delivering an implant to a target location. The method 100 includes coupling a flexible portion of an implant to a guide member of a delivery device, as 101. The delivery device can be any of the delivery devices 1000, 2000, 3000, 4000, 5000, or 6800 described herein. In some embodiments, the implant can be, for example, an intrauterine device and the flexible portion can be, for example, a retraction filament. The guide member can be any suitable guide member. For example, in some embodiments, the guide member can include at least a filament portion that is releasably coupled to the flexible portion of the implant.

At 102, the guide member is removed from a housing of the delivery device. For example, in some embodiments, a proximal end portion of a guide member can be engaged by a user and moved in a proximal direction such that the flexible portion of the implant is pulled at least partially through the housing. In some embodiments, the housing or other feature of the delivery device can include a manipulator that can engage the flexible portion of the implant to decouple the implant from the guide member. For example, in some embodiments, the manipulator can include a cutter configured to sever the flexible portion of the implant.

At 103, the delivery device can be actuated to insert the implant into a target location or tissue. For example, in some embodiments, the delivery device can include a trigger or the like that can be manipulated by a user (e.g., a physician, technician, nurse, etc.) to actuate the delivery device. In some embodiments, the actuator can be operative in moving a portion of the delivery device relative the housing. For example, in some embodiments, the actuator can be coupled to (either directly or indirectly) an insertion assembly that inserts the implant into a target location or tissue. In some embodiments, the actuator can be sequentially manipulated such that the insertion of the implant is performed in discrete actuated stages. For example, in some embodiments, the actuator can be similar to the actuator assembly 5400 described above with reference to FIGS. 9-63. In this manner, the actuator can be manipulated any number of times to insert the implant. In some embodiments, the delivery device can include a force-limiting feature that can limit the amount of force exerted on a target location that would otherwise be exerted by a delivery device without a force-limiting feature. In this manner, the likelihood of injury from the insertion of the implant can be reduced.

In some embodiments, a delivery device (e.g., any of those described herein) can be a manually operated device that inserts an IUD into the uterus. In some embodiments, the ease of insertion can be increased and the risk of complications due to poor insertion techniques can be reduced. In some embodiments, a delivery device may also be used to insert any other suitable device, implant and/or pharmaceutical into a body. For example, the embodiments and methods described herein can be used for insertion of a catheter, enema, drug delivery object, imaging tools, endoscopy, tubes (e.g., into the lungs and other body cavities), or other applications where precise insertion would be beneficial to the efficacy of the treatment and/or to eliminate complications or pain.

In some embodiments, any of the delivery devices described herein can be made with parts formed from various biocompatible materials including but not limited to a housing (such as, for example, the housing 5100), an insertion tube(s) (such as, for example, the push rod tube 5660), and/or a cervical articulator or vacuum tip (such as, for example, the vacuum tip 5250). In some embodiments, a delivery device can articulate with the cervix and can insert the IUD into a woman's uterus without the use of other tools, and without exceptional skill and/or training. Thus, after a short training session, any health care provider can properly insert an IUD safely.

Although specifically described herein, a cervical articulator similar to the vacuum tip 5250 can also be used as a separate medical device to replace the use of a cervical tenaculum. Similarly stated, in some embodiments, all or portions of any of the vacuum assemblies shown herein can be used as an improved tenaculum that provides temporary attachment to the cervix through vacuum/suction mechanism instead of known methods using a sharp tongs-like mechanism.

While various embodiments have been described herein, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

Although the embodiments described herein are shown as delivering an implant through an existing bodily lumen (e.g., an opening and/or canal defined by the cervix), in other embodiments, a device can include a dilator configured to define a bodily lumen and/or expand an existing bodily lumen. In some embodiments, for example, a contact portion of a head includes a dilator configured to dilate a lumen defined by the target location. The dilator can define a channel and/or passageway through which an insertion member can be conveyed to deliver an implant.

In some embodiments, a delivery device can include a sleeve configured to be disposed about a distal portion of the delivery device during the insertion operation. The sleeve can be a thin, flexible sleeve, which can serve to facilitate insertion of the delivery device and/or maintain sterility during the insertion operation. In some embodiments, an outer surface of the sleeve can include a lubricant.

In some embodiments, a device can include a head similar to any of the heads shown and described above, and the head can include a protrusion configured to position the head relative to a lumen defined by the target location. Similarly stated, in some embodiments, a delivery device can include a locating protrusion configured to facilitate the alignment and/or positioning of the device with respect to a target location. In some embodiments, the protrusion can define a channel through which an insertion member can be conveyed to deliver an implant.

In some embodiments, a device can include an articulating (or rotating) head or vacuum tip. In such embodiments, the head and/or portions of the housing can define a curved and/or nonlinear path through which portions of the insertion assembly can be disposed. In some embodiments, all or portions of any of the insertion assemblies described herein can be constructed to be flexible and/or elastically deformable to facilitate transmission through a nonlinear and/or curved passageway.

Although the vacuum assembly 5200 is shown and described above as producing a vacuum via the distal movement of a plunger, in other embodiments, any of the devices shown and described herein can include any suitable mechanism for producing a vacuum. Moreover, in some embodiments, a device can employ an external mechanism for producing a vacuum.

In some embodiments, an implant delivery device includes one or more mechanical biosensors around the rim of the head and/or the insertion member and a light emitting diode (LED) or other electronic output device at the opposite end of the device. Other indicators can be used instead of an LED, such as for, example, any suitable visual output device (LCD screens, etc.), audible output devices (e.g., a whistle), or mechanical output devices (e.g., haptic output devices).

In some embodiments, an implant delivery device can rotate, bend, and/or move with the cervix and insert the IUD into a woman's uterus with no other tools needed, and without the need for exceptional skill and/or training. The design of the embodiments described herein facilitates ease of use such that after a short training session, any health care provider can properly insert an IUD safely with aseptic technique.

Although various embodiments have been described as having particular features and/or combinations of components, other embodiments are possible having a combination of any features and/or components from any of embodiments where appropriate.

The invention is defined by the appended claims.

## Claims

1. An apparatus (5000), comprising:
a housing (5100) defining a housing passageway including a vacuum cylinder (5210);
an engagement member (5230) rotationally coupled to the housing (5100);
a plunger (5240) disposed within the vacuum cylinder (5210), the engagement member (5230) configured to rotate relative to the housing (5100) in response to an applied force, rotation of the engagement member (5230) to move the plunger (5240) in a linear direction within the vacuum cylinder (5210), the movement of the plunger (5240) within the vacuum cylinder (5210) producing a negative pressure within the vacuum cylinder (5210); and a vacuum tip (5250) coupled to the housing, the vacuum tip (5250) defining a lumen (5254) and including a distal surface (5256) having an annular shape that defines an opening (5258), the distal surface (5256) arranged to define a vacuum channel (5257) in fluid communication with the vacuum cylinder (5210) via the lumen (5254) and the distal surface (5256) configured such that when the surface is in contact with a cervix of a uterus:
(1) the distal surface (5256) is arranged to circumscribe a cervical os; and,
(2) the annular shape is arranged such that the opening (5258) is at least partially aligned with the cervical os,
wherein the distal surface is further configured such that a portion of the cervix is disposed in the vacuum channel (5257) when the distal surface (5256) contacts the cervix such that when the plunger (5240) is moved within the vacuum cylinder (5210), a suction force resulting from the negative pressure within the vacuum cylinder (5210) is exerted on the portion of the cervix, the suction force operable to retain the portion of the cervix within the vacuum channel (5257), and wherein the apparatus further comprises:
the apparatus configured to receive an insertion assembly (5600) within the housing (5100), the insertion assembly (5600) having a distal end portion configured to be removably coupled to an implant (5050), at least a portion of the insertion assembly (5600) is configured to be moved in a distal direction through the opening (5258) to insert the implant (5050) into a bodily cavity defined by the uterus when the distal surface (5256) of the vacuum tip (5250) is in contact with the cervix; and
a transfer member (5510) movably disposed in the housing passageway and coupled to the insertion assembly (5600), the transfer member (5510) is configured to transfer force from an actuator (5400) to the insertion assembly (5600) to move at least the portion of the insertion assembly (5600) through the opening (5258) of the vacuum tip (5250), the transfer member (5510) including a coupling portion configured to move relative to the insertion assembly (5600) when the force exceeds a threshold value.

2. The apparatus (5000) of claim 1, wherein the transfer member (5510) is configured to reciprocate within the housing (5100).

3. The apparatus (5000) of claim 1, wherein the insertion assembly (5600) includes a slip member (5630), the slip member (5630) is configured to maintain contact with a slip surface (5522) of the transfer member (5510) when the transfer member (5510) moves relative to the insertion assembly (5600).

4. The apparatus (5000) of claim 3, wherein the slip surface (5522) of the transfer member (5510) includes a plurality of detents configured to matingly receive a portion of the slip member (5630) of the insertion assembly (5600).

5. The apparatus (5000) of claim 1, wherein the transfer member (5510) has plurality of ratchet teeth configured to engage a portion of the actuator (5400) such that actuation of the actuator (5400) results in distal movement of the transfer member (5510).

6. The apparatus (5000) of claim 1, wherein the transfer member (5510) includes a lock out member (5540) configured to engage a rack (5155) of the housing (5100), the lock out member (5540) and the rack (5155) collectively configured to limit movement of transfer member (5510) relative to the housing (5100).

7. The apparatus (5000) of claim 1, further comprising;
a cutter assembly (5700), the cutter assembly (5700) configured to move relative to the insertion assembly (5600) to decouple the implant (5050) from the insertion assembly (5600) when the implant (5050) is placed in a desired portion within the bodily cavity defined by the uterus.

8. The apparatus (5000) of claim 1, further comprising:
an insertion assembly (5600) removably coupled to an implant (5050), at least a portion of the insertion assembly (5600) is configured to be moved in a distal direction through the opening (5258) to insert the implant (5050) in a bodily cavity defined by the uterus when the distal surface (5256) of the vacuum tip (5250) is in contact with the cervix, a distal end surface associated with the insertion assembly (5600) is spaced apart from the distal surface (5256) of the vacuum tip (5250) between approximately five centimeters and approximately 13 centimeters.

9. The apparatus (5000) of any preceding claim, wherein the implant (5050) is an intrauterine device, a portion of the intrauterine device being flexible.

10. The apparatus (5000) of claim 1, wherein the housing passageway is nonlinear.

11. The apparatus (5000) of claim 1, wherein the vacuum channel (5257) circumscribes the opening (5258).

12. The apparatus (5000) of claim 1, wherein the plunger (5240) is moved from a first plunger position to a second plunger position to produce the negative pressure within the vacuum cylinder (5210), the apparatus further comprising:
a lock (5220) movably disposed in the housing (5100), the plunger (5240) configured to selectively contact a tab (5225) of the lock (5220) when the plunger (5240) is moved from the first plunger position to the second plunger position, the lock (5220) is configured to be moved by the plunger (5240) from a first lock position, in which the lock (5220) limits actuation of an actuator (5400), to a second lock position in which the lock (5220) does not limit actuation of the actuator (5400).

## Patentansprüche

1. Vorrichtung (5000), die Folgendes umfasst:
ein Gehäuse (5100), das einen Gehäusedurchgang mit einem Vakuumzylinder (5210) definiert;
ein Eingriffselement (5230), das mit dem Gehäuse (5100) rotatorisch gekoppelt ist;
einen Kolben (5240), der innerhalb des Vakuumzylinders (5210) angeordnet ist, wobei das Eingriffselement (5230) zum Drehen relativ zum Gehäuse (5100) als Reaktion auf eine aufgebrachte Kraft konfiguriert ist, wobei die Drehung des Eingriffselements (5230) den Kolben (5240) in einer linearen Richtung innerhalb des Vakuumzylinders (5210) bewegt, wobei die Bewegung des Kolbens (5240) innerhalb des Vakuumzylinders (5210) einen Unterdruck innerhalb des Vakuumzylinders (5210) erzeugt; und
eine Vakuumspitze (5250), die mit dem Gehäuse gekoppelt ist, wobei die Vakuumspitze (5250) ein Lumen (5254) definiert und eine distale Fläche (5256) mit einer ringförmigen Gestalt aufweist, die eine Öffnung (5258) definiert, wobei die distale Fläche (5256) zum Definieren eines Vakuumkanals (5257) in Fluidverbindung mit dem Vakuumzylinder (5210) über das Lumen (5254) ausgelegt ist und die distale Fläche (5256) so konfiguriert ist, dass, wenn die Fläche mit dem Hals einer Gebärmutter in Kontakt ist :
(1) die distale Fläche (5256) so angeordnet ist, dass sie einen Muttermund umschreibt; und
(2) die ringförmige Gestalt so ausgelegt ist, dass die Öffnung (5258) zumindest teilweise mit dem Muttermund ausgerichtet ist,
wobei die distale Fläche ferner so konfiguriert ist, dass ein Abschnitt des Gebärmutterhalses im Vakuumkanal (5257) angeordnet ist, wenn die distale Fläche (5256) den Gebärmutterhals berührt, so dass, wenn der Kolben (5240) innerhalb des Vakuumzylinders (5210) bewegt wird, eine Saugkraft, die aus dem Unterdruck innerhalb des Vakuumzylinders (5210) resultiert, auf den Abschnitt des Gebärmutterhalses ausgeübt wird, wobei die Saugkraft die Aufgabe hat, den Abschnitt des Gebärmutterhalses innerhalb des Vakuumkanals (5257) zu halten, und wobei die Vorrichtung ferner Folgendes umfasst:
die Vorrichtung ist zum Aufnehmen einer Einführungsanordnung (5600) innerhalb des Gehäuses (5100) konfiguriert, wobei die Einführungsanordnung (5600) einen distalen Endabschnitt aufweist, der zum entfernbaren Koppeln mit einem Implantat (5050) konfiguriert ist, mindestens ein Abschnitt der Einführungsanordnung (5600) ist zum Bewegen in einer distalen Richtung durch die Öffnung (5258) konfiguriert, um das Implantat (5050) in einen durch die Gebärmutter definierten Körperhohlraum einzuführen, wenn die distale Fläche (5256) der Vakuumspitze (5250) mit dem Gebärmutterhals in Kontakt ist; und
ein Übertragungselement (5510), das beweglich in dem Gehäusedurchgang angeordnet und mit der Einführungsanordnung (5600) gekoppelt ist, wobei das Übertragungselement (5510) zum Übertragen von Kraft von einem Aktuator (5400) auf die Einführungsanordnung (5600) konfiguriert ist, um zumindest den Abschnitt der Einführungsanordnung (5600) durch die Öffnung (5258) der Vakuumspitze (5250) zu bewegen, wobei das Übertragungselement (5510) einen Kopplungsabschnitt aufweist, der zum Bewegen relativ zur Einführungsanordnung (5600) konfiguriert ist, wenn die Kraft einen Schwellenwert überschreitet.

2. Vorrichtung (5000) nach Anspruch 1, wobei das Übertragungselement (5510) zum Hin- und Herbewegen innerhalb des Gehäuses (5100) konfiguriert ist.

3. Vorrichtung (5000) nach Anspruch 1, wobei die Einführungsanordnung (5600) ein Gleitelement (5630) aufweist, wobei das Gleitelement (5630) so konfiguriert ist, dass es mit einer Gleitfläche (5522) des Übertragungselements (5510) in Kontakt bleibt, wenn sich das Übertragungselement (5510) relativ zur Einführungsanordnung (5600) bewegt.

4. Vorrichtung (5000) nach Anspruch 3, wobei die Gleitfläche (5522) des Übertragungselements (5510) mehrere Arretierungen aufweist, die zum paarenden Aufnehmen eines Abschnitts des Gleitelements (5630) der Einführungsanordnung (5600) konfiguriert sind.

5. Vorrichtung (5000) nach Anspruch 1, wobei das Übertragungselement (5510) mehrere Ratschenzähne aufweist, die zum Eingreifen in einen Abschnitt des Aktuators (5400) konfiguriert sind, so dass eine Betätigung des Aktuators (5400) zu einer distalen Bewegung des Übertragungselements (5510) führt.

6. Vorrichtung (5000) nach Anspruch 1, wobei das Übertragungselement (5510) ein Aussperrelement (5540) aufweist, das zum Eingreifen in eine Zahnstange (5155) des Gehäuses (5100) konfiguriert ist, wobei das Aussperrelement (5540) und die Zahnstange (5155) gemeinsam so konfiguriert sind, dass sie die Bewegung des Übertragungselements (5510) relativ zum Gehäuse (5100) begrenzen.

7. Vorrichtung (5000) nach Anspruch 1, die ferner Folgendes umfasst;
eine Cutter-Anordnung (5700), wobei die Cutter-Anordnung (5700) zum Bewegen relativ zur Einführungsanordnung (5600) konfiguriert ist, um das Implantat (5050) von der Einführungsanordnung (5600) abzukoppeln, wenn das Implantat (5050) in einem gewünschten Abschnitt innerhalb des durch die Gebärmutter definierten Körperhohlraums platziert wird.

8. Vorrichtung (5000) nach Anspruch 1, die ferner Folgendes umfasst:
eine Einführungsanordnung (5600), die entfernbar mit einem Implantat (5050) gekoppelt ist, wobei mindestens ein Abschnitt der Einführungsanordnung (5600) zum Bewegen in einer distalen Richtung durch die Öffnung (5258) konfiguriert ist, um das Implantat (5050) in einen durch die Gebärmutter definierten Körperhohlraum einzuführen, wenn die distale Fläche (5256) der Vakuumspitze (5250) mit dem Gebärmutterhals in Kontakt ist, eine mit der Einführungsanordnung (5600) assoziierte distale Endfläche von der distalen Fläche (5256) der Vakuumspitze (5250) zwischen etwa fünf Zentimeter und ungefähr 13 Zentimeter beabstandet ist.

9. Vorrichtung (5000) nach einem vorherigen Anspruch, wobei das Implantat (5050) ein Intrauterinpessar ist, wobei ein Abschnitt des Intrauterinpessars flexibel ist.

10. Vorrichtung (5000) nach Anspruch 1, wobei der Gehäusedurchgang nichtlinear ist.

11. Vorrichtung (5000) nach Anspruch 1, wobei der Vakuumkanal (5257) die Öffnung (5258) umschreibt.

12. Vorrichtung (5000) nach Anspruch 1, wobei der Kolben (5240) von einer ersten Kolbenposition in eine zweite Kolbenposition bewegt wird, um den Unterdruck innerhalb des Vakuumzylinders (5210) zu erzeugen, wobei die Vorrichtung ferner umfasst:
eine Sperre (5220), die beweglich in dem Gehäuse (5100) angeordnet ist, wobei der Kolben (5240) zum selektiven Berühren einer Zunge (5225) der Sperre (5220) konfiguriert ist, wenn der Kolben (5240) von der ersten Kolbenposition in die zweite Kolbenposition bewegt wird, die Sperre (5220) zum Bewegen durch den Kolben (5240) von einer ersten Sperrenposition, in der die Sperre (5220) die Betätigung eines Aktuators (5400) begrenzt, in eine zweite Sperrenposition konfiguriert ist, in der die Sperre (5220) die Betätigung des Aktuators (5400) nicht begrenzt.

## Revendications

1. Appareil (5000), comprenant :
un boîtier (5100) définissant un conduit de boîtier qui comporte un cylindre à vide (5210) ;
un élément d'engagement (5230) couplé en rotation au boîtier (5100) ;
un piston (5240) disposé à l'intérieur du cylindre à vide (5210), l'élément d'engagement (5230) étant configuré pour tourner par rapport au boîtier (5100) en réponse à une force appliquée, la rotation de l'élément d'engagement (5230) déplaçant le piston (5240) dans une direction linéaire à l'intérieur du cylindre à vide (5210), le mouvement du piston (5240) à l'intérieur du cylindre à vide (5210) produisant une pression négative à l'intérieur du cylindre à vide (5210) ; et
un embout à vide (5250) couplé au boîtier, l'embout à vide (5250) définissant une lumière (5254) et comportant une surface distale (5256) ayant une forme annulaire qui définit une ouverture (5258), la surface distale (5256) étant disposée pour définir un canal à vide (5257) en communication fluidique avec le cylindre à vide (5210) par l'intermédiaire de la lumière (5254) et la surface distale (5256) étant configurée de telle façon que, lorsque la surface est en contact avec un col d'un utérus :
(1) la surface distale (5256) est disposée pour entourer un orifice du col de l'utérus ; et
(2) la forme annulaire est disposée de telle façon que l'ouverture (5258) est alignée au moins partiellement avec l'orifice du col de l'utérus,
dans lequel la surface distale est en outre configurée de telle façon qu'une partie du col est disposée dans le canal à vide (5257) lorsque la surface distale (5256) est en contact avec le col, de telle sorte que, lorsque le piston (5240) est déplacé à l'intérieur du cylindre à vide (5210), une force d'aspiration résultant de la pression négative à l'intérieur du cylindre à vide (5210) est exercée sur la partie du col, la force d'aspiration étant utilisable pour retenir la partie du col à l'intérieur du canal à vide (5257), et dans lequel l'appareil comprend en outre :
l'appareil étant configuré pour recevoir un ensemble d'insertion (5600) à l'intérieur du boîtier (5100), l'ensemble d'insertion (5600) ayant une partie d'extrémité distale configurée pour être couplée de manière amovible à un implant (5050), au moins une partie de l'ensemble d'insertion (5600) étant configurée pour être déplacée dans une direction distale à travers l'ouverture (5258) pour insérer l'implant (5050) dans une cavité corporelle définie par l'utérus lorsque la surface distale (5256) de l'embout à vide (5250) est en contact avec le col ; et
un élément de transfert (5510) disposé de manière mobile dans le conduit de boîtier et couplé à l'ensemble d'insertion (5600), l'élément de transfert (5510) étant configuré pour transférer de la force depuis un actionneur (5400) à l'ensemble d'insertion (5600) pour déplacer au moins la partie de l'ensemble d'insertion (5600) à travers l'ouverture (5258) de l'embout à vide (5250), l'élément de transfert (5510) comportant une partie d'accouplement configurée pour se déplacer par rapport à l'ensemble d'insertion (5600) lorsque la force dépasse une valeur seuil.

2. Appareil (5000) selon la revendication 1, dans lequel l'élément de transfert (5510) est configuré pour effectuer un mouvement de va-et-vient à l'intérieur du boîtier (5100) .

3. Appareil (5000) selon la revendication 1, dans lequel l'ensemble d'insertion (5600) comporte un élément de glissement (5630), l'élément de glissement (5630) étant configuré pour maintenir le contact avec une surface de glissement (5522) de l'élément de transfert (5510) lorsque l'élément de transfert (5510) se déplace par rapport à l'ensemble d'insertion (5600).

4. Appareil (5000) selon la revendication 3, dans lequel la surface de glissement (5522) de l'élément de transfert (5510) comporte une pluralité de crans configurés pour recevoir de manière complémentaire une partie de l'élément de glissement (5630) de l'ensemble d'insertion (5600).

5. Appareil (5000) selon la revendication 1, dans lequel l'élément de transfert (5510) possède une pluralité de dents de cliquet configurées pour venir en prise avec une partie de l'actionneur (5400) de façon que l'actionnement de l'actionneur (5400) entraîne un mouvement distal de l'élément de transfert (5510).

6. Appareil (5000) selon la revendication 1, dans lequel l'élément de transfert (5510) comporte un élément de verrouillage (5540) configuré pour venir en prise avec une crémaillère (5155) du boîtier (5100), l'élément de verrouillage (5540) et la crémaillère (5155) étant collectivement configurés pour limiter le mouvement de l'élément de transfert (5510) par rapport au boîtier (5100) .

7. Appareil (5000) selon la revendication 1, comprenant en outre :
un ensemble de coupe (5700), l'ensemble de coupe (5700) étant configuré pour se déplacer par rapport à l'ensemble d'insertion (5600) pour désaccoupler l'implant (5050) de l'ensemble d'insertion (5600) lorsque l'implant (5050) est placé dans une partie voulue à l'intérieur de la cavité corporelle définie par l'utérus.

8. Appareil (5000) selon la revendication 1, comprenant en outre :
un ensemble d'insertion (5600) couplé de manière amovible à un implant (5050), au moins une partie de l'ensemble d'insertion (5600) étant configurée pour être déplacée dans une direction distale à travers l'ouverture (5258) pour insérer l'implant (5050) dans une cavité corporelle définie par l'utérus lorsque la surface distale (5256) de l'embout à vide (5250) est en contact avec le col, une surface d'extrémité distale associée avec l'ensemble d'insertion (5600) étant espacée de la surface distale (5256) de l'embout à vide (5250) entre approximativement cinq centimètres et approximativement treize centimètres.

9. Appareil (5000) selon l'une quelconque des revendications précédentes, dans lequel l'implant (5050) est un dispositif intra-utérin, une partie du dispositif intra-utérin étant flexible.

10. Appareil (5000) selon la revendication 1, dans lequel le conduit de boîtier est non linéaire.

11. Appareil (5000) selon la revendication 1, dans lequel le canal à vide (5257) entoure l'ouverture (5258).

12. Appareil (5000) selon la revendication 1, dans lequel le piston (5240) est déplacé d'une première position de piston à une deuxième position de piston pour produire la pression négative à l'intérieur du cylindre à vide (5210), l'appareil comprenant en outre :
un verrou (5220) disposé de manière mobile dans le boîtier (5100), le piston (5240) étant configuré pour venir sélectivement en contact avec une languette (5225) du verrou (5220) lorsque le piston (5240) est déplacé de la première position de piston à la deuxième position de piston, le verrou (5220) étant configuré pour être déplacé par le piston (5240) d'une première position de verrou, dans laquelle le verrou (5220) limite l'actionnement d'un actionneur (5400), à une deuxième position de verrou dans laquelle le verrou (5220) ne limite pas l'actionnement de l'actionneur (5400).
